# EUROPEAN PATENT SPECIFICATION

(11) **EP 0 975 668 B1**
(45) Date of publication and mention of the grant of the patent: **17.05.2006**
(21) Application number: 98916866.1
(22) Date of filing: 15.04.1998
(51) Int. Cl.: C07K 14/525, C07K 19/00

(54) **MODIFIED TNFalpha MOLECULES, DNA ENCODING SUCH MODIFIED TNFalpha MOLECULES AND VACCINES COMPRISING SUCH MODIFIED TNFalpha MOLECULES AND DNA**
VERÄNDERTE TNFalpha-MOLEKÜLE, DNS, WELCHE FÜR DERARTIGE VERÄNDERTE TNFalpha-MOLEKÜLE KODIERT UND IMPFSTOFFE, DIE DERARTIGE VERÄNDERTE TNFalpha-MOLEKÜLE UND DNS ENTHALTEN
MOLECULES TNFalpha MODIFIEES, ADN CODANT POUR CES MOLECULES ET VACCINS COMPRENANT CES MOLECULES TNFalpha MODIFIEES ET CET ADN

(30) Priority: 15.04.1997 DK 41897; 24.04.1997 US 44187 P
(43) Date of publication of application: 02.02.2000
(73) Proprietor: Pharmexa A/S, 2970 Horsholm (DK)
(72) Inventor: JENSEN, Martin, Roland, DK-2840 Holte (DK); MOURITSEN, Soren, DK-3460 Birkerod (DK); ELSNER, Henrik, DK-2700 Bronshoj (DK); DALUM, Iben, DK-2970 Horsholm (DK)
(74) Representative: Inspicos A/S
(86) International application number: PCT/DK1998/000157
(87) International publication number: WO 1998/046642

(56) References cited:
- EP-A- 0 251 037
- EP-A- 0 619 372
- WO-A-90/07579
- WO-A-95/05849

## Description

### Field of the invention

The present invention relates to human cytokine Tumor Necrosis Factor α (TNFα) molecules which has been modified so that they are capable of raising neutralizing antibodies towards wild-type human TNFα following administration of the modified TNFα molecule to the human host. The invention also relates to human TNFα vaccines based on said modified TNFα molecules. Further aspects of the invention will appear from the discussion below.

### Background of the invention

Physiologically, the vertebrate immune system serves as a defense mechanism against invasion of the body by infectious objects such as microorganisms. Foreign proteins are effectively removed via the reticuloendothelial system by highly specific circulating antibodies, and viruses and bacteria are attacked by a complex battery of cellular and humoral mechanisms including antibodies, cytotoxic T lymphocytes (CTL), natural killer cells (NK), complement, etc. The leader of this battle is the T helper (T_{H}) lymphocyte which, in collaboration with the Antigen Presenting Cells (APC), regulate the immune defense via a complex network of cytokines.

T_{H} lymphocytes recognize protein antigens presented on the surface of the APC. They do not recognize, however, native antigen per se. Instead, they appear to recognize a complex ligand consisting of two components, a "processed" (fragmented) protein antigen (the so-called T cell epitope) and a Major Histocompatibility Complex class II molecule (O. Werdelin et al., Imm. Rev. 106, 181 (1988)). This recognition eventually enables the T_{H} lymphocyte specifically to help B lymphocytes to produce specific antibodies towards the intact protein antigen (Werdelin et al., supra). A given T cell only recognizes a certain antigen-MIIC combination and will not recognize the same or another antigen presented by a gene product of another MHC allele. This phenomenon is called MHC restriction.

Fragments of self-proteins are also presented by the APC, but normally such fragments are ignored or not recognized by the T helper lymphocytes. This is the main reason why individuals generally do not harbour autoantibodies in their serum eventually leading to an attack on the individual's own proteins (the so-called self- or autoproteins). However, in rare cases the process goes wrong, and the immune system turns towards the individual's own components, which may lead to an autoimmune disease.

The presence of some self-proteins is inexpedient in situations where they, in elevated levels, induce disease symptoms. Thus, tumour necrosis factor α (TNFα) is known to be able to cause cachexia in cancer patients and patients suffering from other chronic diseases (H.N. Langstein et al. Cancer Res. 51, 2302-2306, 1991). TNFα also plays important roles in the inflammatory process (W.F. Arend et al. Arthritis Rheum. 33, 305-315, 1990) and neutralization of TNFα by the use of monoclonal antibodies has thus been demonstrated to be beneficial in patients with chronic inflammatory diseases such as rheumatoid arthritis, Elliott et al., Lancet 1994, 344:1105-10 and Crohn's disease, van Dullemen et al., Gastroenterology 109(1):129-135 (1995). There is therefore a need for a method for the induction of neutralizing antibodies against such TNFα proteins, and the present invention comprises a vaccine against TNFα which provide this property.

### TUMOUR NECROSIS FACTOR

### 1. General Background

Tumour necrosis factor (TNF) is a member of the cytokine family of regulatory proteins (see Walsh, G. and Headon, D.E., Protein Biotechnology, 1994, John Wiley & Sons Ltd. England, p. 257-267) which also include the interferons and the interleukins. Two forms of TNF are now recognized, TNFα and TNFβ, respectively. Although both proteins bind the same receptors and elicit broadly similar biological responses, they are distinct molecules and share less than 30% homology. The original protein termed tumour necrosis factor, referred to as TNF, is more properly termed TNFα; it is also known as cachectin. TNFβ is also referred to as lymphotoxin.

TNFα is produced by a wide variety of cell types, most notably activated macrophages, monocytes, certain T lymphocytes and NK cells, in addition to brain and liver cells. The most potent known inducer of TNFα synthesis, is a complex biomolecule termed lipopolysaccharide (LPS). It contains both lipid and polysaccharide components, and is also referred to as endotoxin. Lipopolysaccharide itself is devoid of any anti-tumour activity. The serum of animals injected with lipopolysaccharide was found to contain a factor toxic to cancerous cells, and this factor, produced by specific cells in response to lipopolysaccharide, was termed tumour necrosis factor. Various other agents such as some viruses, fungi and parasites also stimulate the synthesis and release of this cytokine. Furthermore, TNFα may act in an autocrine manner, stimulating its own production.

Native human TNFα is a homotrimer, consisting of three identical polypeptide subunits tightly associated around a threefold axis of symmetry as will be further explained below. This arrangement resembles the assembly of protein subunits in many viral capsid proteins. The individual polypeptide subunits of human TNFα are non-glycosylated and consist of 157 amino acids. The molecule has a molecular weight of 17300 Da and contains a single intrachain disulphide linkage. Human TNFα is synthesized initially as a 233 amino acid precursor molecule. Proteolytic cleavage of the -76 to -1 presequence including a signal sequence releases native TNFα. TNFα may also exist in a 26000 Da membrane-bound form. Three TNFα monomeric subunits associate noncovalently to form a trimer as further explained below.

TNFα induces its biological effects by binding specific receptors present on the surface of susceptible cells. Two distinct TNFα receptors have been identified. One receptor (TNF-R55) has a molecular weight of 55000 Da, whereas the second receptor (TNF-R75) has a molecular weight of about 75000 Da. These two distinct receptor types show no more than 25% sequence homology. TNF-R55 is present on a wide range of cells, whereas the distribution of the TNF-R75 receptor is more limited. Both are transmembrane glycoproteins with an extracellular binding domain, a hydrophobic transmembrane domain and an intracellular effector domain.

The exact molecular mechanisms by which TNFα induces its biological effects remain to be determined. Binding of TNFα to its receptor seems to trigger a variety of events mediated by G-proteins in addition to the activation of adenylate cyclase, phospholipase A and protein kinases. The exact biological actions induced by TNFα may vary from cell type to cell type. Other factors, such as the presence of additional cytokines, further modulate the observed molecular effects attributed to TNFα action on sensitive cells.

The TNFα gene has been cloned and inserted in a variety of recombinant expression systems, both bacterial and eukaryotic. The resultant availability of large quantities of purified, biologically active TNFα has facilitated clinical evaluation a number of diseases, most notably cancer. Many such trials, using TNFα either alone or in combination with interferons, yielded, however, very disappointing results. Large quantities of TNFα can not be administered to patients owing to its toxic - if not lethal - side-effects.

As mentioned above prolonged production of inappropriately elevated levels of TNFα has also been implicated in the development of cachexia, the wasting syndrome often associated with chronic parasitic or other infections, and with cancer. TNFα is also involved in the metastasis and growth of certain tumours as well as in induction of anaemia. Furthermore, TNFα is also directly involved in the development of certain chronic inflammatory disorders in humans, including rheumatoid arthritis and Crohn's disease where administration of monoclonal anti-TNFα antibodies has been shown to be beneficial. TNFα is also involved in osteoporosis and Psoriasis. In addition, it has been shown in animal models that administration of anti-TNFα antibodies may decrease or prevent, rejection of grafted or transplanted tissues.

### 2. Structure of TNFα

### I. Introduction

The three-dimensional structure of human tumour necrosis factor (TNFα) has been solved (see "Tumor Necrosis Factors, Structure, Function and Mechanism of Action" edited by Bharat B. Aggarwal and Jan Vilcek, 1992 Marcel Dekker, Ind., New York, Chapter 5 "Crystal structure of TNFα ", by Jones, E.Y. Stuart, D.I. and Walker N.F.C.). The biological action of TNFα is dependent on its interaction with its receptors. These interactions are governed by the precise arrangement of the correctly folded tertiary structure. Thus, to understand how the TNFα molecule performs its biological function at the level of amino acid interactions, one must not only know the amino acid sequence, but also the three-dimensional structure.

### II. Three-dimensional structure

Biologically active THFα has been shown by analytical ultracentrifugation, small angle x-ray scattering, and gel electrophoresis to be in a trimer conformation in solution, and cross-linking studies have indicated that this is the active form of the protein (Smith and Baglioni, 1997, J. Biol. Chemistry 252, 6951-6954). Binding assays showed that the trimer was at least 8-fold more active than the monomer. The experimental evidence indicated that both natural and recombinant TNFα exist predominantly as a trimer under physiological conditions. Analysis of circular dichroism spectra placed TNFα in the all-sheet class of proteins (Davis et al., Biochemistry 1987, 26, 1322-1326 who reported results of structure analyses of purified recombinant TNFα by sulfhydryl titration, gelfiltration and circular dichroism.). Several different crystal forms have been reported for human recombinant TNFα. All the reported crystal forms exhibit crystallographic and/or non-crystallographic threefold symmetry indicative of the presence of TNFα as a trimer within the crystal. The TNFα trimers lie in loosely packed arrays perforated by 100 Å diameter solvent channels. Only a small proportion of the molecular surface is involved in crystal packing contacts. Such contacts could slightly perturb a few side chains and perhaps even short portions of inherently flexible main chain from their preferred solution conformations.

### A. Main-Chain Fold of the TNFα Monomer

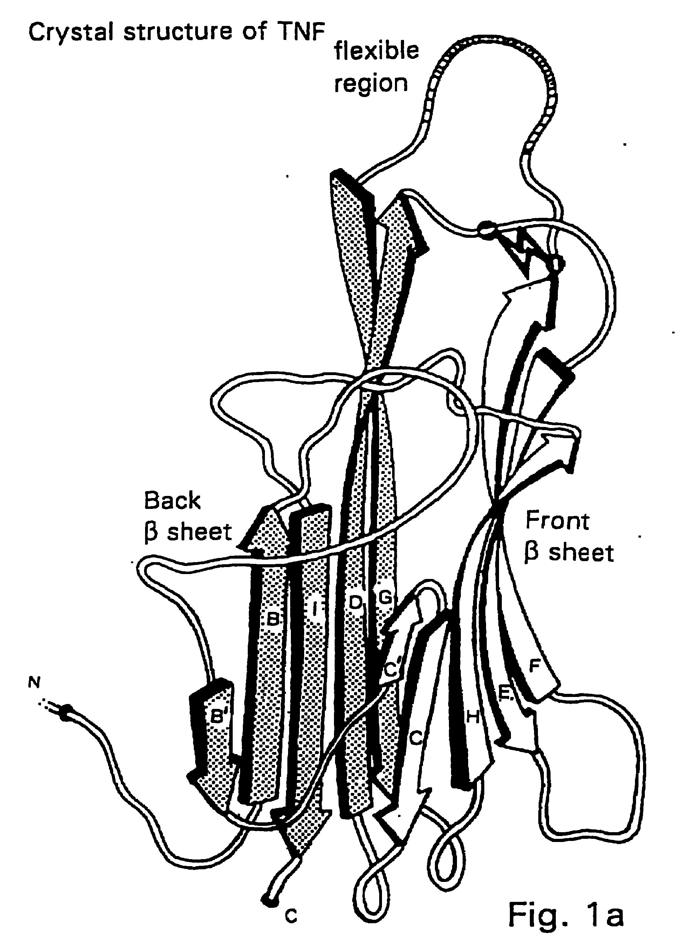

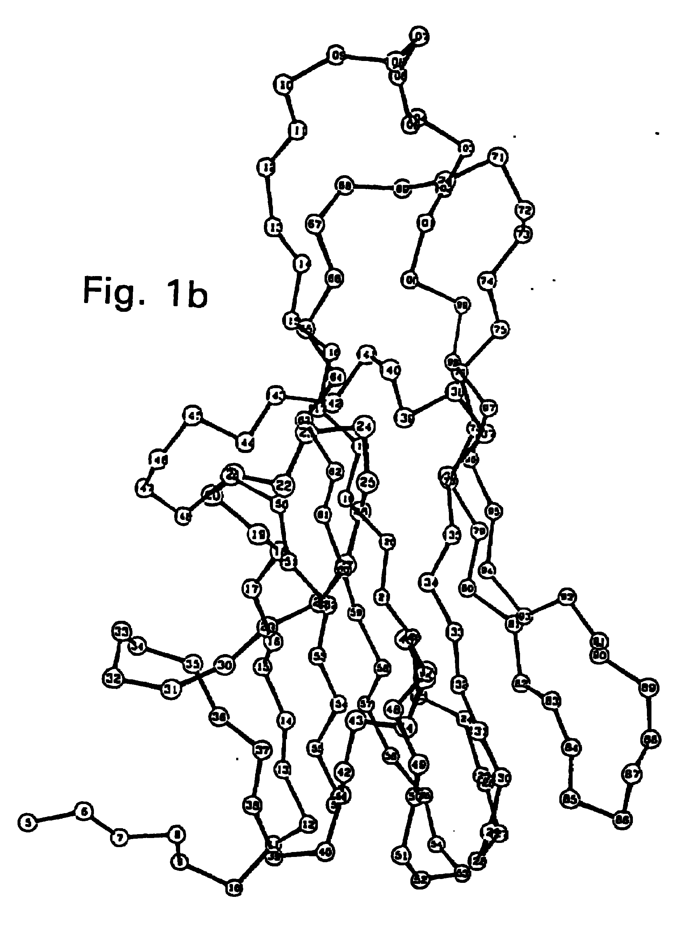

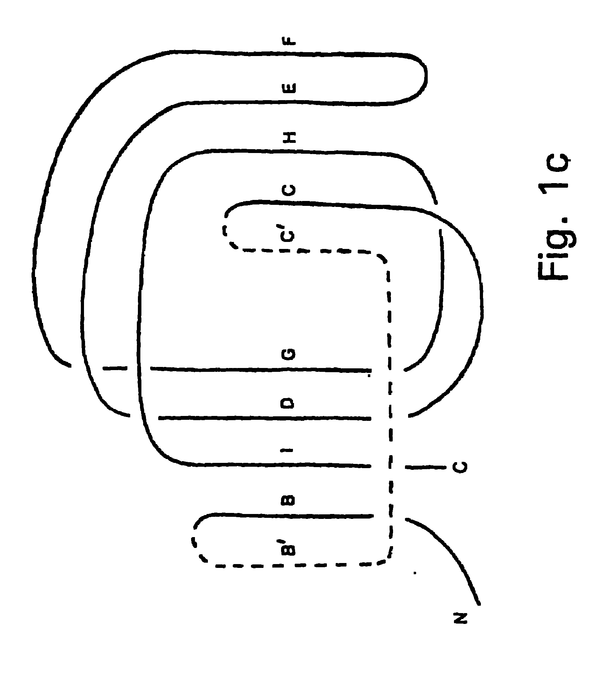

The overall shape of a single 157-amino-acid subunit of the TNFα trimer is wedgelike with a height of approximately 55Å and a maximum breadth of 35Å near the base. The main-chain topology is illustrated in Fig. 1a-c; it is essentially a β-sandwich structure formed by two antiparallel β-pleated sheets. The main-chainfold conforms to that of the classic jellyroll motif (Fig. 1c) (common in viral capsid protein) . The nomenclature adopted in Fig. 1 for the labels of the secondary structural units follows the established convention for viral structures. The standard eight β-strands (B to I) are all present but with an insertion between B and C that adds a short strand onto the edge of both β-sheets and truncates the N-terminal half of C, so that each β-pleated sheet contains five antiparallel β-strands, the back β-sheet comprising β-strands B', B, I, D and G and the front sheet comprising β-strands C', C, H, E and F.

The N terminus is highly flexible. This region, as far as residue 10 (see fig. 1b), is rather independent of the rest of the molecule, with the first few residues free to sample a variety of conformations in the solvent. In contrast, the C terminus is embedded in the base of the back β-sheet and forms an integral part of this relatively flat secondary structural unit. The gradation in β-strand lengths and the insertion between β-strands B and C conspire to produce a front surface formed almost entirely of loops, and it is this "masked" side of the β-sandwich, which in the trimer is presented to the solvent. The crystallographic data yield a measure of the relative flexibility of the various parts of the structure. The β-strands form a fairly inflexible scaffold; in particular, the back β-sheet is situated at the core of the trimer and consequently is particularly rigid. As would be expected, it is the loops that adorn the outer solvent-accessible surface of the molecule, which exhibit high levels of flexibility/mobility. Overall, there is a general decrease in rigidity as the core becomes more loosely packed in the upper half of the molecule.

### B. General Topology of the TNFα Trimer

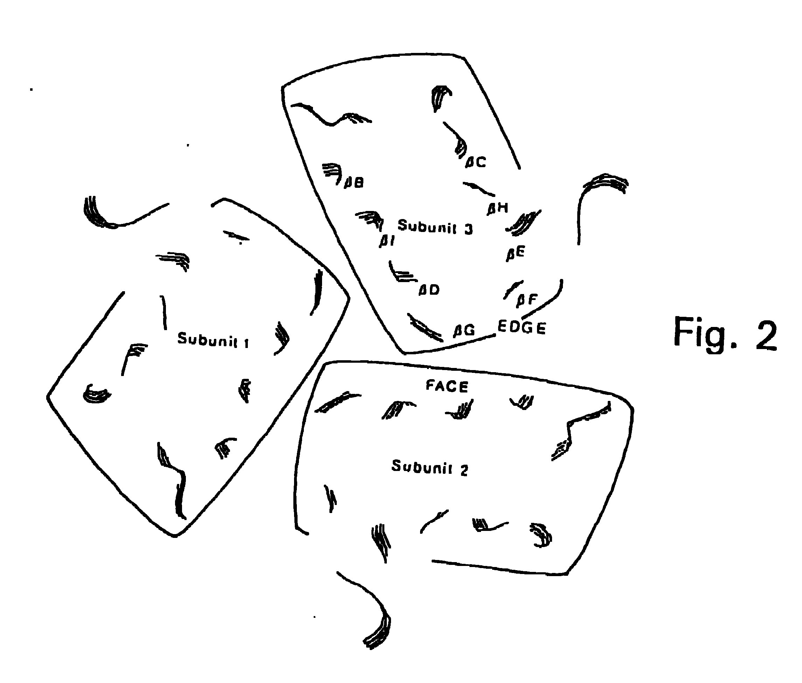

Three TNFα monomeric subunits associate noncovalently to form a compact, conical trimer having a length of about 55 Å and a maximum breadth of 50 Å. The β-strands of the three individual β-sandwiches lie approximately parallel (the tilt is about 30°) to the threefold axis of the trimer. The interaction between subunits related by the three-fold axis is through a simple edge-to-face packing of the β-sandwich; the edge of the β-sandwich, consisting of strands F and G from one subunit, lies across the back β-sheet [GDIBB'] of a threefold related subunit (see Fig. 2). The carboxy termini lie close to the threefold axis. The edge-to-face mode of packing produces an extremely tight association between the subunits. Thus the core of the trimer is completely inaccessible to solvent.

### C. Amino Acid-Type Distribution

The overall distribution of residue types in the three-dimensional structure of TNFα echoes the general rule for proteins: namely, that hydrophobic residues cluster in the core of the molecule while charged residues decorate the surface. Thus the core of the TNFα sandwich has the expected filling of tightly intercalating large apolar residues.

The energetics of the system do not favour the existence of TNFα in a monomeric state. For a large interface area composed of complementary residues (e.g., polar residues matched against polar residues) the loss of solvent-accessible surface area confers a considerable energetic advantage to formation of the oligomer (i.e., the trimer). The exposure to solvent of the large patch of strongly hydrophobic residues normally buried in the lower portion of the trimeric interface would also act to destabilize the TNFα monomer.

### 3. Probes of structure-function

### A. TNFα/Antibody Interactions

It has been observed that antibodies raised against TNFα from one species (e.g., human) do not cross-react with TNFα from another species (e.g., mouse) despite a sequence identity in excess of 80% and the ability of TNFα to bind to the TNFα receptors of other species. If the degree of sequence variation is mapped onto the three-dimensional structure, it is immediately apparent that the most sequence-variable regions of the molecule correspond to the antibody-accessible surface loops. The regions of highly conserved residues within the sandwich or at the trimeric interface are effectively invisible to antibodies. Thus the epitope for an antibody against TNFα will always contain some residues that will vary between species, thus abolishing antibody binding. This implies that the characteristics of the interaction between TNFα and its receptor must somehow differ from those required for binding of an antibody to TNFα.

### B. Site-Directed Mutagenesis

The role of various specific residues and regions of the TNFα molecule with regard to its biological (cytotoxic) activity and receptor binding has been probed by replacement of those residues by different amino acids or deletion of part of the sequence using the techniques of site-directed mutagenesis (Jones et al, op.cit. p. 113-119).

The deletion of up to eight residues from the N-terminus without any deleterious effect on biological activity serves to emphasize the nonessential nature of this region for overall molecular stability. N-terminal residues appear to exert an indirect, second-order effect on the biological efficacy of the TNFα trimer.

Non-conservative substitutions of the normally highly conserved residues which form the tightly packed core of the β-sandwich distort the structure and hence abrogate the biological activity of TNFα (Yamagishi et al., Protein Engineering, Vol. 3, No. 8, p. 713-719 (1989)). Many such mutated proteins (muteins) fail to form a stable, correctly folded molecule. Some conservative substitutions are permitted within the hydrophobic patch at the bottom of the threefold axis; however, there appears to be much greater leeway in the more loosely packed region near the top of the trimer. In particular, Cys 69 and Cys 101, which form the disulphide bridge between two connecting loops at the loosely packed top of the molecule, are relatively insensitive to changes (see Fig. 1a). Generally, however, in order to retain some biological activity of TNFα the mutations near the central axis of TNFα must be highly conservative, preserving the overall shape of TNFα.

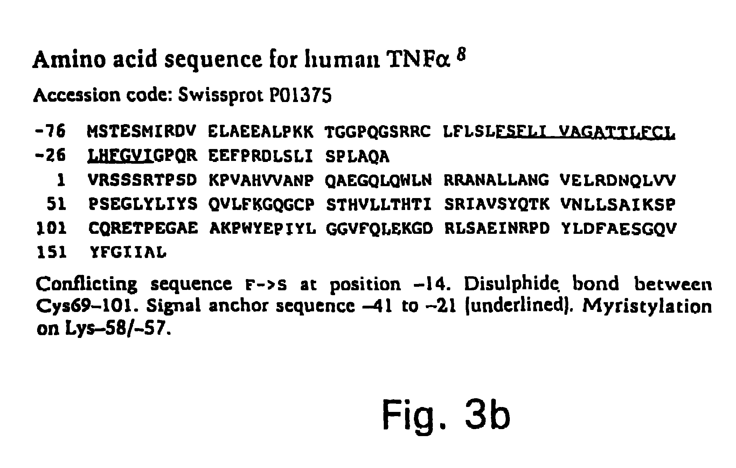

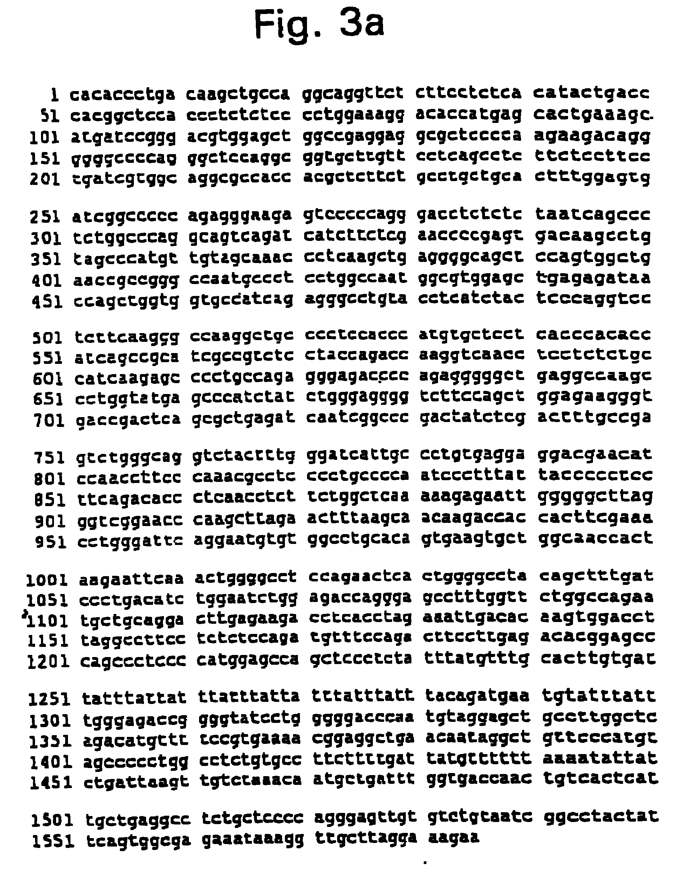

The residues on the surface of the molecule have a considerably greater freedom to mutate without incurring disastrous structural penalties as witnessed by the proliferation of variations of residues in this category between species. Thus drastic reductions in biological activity of TNFα due to substitutions in this area points to the direct involvement of such residues in the functional interaction of the TNFα trimer with its receptor. Residues comprising Arg 31, Arg 32 and Ala 33 situated in the connecting loop between the B and B' strand of the back β sheet, Ser 86 and Tyr 87 situated in the connecting loop between the E and F strands of the front β-sheet, and Glu 146 situated in the connecting loop between the H strand of the front β sheet and the I-strand of the back β-sheet appear to be such amino acid residues (see Fig.3). They appear to fall into two distinct "hotspot" regions on the front and the back sides of the TNFα monomer. The distribution of all deleterious mutations regardless of structural category further reinforces this picture. The existence of these "hot spots" for sensitivity of biological function to Mutation has been reviewed by Yamagishi et al., op.cit., and Goh et al. Protein Engineering, Vol. 4, No. 4, p. 385-389 (1991).

Studies on the mutation of human TNFα polypeptides have been reported in a number of patent applications.

Thus, Yamagishi et al. (EP 251 037) discloses numerous site-specific mutated TNFα molecules which are soluble and have the cytotoxic and antitumour activity characteristic for human TNFα in vitro and/or in vivo.

Other muteins with TNFα activity are described in WO 90/07579. Muteins with higher binding affinity for the human p75-TNF receptor than for human p55-TNF receptor are described in EP-A-619 372.

None of these citations discloses a modification of the TNFα-molecule in order to abrogate the biological activity of TNFα and provide the ability to induce antibodies against wild-type human TNFα.

However, they provide useful background information with regard to TNFα and its biological effect. Also production and expression of TNFα-analogs and their formulation are disclosed as are receptor-binding assays.

### 4. Summary

A rich variety of data may now be brought to bear on the specific relationship of structure to function for TNFα. All available evidence points to the importance of the trimer as the stable natural unit. It is apparent that the two hotspot regions situated on separate sides of the TNFα monomer are brought close to each other in terms of neighbouring subunits in the trimer. Thus a region of functional importance consisting of residues 31 to 35, 84 to 87, and 143 to 148 appears to be located at the interface between two subunits on the lower half of the trimer. Yamagishi et al. op.cit report loss of receptor binding ability as well as cytotoxicity for the mutation of Asp 143 to Tyr, and Tsujimoto et al., J. Biochem. 101, p. 919-925 (1987) report a similar effect for Arg 31 and Arg 32 to Asn and Thr. Thus the site may be associated directly with receptor binding as well as cytotoxicity. It is interesting that the receptor binding region of TNFα appears to lie at the interface between two subunits.

In summary, the detailed three-dimensional structure for TNFα serves to explain a wide range of observations on antibody binding, oligomerisation, and site-directed mutagenesis. When the structure is considered in combination with recent, extensive site-directed mutants, a region of biological importance with regard to receptor binding is apparently at the subunits on the lower half of the trimer.

### Description of the prior art

In WO 95/05849, some of the present inventors disclose a method for the Modification of self-proteins so as to induce antibody response against the unmodified self-protein, wherein a self-protein analog is provided by molecular biological means. More specifically one or more peptide fragments of the self-protein are substituted by one or more peptide fragments containing immunodominant, foreign T-cell epitopes.

Before the invention which lead to WO 95/05849, it was known to conjugate peptides or proteins, including self-proteins, with carrier proteins comprising a T-cell epitope. WO 92/19746 discloses a recombinant polypeptide comprising the LHRH sequence, one or more T-cell epitopes and a purification site, which is useful in animal immunocastration vaccines.

It is stated as preferable in WO 95/05849 that the immunodominant T-cell epitope is inserted so that flanking regions from the original protein comprising at least 4 amino acids are preserved on either side of the inserted epitope. In other words the epitope should not be conjugated with the self-protein as a fusion protein. Preferably, the substitution should be made so as to essentially preserve the tertiary structure of the original protein. Apart from that no specific guidance is provided as to the optimal intramolecular position of the inserted epitope in order to create the most powerful antibody response against the unmodified self-protein. Presumably, this will vary from self-protein to self-protein but based on the general guidance in the specification the most appropriate position(s) can be determined without undue experimentation by selecting peptides comprising appropriate immunodominant epitopes, exchanging peptide sequences of essentially the same length in various parts of the self-protein molecule and determining the raised antibody response by suitable assay techniques.

It is probably very difficult by using current modelling tools, to predict whether substitution of one or more peptide fragments in a protein results in a change in the tertiary structure of the original protein. Therefore, in a drug development program's search for lead compounds, it must be considered as a standard screening procedure early in the development phase to evaluate which modified self-proteins have preserved the tertiary structure of the original protein. This can be done using several experimental techniques as have been described in numerous text books on protein characterization, e.g. fluorescence spectroscopy, near-UV circular dichroism, Fourier transformed infrared spectroscopy and multidimensional NMR techniques ("Physical Methods to Characterise Pharmaceutical Proteins", Pharmaceutical Biotechnology Vol. 7. Eds. J.N. Herron, W. Jiskoot & D.J.A. Crommelin, Plenum Press, New York (1995)). Ideally, in a screening process for lead compounds, two or more of the experimental techniques mentioned above should be combined in order to evaluate whether a change in the tertiary structure has occurred or not.

### Brief description of the drawings

Figure 1(a) illustrates the crystal structure of native TNFα monomer. The figure is a diagrammatic sketch of the subunit fold, β strands are shown as thick arrows in the amino-to-carboxy direction and connecting loops are depicted as thin lines. The disulfide bridge is denoted by a lightning flash and a region of high flexibility is crosshatched. The trimer threefold axis would be vertical for this orientation.
Figure 1(b) is a Cα chain trace of the THFα monomer crystal structure. This detailed representation should be used in conjunction with Figure 1(a) to give the precise alignment of the amino acid sequence with the clearer but stylized representation of the subunit fold.
Figure 1(c) shows the TNFα structure as a jelly roll motif. The insertion between β strands B and C is shown in dashed lines; the connection between B and C would run straight across at the top of the molecule.
Figure 2 shows the edge-to-face packing of β sandwiches in the TNF trimer. The view, down the threefold axis, shows a narrow slab of the trimer with β strands represented by ribbons running into and out of the page.
Figure 3(a) illustrates the DNA sequence encoding tumor necrosis factor (TNFα) having the amino acid sequence shown in Fig. 3(b)
   The DNA sequence is available from Gen Bank under accession no. M10988, SEQ ID NO:339737.
   The sequence has been described by Wang et al., Science 228, 149-154 (1985). The sequence includes codons encoding the -76--1 presequence of human TNFα.
   The complete gene sequence including introns has been described by Nedwin et al., Nucleic Acids, Res. 13(17) 6361-6373 (1985), Shirai et al., Nature 313(6005), 803-806 (1985) and Dennica et al., Nature 312 (5996), 724-729 (1984).
Figure 3 (b) shows the amino acid sequence of human TNFα including the -76--1 presequence.

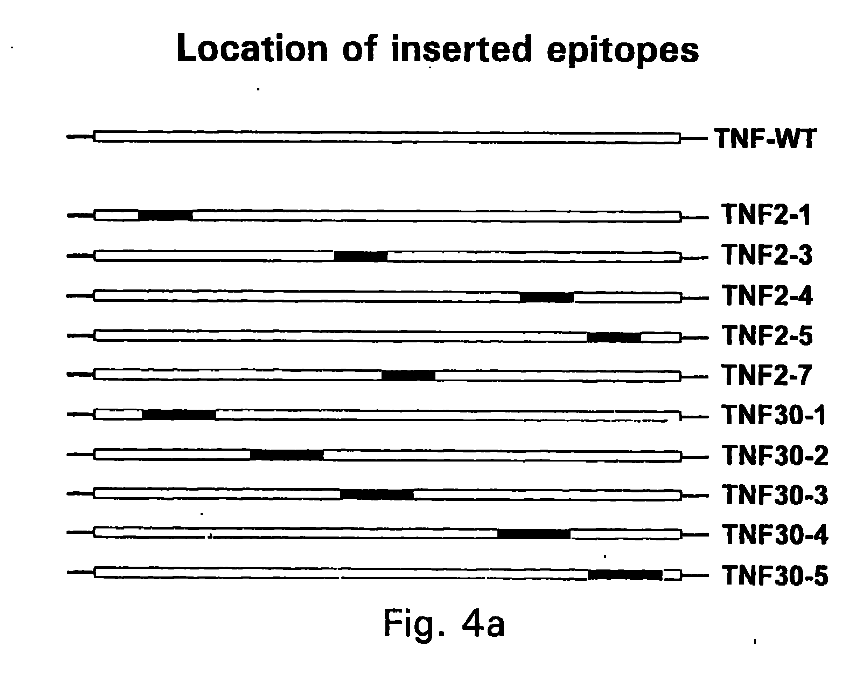

Figure 4 (a) schematically illustrates the substitutions of immunodominant epitopes P2 and P30 in a wild type TNFα (WT) to form the TNFα analogs TNF2-1 to 2-7 and TNF30-1 to 30-5.

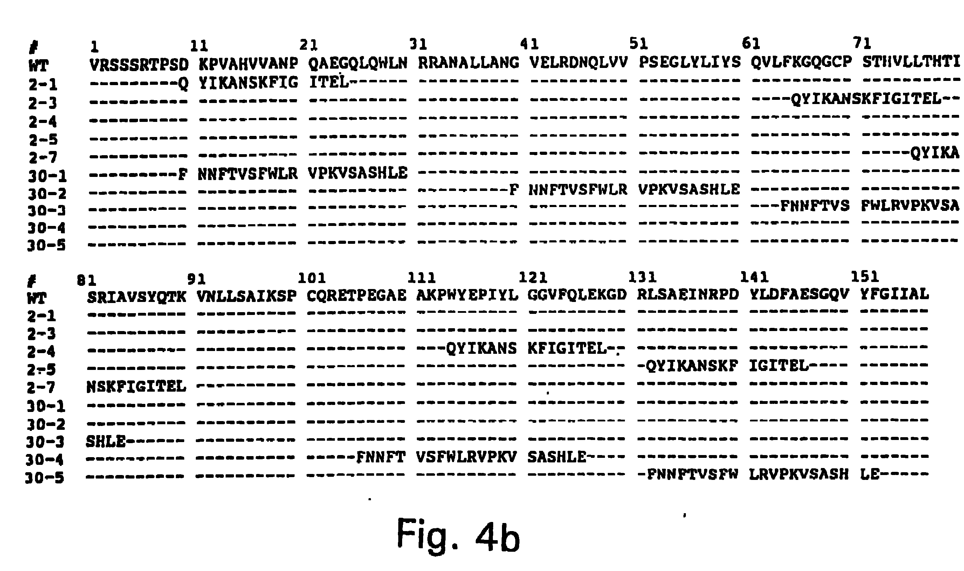

Figure 4(b) shows the exact locations of the substitutions in the WT sequence for the individual TNFα analogs.

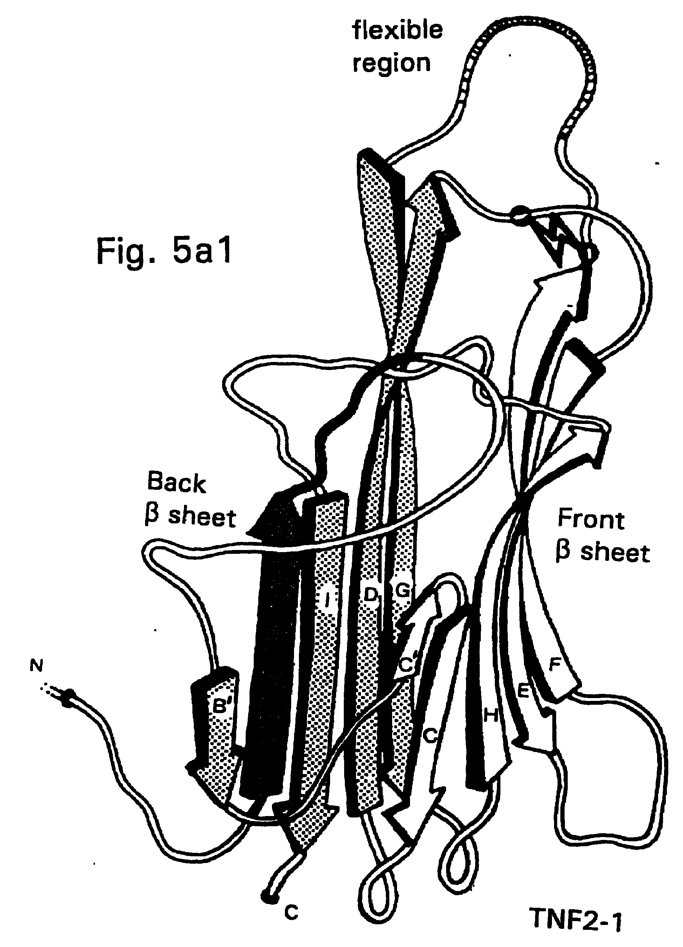

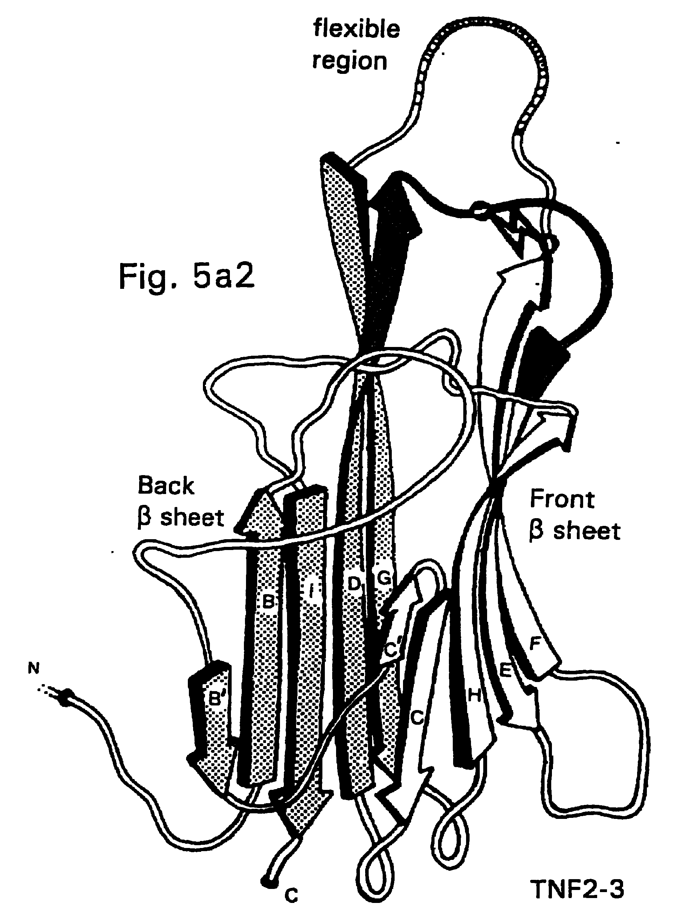

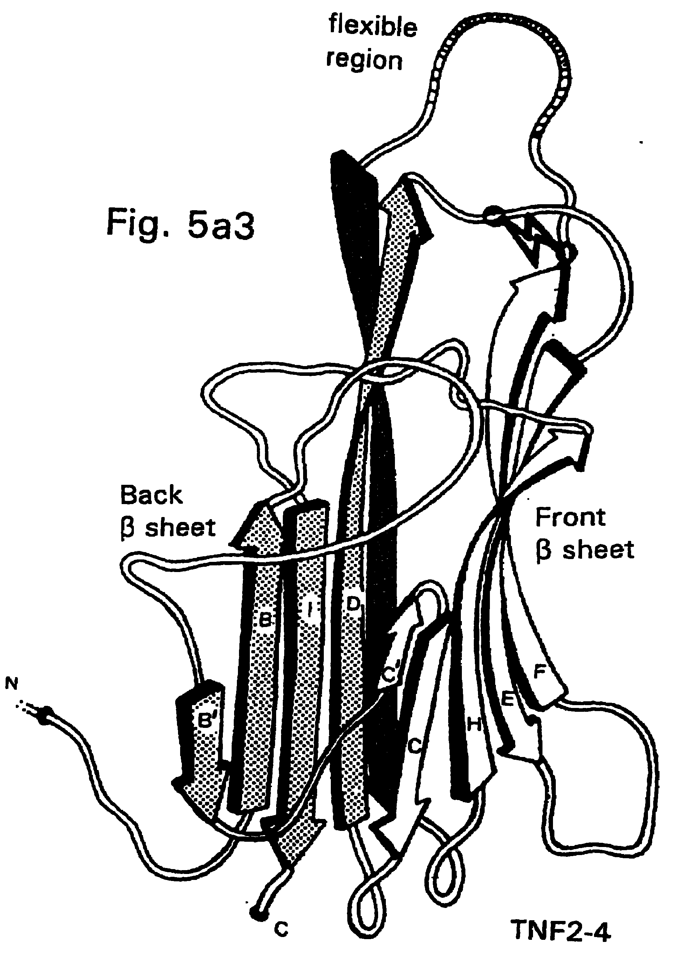

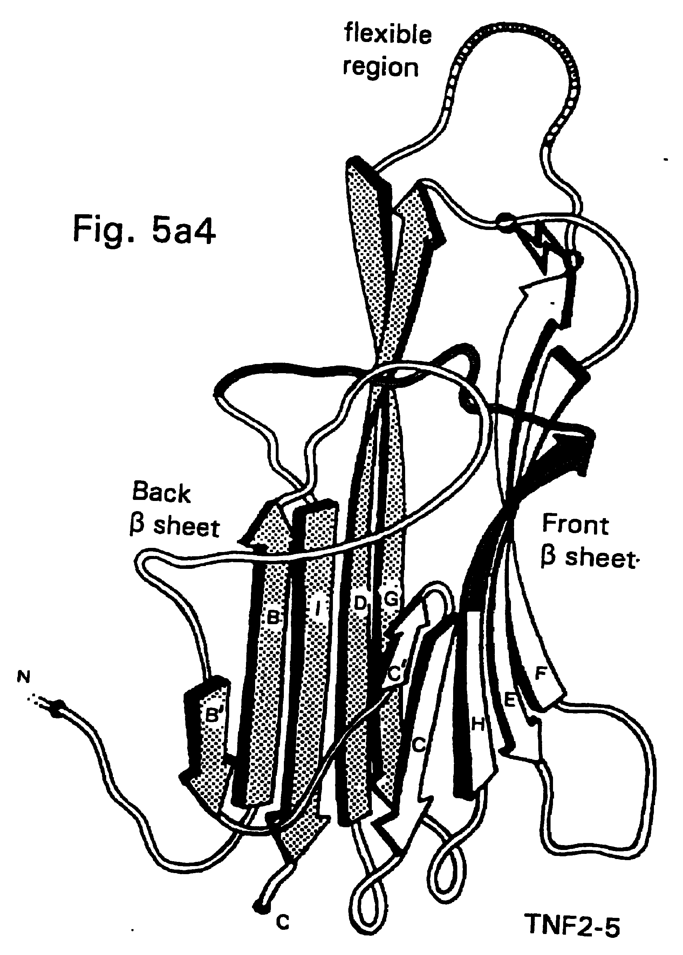

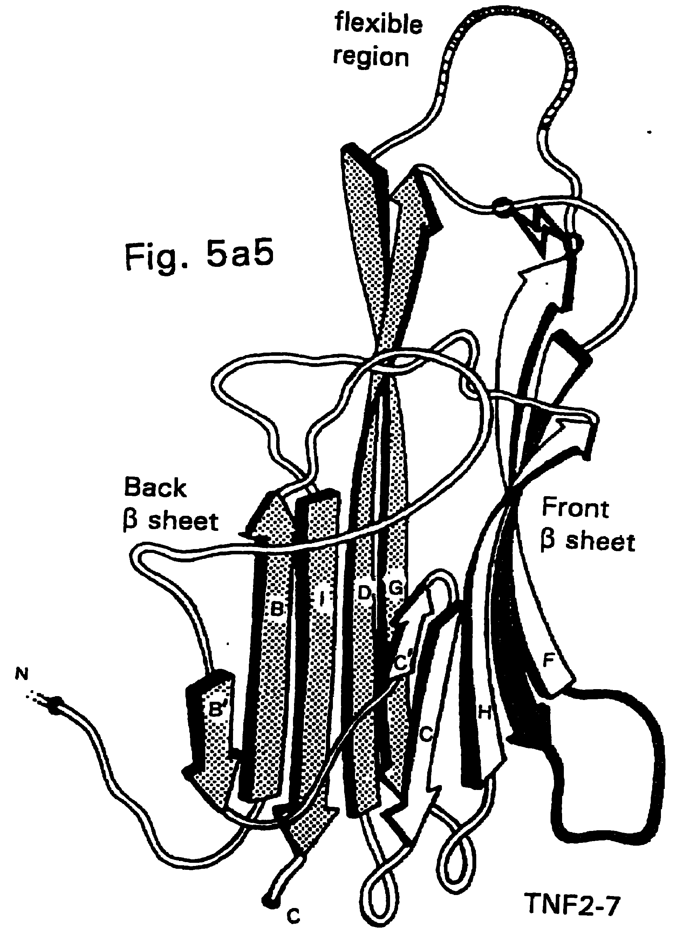

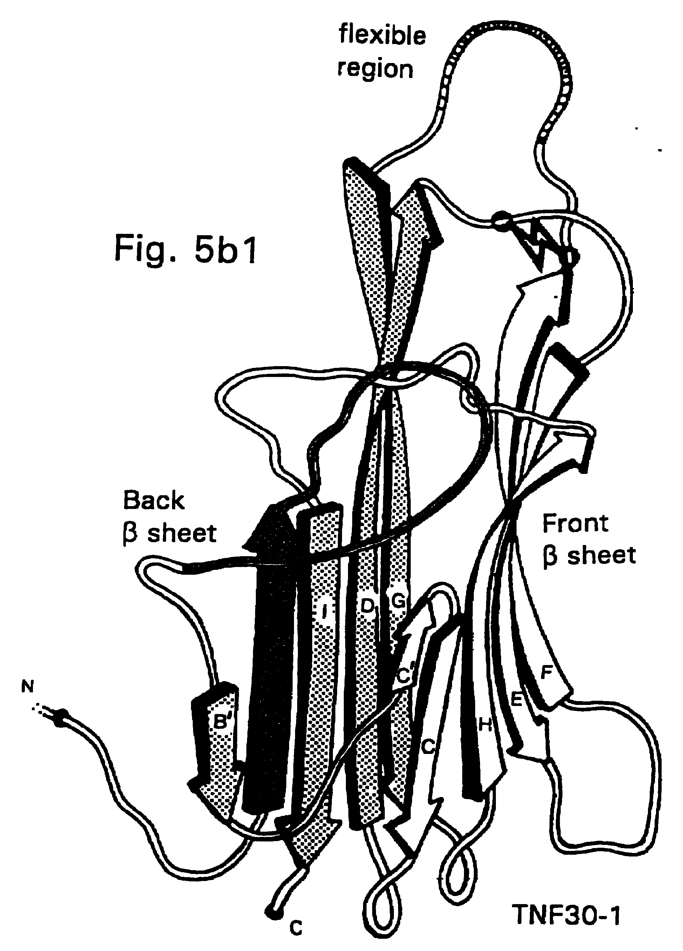

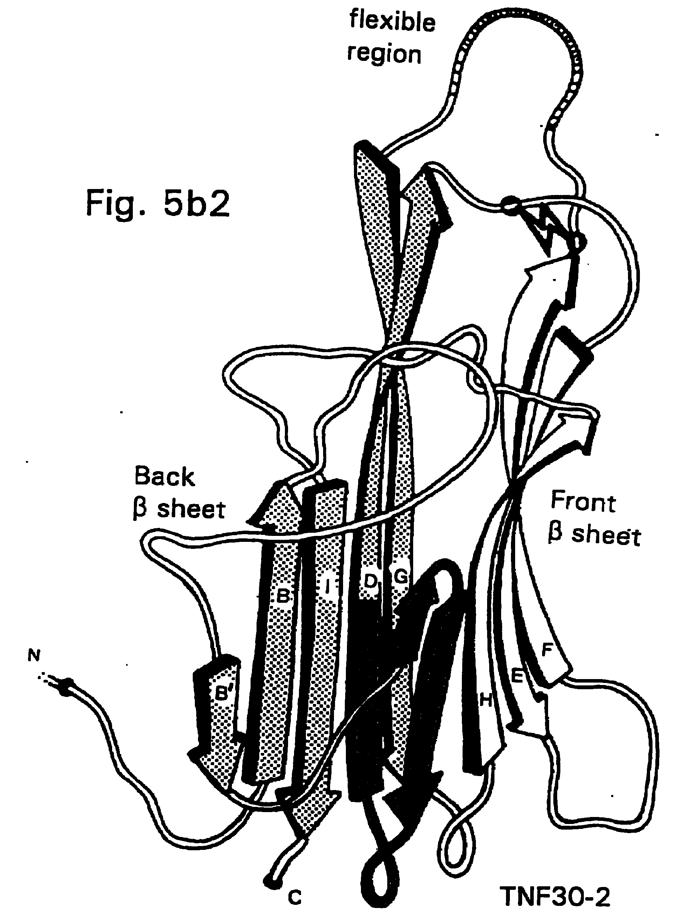

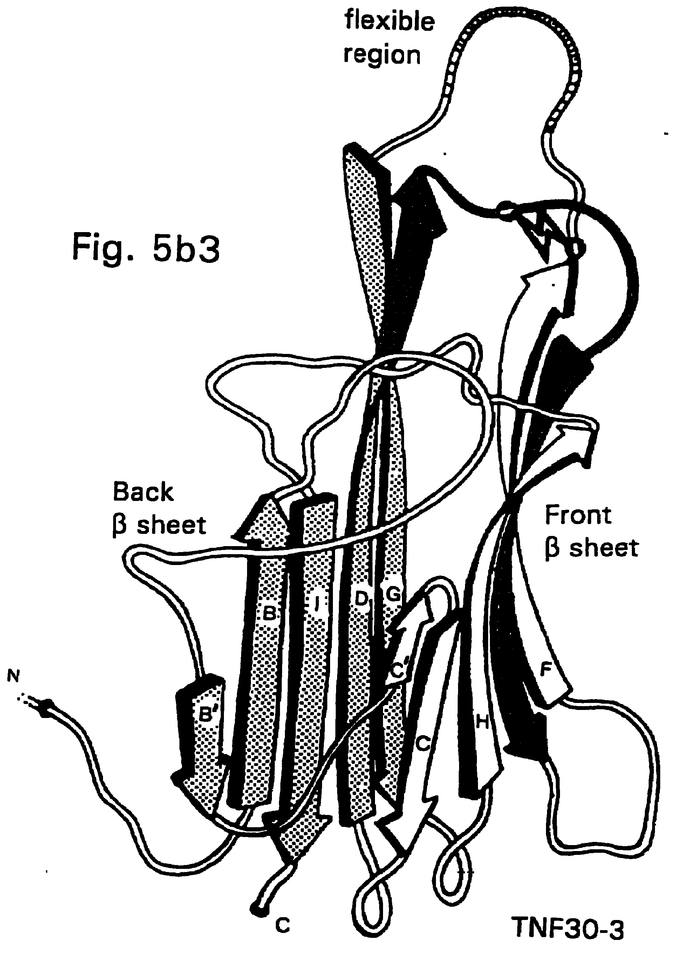

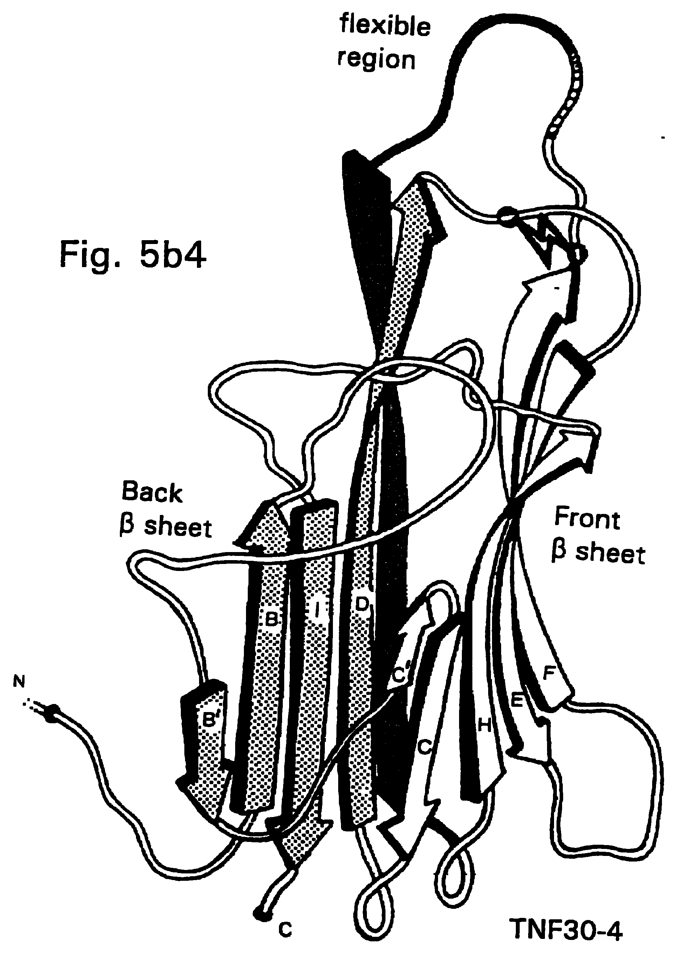

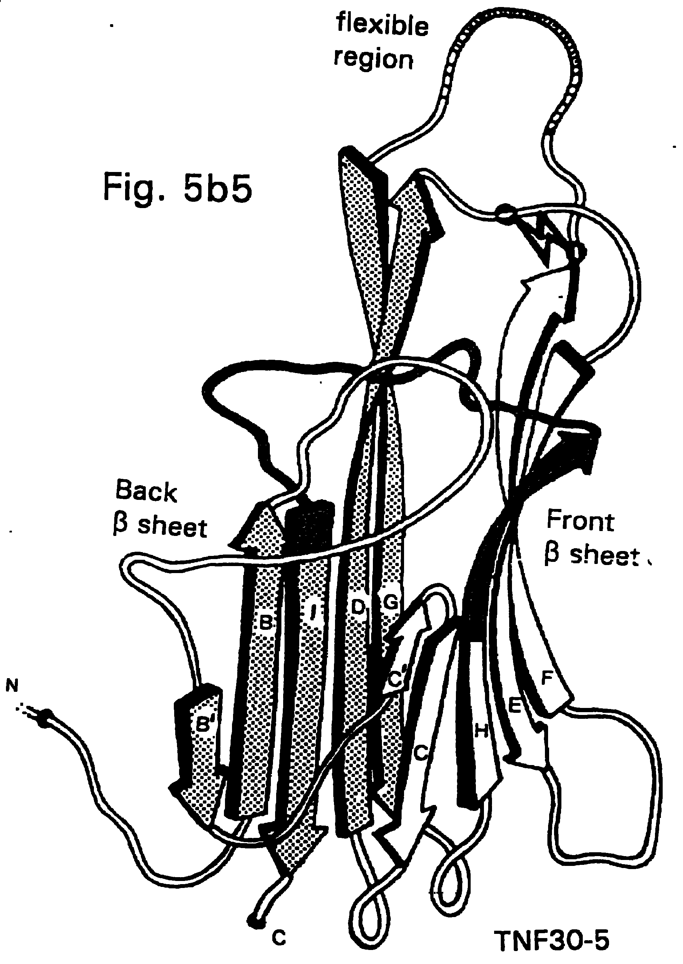

Figures 5(a) and 5(b) show the structure of the analogs based on Fig. 1(a), where the individual substitutions by P2 and P30 in the strands of the β-sheets and the connection loops, respectively, are marked in black.

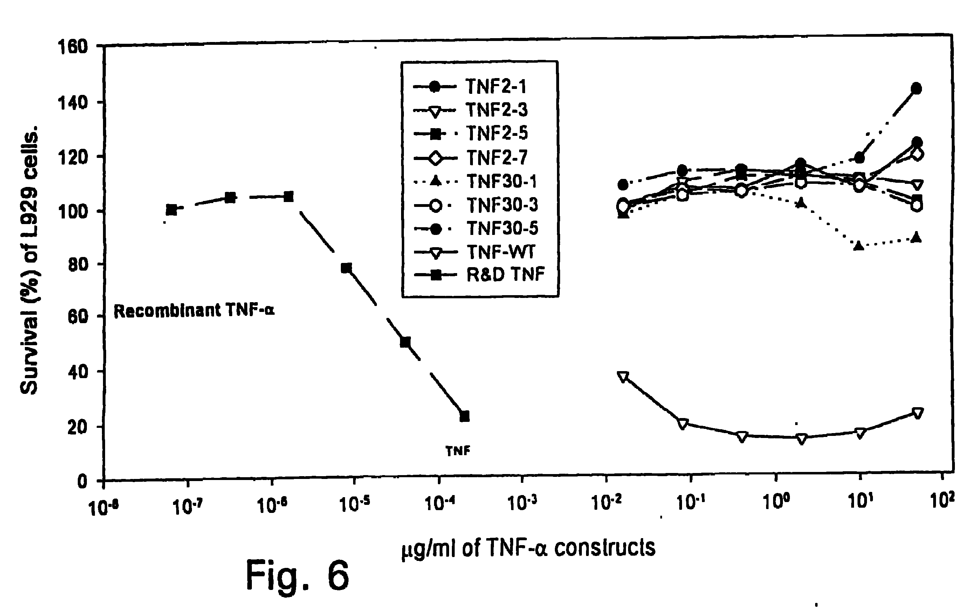

Figure 6 shows the biological activity of the TNFα analogs in the L929 assay (Meager, A., Leung, H., & Woolley, J. Assays for Tumour Necrosis Factor and related cytokines., J. Immunol. Meth. 116, 1-17 (1989)) compared with recombinant TNFα.

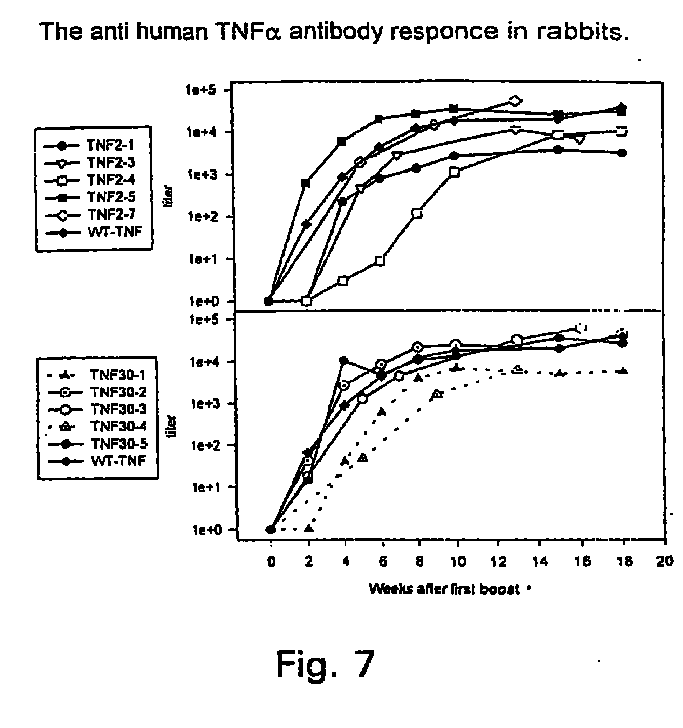

Figure 7 shows the anti-human TNFα antibody response in rabbits to vaccination with the modified TNFα molecules in rabbits.

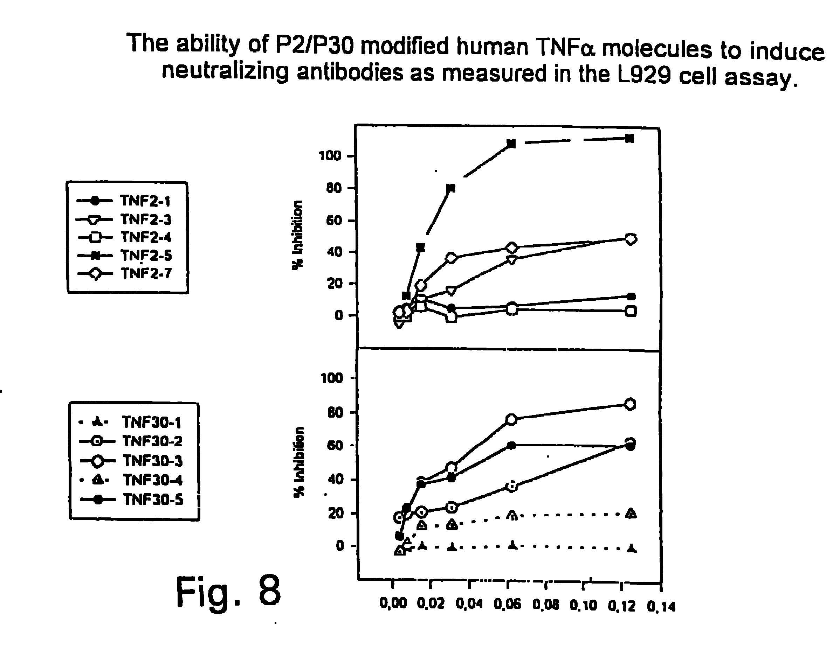

Figure 8 shows the ability of P2/P30 modified human TNFα molecules to induce neutralizing antibodies as measured in the L929 cell assay.

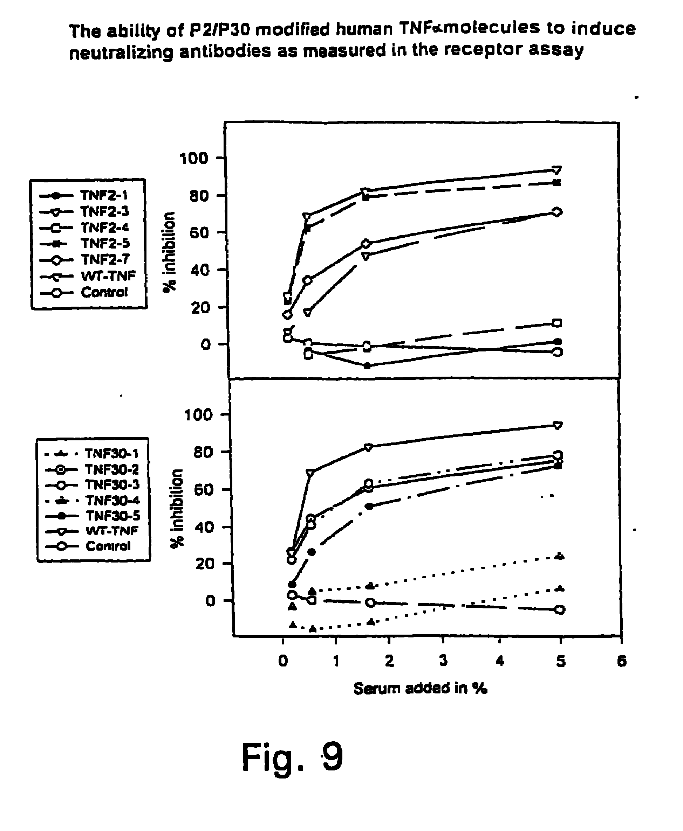

Figure 9 shows the ability - when administered to rabbits - of P2/P30 modified human TNFα molecules to induce neutralizing antibodies as measured in receptor assay.

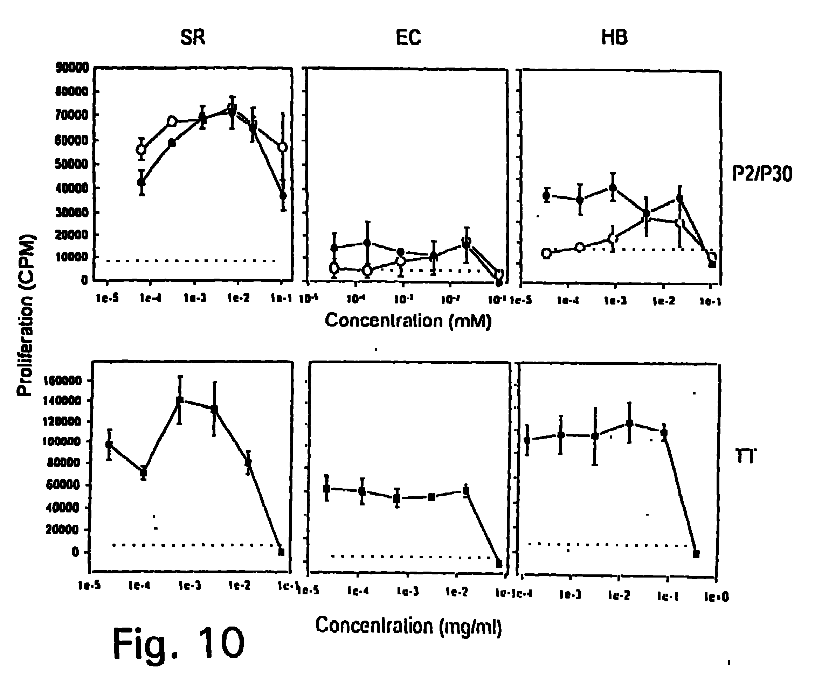

Figure 10 shows the Peripheral Blood Mononuclear Cell (PBMC) response in three donors towards TT and the P2 and P30 peptides.

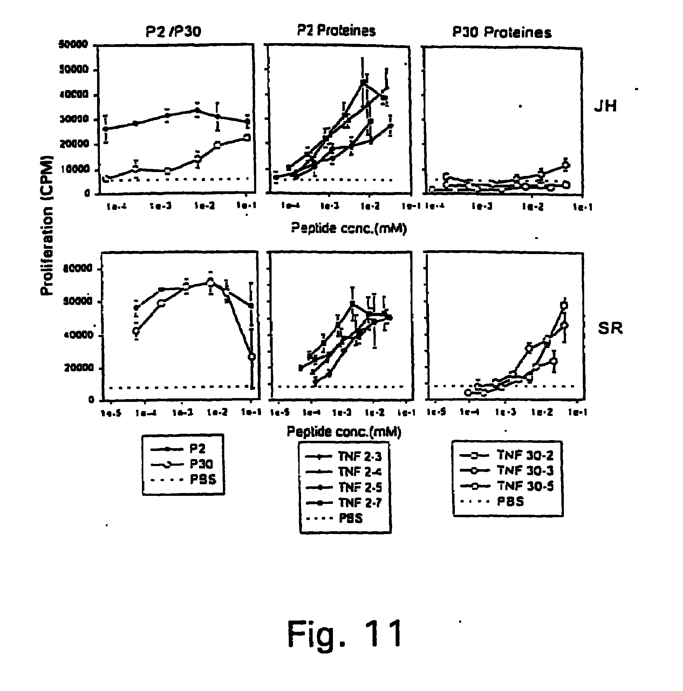

Figure 11 shows the polyclonal proliferation response in two donors using the different P2 and P30 modified TNFα molecules.

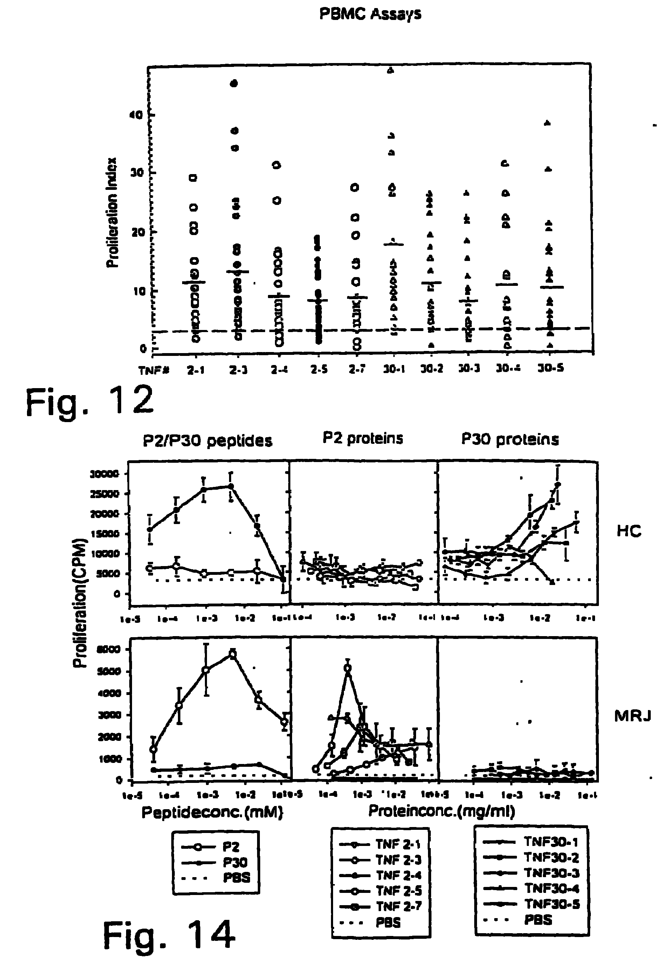

Figure 12 shows the Proliferation Indexes(PIs) calculated from 34 experiments for the P2 and P30 modified TNFα molecules.

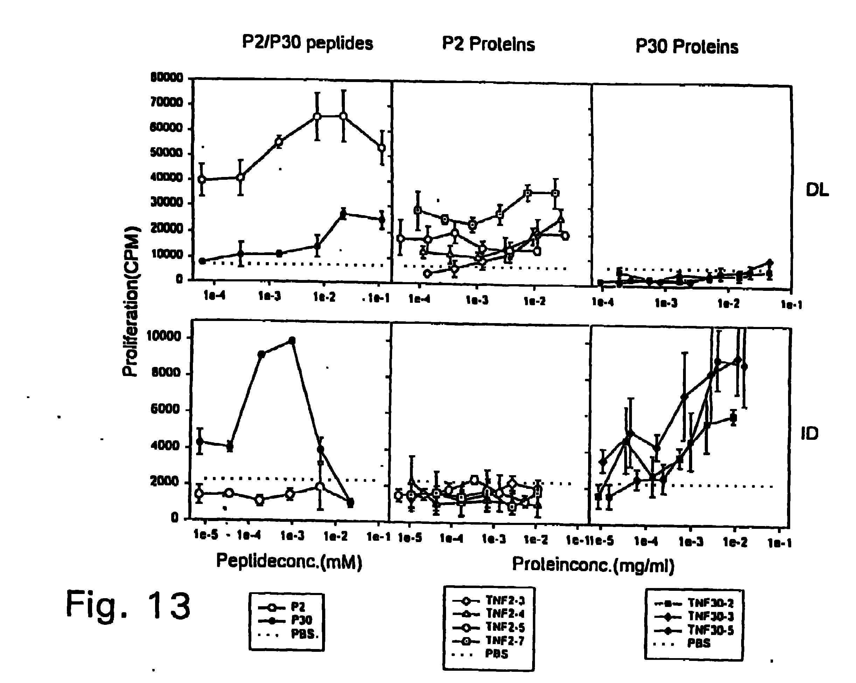

Figure 13 shows the PBMC response against P2 and P30 modified TNFα proteins in P2 and P30 specific responders, respectively.

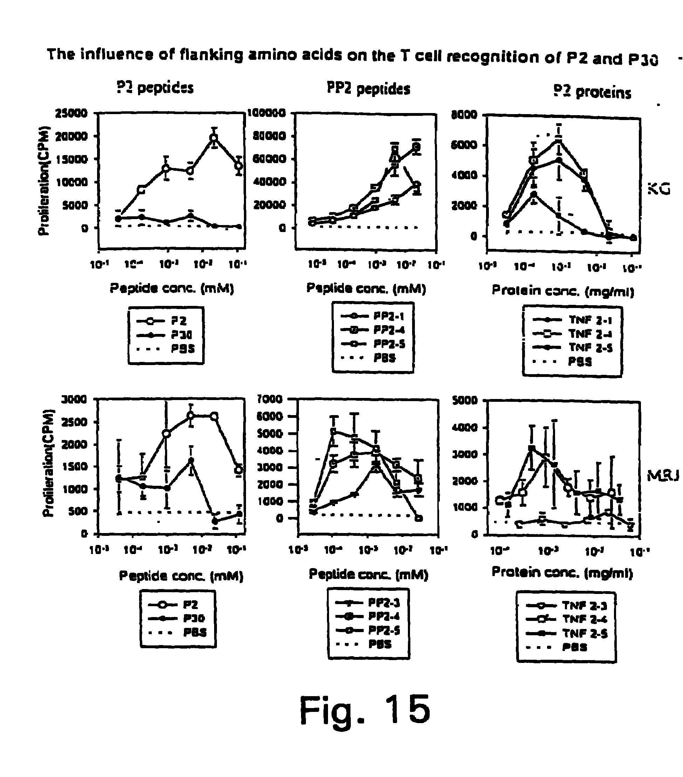

Figure 14 shows a similar PBMC response in two other donors.
Figure 15 shows the influence of flanking amino acids on the T cell recognition of P2 and P30.

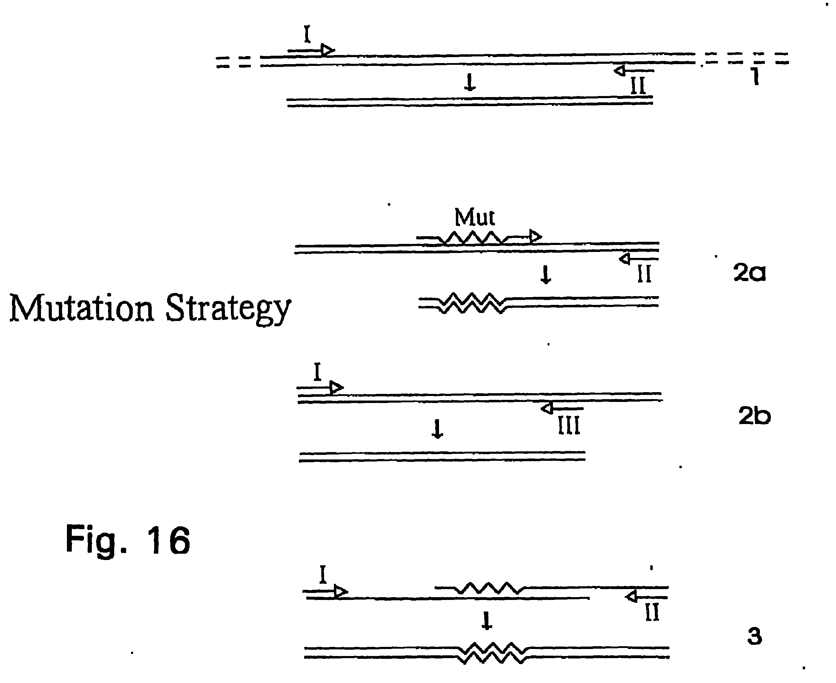

Figure 16 shows the mutation strategy used for the preparation of the modified TNFα molecules.

### Summary of the invention

The purpose of the present invention is to provide guidelines as to how a particular self-protein within the general scope of the above-mentioned WO 95/05849, viz. human TNFα should be modified in order to be biologically inactive as well as be able to induce a strong neutralizing antibody response towards wild-type, biologically active TNFα. In the present context "biologically inactive" refers to the activities of the wild-type TNFα, mainly its cytotoxic activity.

From the discussion of the tertiary structure of TNFα given above, it is recalled that the biologically active TNFαis a trimer of three subunits. Due to the "edge-to-face" packing the "back β-sheet" represents the "hidden" area of contact between the subunits which is completely inaccessible to solvent. Significant substitutions in this area will almost inevitably deprive the TNFα molecule of all biological activity. On the contrary the "front β-sheet" and the connecting regions provide the accessible surface area which includes the areas interacting with the TNFα receptors. Antibodies towards these areas would therefore probably be able to interfere with receptor binding and would hence possess TNFα neutralizing properties.

A person skilled in the art who wanted to construct a detoxified and yet immunogenic TNFα molecule according to WO 95/05849 would therefore as the first choice insert the immunodominant T cell epitope in the back β-sheet of the TNFα monomer. Modifications of this area would thus most probably interrupt the biological activity of TNFα and leave the receptor-accessible front β-sheet free for interaction with antibodies. This is also consistent with the discussion of the site-directed mutagenesis in the tightly packed core of the β-sandwich discussed above.

However, surprisingly it is not so. As it will appear from the test results below, the result was quite the contrary, since substitutions involving the B and G strands of the back β-sheet surprisingly provided TNFα analogs which were unable to induce neutralizing antibodies against TNFα.

Thus, the present invention seeks to provide a modified human TNFα molecule capable of raising neutralizing antibodies towards wild-type human TNFα following administration of said modified TNFα molecule to a human host, wherein at least one segment of the human TNFα molecule has been substituted by at least one peptide known to contain an immunodominant T cell epitope, or a truncated form of said molecule containing an immunodominant T-cell epitope and one or both flanking regions of the human TNFα molecule comprising at least one TNFα B cell epitope, wherein the substitution is introduced in anyone of the strands of the front β sheet, in anyone of the connecting loops and/or in any one of the B', I or D strands of the back β-sheet, wherein the substitution has been made in regions of the TNFα molecule so as to preserve the β-sheet structure of the B and G strands, and wherein the substitution leads to inactivation of the biological activity of human TNFα when tested in the L929 bioassay.

Thus, the substitution goes beyond a mere conservative substitution of the individual amino acids, and beyond the point mutations which do not alter the secondary and/or tertiary structure of the wild-type TNFα. In other words, the T cell epitope Introduced in the TNFα sequence should preferably introduce a sequence which is of very low homology with the wild-type TNFα sequence.

Hence, the present invention provides for a modified human TNFα molecule capable of raising neutralizing antibodies towards wild-type human TNFα following administration of said modified TNRα molecule to a human host, wherein at least one peptide fragment of the human TNRα molecule has been substituted by at least one peptide known to contain an immunodominant T cell epitope or a truncated form of said molecule containing an immunodominant epitope and one or both flanking regions of the human THFα-molecule comprising at least one TNFα B cell epitope, wherein said modified TNFα molecule is substantially free from TNFα activity.

The term "substantially free from TNFα activity" is in the present context intended to mean that the administration to a human being of such a modified TNFα molecule will not result in significant adverse effects due to the known cytokine effects exerted by native TNFα. In other words, the modified TNFα molecules of the invention are pharmaceutically acceptable substances.

In order to test whether the modified human TNFα molecule according to the invention is substantially free from TNFα activity, the L929 bioassay as defined herein can be applied. Furthermore, in order to confirm the immunogenic character of the modified TNFα molecules, antibodies raised against the modified TNFα molecule in a suitable host will significantly inhibit the activity of wild-type TNFα in the L929 bioassay as defined herein, and/or said antibodies will significantly inhibit the binding of wild-type TNFα to the 55 kD TNFα receptor 1 (TNFα-R55) or the to the 75 kD TNFα receptor (TNFα-R75).

These suitable hosts can for instance by a primate such as a Rhesus or Cynomuleus monkey, a rodent such a rat or mouse or guinea pig, or a lagomorph such as a rabbit.

The assays suitable for evaluating the potential of the modified molecules according to the invention are carried out using antibody or antiserum in a concentration relative to the assay setup and ought to reflect physiological conditions with respect to the involved reactants, or it ought to he possible to extrapolate to physological conditions from the results obtained from the assay. In other words, the results from the assay must indicate that a physiological concentration of antibodies against TNFα in vivo is able to reduce the TNFα activity to an extent of at least 10%, 15%, 25%, 30%, 35%, 40%, 45%, 50%, 55%, 60%, 65%, 70%, 75%, 80%, 85%, 90%, 95% or 100%. The person skilled in the art will readily know how to determine such conditions.

It is to be understood that a "significant inhibition of the wild-type TNFα." may suitably be such which results in reduction or elimination of the adverse effects of wild-type TNFα. Such inhibition may suitably be at least 10%, at least 15%, at least 25%, at least 30%, at least 35%, at least 40%, at least 45%, at least 50%, at least 55%, at least 60%, at least 65%, at least 70 , at least 75%, at least 80%, at least 85%, at least 90%, at least 95% or even 100%.

On this background the modified human TNFα molecules according to the present invention are characterized in that the substitution has been made in regions of the TNFα molecule, which involves the strands of the front β-sheets and/or the connecting loops so as essentially preserve the β-sheet structure of any of the strands of the back β-sheet.

Although it has not yet been fully verified experimentally, it must be assumed that this property is common to the strands forming the back β-sheet, so that preferably the substitutions should he made in regions of the TNFα molecule which do not comprise any complete strand of the back β-sheet. In view of the discussion above of the functional importance of the residues 31-35 it can be assumed that the connecting loops between the individual strands of the back β-sheet should preferably also be avoided.

However, it is permissible that the substitution is made in regions of the TNFα molecule which only involve a segment of the D strand of the back β-sheet.

According to a preferred embodiment of the invention the substitution comprises at least a segment of the H strand of the front β-sheet and of the connecting loop to the I strand of the back β-sheet, preferably amino acids 132 to 146. According to another embodiment of the invention the substitution comprises segments of the H and I strands and the entire connecting loop, preferably amino acids 132 to 152. According to yet another presently preferred embodiment of the invention the substitution comprises a segment of the D strand of the back β-sheet, at least a segment of the E strand of the front β-sheet and the entire connecting loop, preferably amino acids 65 to 79 or 64 to 84.

According to a further embodiment of the invention the substitution comprises the entire C' and C strands of the front β-sheet and a segment of the D strand of the back β-sheet, preferably ainino acids 40 to 60.

According to a still further embodiment of the invention the substitution comprises at least a segment of the E strand of the front β-sheet and of one or both of the connecting loops, preferably amino acids 76 to 90.

The inserted T cell epitope should preferably be promiscuous and known to be immunogenic in a majority of human HLA class II types. Applicable epitopes can be derived e.g. from Tetanus toxoid, preferably epitope P2 and/or P30, Panina-Bordignon et al., Eur. J. Immunol. 19:2237-42, 1989. Also epitopes derived from diphtheria toxoid may be used.

The preferred modified human TNFα molecules (TNFα analogs) as referred to above with reference to the location of the substitution are shown in the enclosed sequence listing as SEQ ID NO:8 and SEQ ID NO:16.

Other applicable TNFα analogs are listed as SEQ ID NO:4, 10, 14 and 16.

The invention also relates to truncated analogs of the above-mentioned modified TNFα analogs according to the present invention. Thus, truncated analogs of TNFα molecules containing a promiscuous and immunodominant T cell epitope and one or both flanking regions comprising at least one TNFα B cell epitope, preferably comprising at least five amino acids, would also constitute a possible active ingredient in a TNFα vaccine according to the invention. The T cell epitope would induce the proliferation of T cells when presented to MHC class II molecules by the APC, while the B cell epitope would potentially be recognized by the immunoglobulin receptors on B cells, and would subsequently be presented by MHC class I molecules on these cells. This constitutes the basis for raising an immune response towards wild-type TNFα, harbouring the B cell epitope, according to WO 95/05849 and the present invention.

B cell epitopes could be identified in the TNFα both theoretically and experimentally. Algorithms for identification of potential linear B cell epitopes have been published, and this would form the basis of an experimentally based investigation of the nature of these potential epitopes. Antibodies raised in a heterologous system (e.g. rabbits) in response to injections of such truncated TNFα molecules comprising T cell epitopes could be analyzed for *in vitro* capability to bind native human TNFα, preferentially in neutralizing manner. Panels of monoclonal antibodies known to be neutralizing could be screened *in vitro* for capability to bind the potential B cell epitopes of TNFα. Both of these are strategies for identifying the possible and best B cell epitopes.

The above-mentioned TNFα-analogs are believed to remain in monomer form, since the modification probably destroys the inherent trimerization tendency of the wild-type TNFα.

However, the invention further relates to dimers, oligomers, especially trimers or multimers of the claimed modified TNFα molecules provided the are devoid of TNFα-activity and also isolated DNA molecules that code for the claimed modified TNFα molecules.

The isolated DNA molecules encoding the preferred TNFα analogs have the sequences listed as SEQ ID NO:7 and 15, and the DNA molecules encoding the other applicable analogs are listed as SEQ ID NO:3, 9, 13 and 15.

The invention further comprises vectors comprising the isolated DNA molecules encoding the analogs and expression vectors comprising said DNA molecules operatively linked to a suitable expression control sequence.

Another aspect of the invention is a host transformed with an expression vector for said analog.

Said host may be any of the hosts commonly used for expression, e.g. a strain of bacteria, yeast or fungi or insect, mammalian or avian cell lines.

The invention further relates to a method of producing the claimed TNFα analogs, whereby host cells transformed with an expression vector for the analog is grown under suitable conditions permitting production of the analog, and the produced analog is recovered.

If desired, the modified TNFα molecules according to the invention may be expressed as or form part of a fusion protein with a suitable adjuvant molecule, preferably an immunologically active adjuvant, such as GM-CSF, HSP70 or an interleukin, e.g. interleukin 2.

More specifically, the modified TNFα molecules are produced by substituting the appropriate gene segments encoding peptide containing or constituting immunodominant T cell epitopes into the gene encoding the native human TNFα molecule. Subsequently the modified TNFα gene is expressed in an appropriate eukaryotic or prokaryotic expression vector. The expressed modified TNFα molecules are purified and refolded as described below.

Although it may be preferred to insert the whole T cell epitope, it may in some cases be advantageous to insert a peptide sequence comprising both the epitope as well as flanking regions from the protein from which the epitope is derived.

According to the invention the modified human TNFα molecules may be used in vaccines against TNFα. Strategies in formulation development of vaccines based on purified proteins, such as modulated self-proteins, generally correspond to formulation strategies for any other protein-based drug product. Potential problems and the guidance required to overcome these problems - as for instance preservation of tertiary structure - are dealt with in several textbooks, e.g. "Therapeutic Peptides and Protein Formulation. Processing and Delivery Systems" Ed. A.K. Banga; Technomic Publishing AG, Basel 1995. The use of an adjuvant, e.g., aluminium hydroxide, aluminium phosphate (Adju-Phos), calcium phosphate, muramyl dipeptide analog, or some of the more recent developments in vaccine adjuvants such as biodegradable microparticles and Iscom's is a formulation challenge familiar to a pharmaceutical scientist working in this area.

Preparation of the vaccines according to the invention which contain peptide sequences as active ingredients is generally well understood in the art, as exemplified by U.S. Patents 4,608,251; 4,601,903; 4,599,231; 4,599,230; 4,596,792; and 4,578,770. Typically, such vaccines are prepared as injectables either as liquid solutions or suspensions; solid forms suitable for solution in, or suspension in, liquid prior to injection may also be prepared. The preparation may also be emulsified. The active immunogenic ingredient is often mixed with excipients which are pharmaceutically acceptable and compatible with the active ingredient. Suitable excipients are, for example, water, saline, dextrose, glycerol, ethanol, or the like, and combinations thereof. In addition, if desired, the vaccine may contain minor amounts of auxiliary substances such as wetting or emulsifying agents, pH buffering agents, or adjuvants which enhance the effectiveness of the vaccines.

The vaccines are conventionally administered parenterally, by injection, for example, either subcutaneously or intramuscularly. Additional formulations which are suitable for other modes of administration include suppositories and, in some cases, oral formulations. For suppositories, traditional binders and carriers may include, for example, polyalkylene glycols or triglycerides; such suppositories may be formed from mixtures containing the active ingredient in the range of 0.5 to 10, preferably 1-2%. Oral formulations include such normally employed excipients as, for example, pharmaceutical grades of mannitol, lactose, starch, magnesium stearate, sodium saccharine, cellulose, magnesium carbonate, and the like. These compositions take the form of solutions, suspensions, tablets, pills, capsules, sustained release formulations or powders and contain 10-95 of active ingredient, preferably 25-70%.

The polypeptides may be formulated into the vaccine as neutral or salt forms. Pharmaceutically acceptable salts include acid addition salts (formed with the free amino groups of the peptide) and which are formed with inorganic acids such as, for example, hydrochloric or phosphoric acids, or such organic acids as acetic oxalic, tartaric, mandelic, and the like. Salts formed with the free carboxyl groups may also be derived from inorganic bases such as, for example, sodium, potassium, ammonium, calcium, or ferric hydroxides, and such organic bases as isopropylamine, trimethylamine, 2-ethylamino ethanol, histidine, procaine, and the like.

The vaccines are administered in a manner compatible with the dosage formulation, and in such amount as will be therapeutically effective and immunogenic. The quantity to be administered depends on the subject to be treated, including, e.g., the capacity of the individual's immune system to mount an immune response, and the degree of protection desired which in turn depends on the level of TNFα in the patient. Suitable dosage ranges are of the order of several hundred micrograms active ingredient per vaccination with a preferred range from about 0.1 µg to 1000 pg, such as in the range from about 1 µg to 300 µg, and especially in the range from about 10 µg to 50 µg. Suitable regimens for initial administration and booster shots are also variable but are typified by an initial administration followed by subsequent inoculations or other administrations.

The manner of application may be varied widely. Any of the conventional methods for administration of a vaccine are applicable. These are believed to include oral application on a solid physiologically acceptable base or in a physiologically acceptable dispersion, parenterally, by injection or the like. The dosage of the vaccine will depend on the route of administration and will vary according to the age of the person to be vaccinated and, to a lesser degree, the size of the person to be vaccinated.

Some of the modified TNFα molecules according to the present invention are sufficiently immunogenic in a vaccine, but for some of the others the immune response will be enhanced if the vaccine further comprises an adjuvant substance.

Various methods of achieving adjuvant effect for the vaccine include use of agents such as aluminum hydroxide or phosphate (alum), commonly used as 0.05 to 0.1 percent solution in phosphate buffered saline, admixture with synthetic polymers of sugars (Carbopol) used as 0.25 percent solution, aggregation of the protein in the vaccine by heat treatment with temperatures ranging between 70° to 101°C for 30 second to 2 minute periods respectively. Aggregation by reactivating with pepsin treated (Fab) antibodies to albumin, mixture with bacterial cells such as C. parvum or endotoxins or lipopolysaccharide components of gram-negative bacteria, emulsion in physiologically acceptable oil vehicles such as mannide mono-oleate (Aracel A) or emulsion with 20 percent solution of a perfluorocarbon (Fluosol-DA) used as a block substitute may also be employed. According to the invention DDA (dimethyldioctadecylammonium bromide) is an interesting candidate for an adjuvant, but also QuilA and RIBI are interesting possibilities. Further possibilities are monophosphoryl lipid A (MPL), and muramyl dipeptide (MDP).Other suitable adjuvants are aluminium hydroxide, aluminium phosphate (Adju-Phos), calcium phosphate, muramyl dipeptide analog. Some of of the more recent developments in vaccine adjuvants such as biodegradable microparticles and Iscom's may also suitably be used.

Another highly interesting (and thus, preferred) possibility of achieving adjuvant effect is to employ the technique described in Gosselin *et al.,* 1992. In brief, the presentation of a relevant antigen such as a modified TNFα molecule of the present invention can be enhanced by conjugating such antigen to antibodies (or antigen binding antibody fragments) against the Fcγ receptors on monocytes/macrophages. Especially conjugates between antigen and anti-FcγRI have been demonstrated to enhance immunogenicity for the purposes of vaccination.

Other possibilities involve the use of immune modulating substances such as lymphokines (e.g. IFN-γ, IL-2 and IL-12) or synthetic IFN-γ inducers such as poly I:C in combination with the above-mentioned adjuvants.

In many instances, it will be necessary to have multiple administrations of the vaccine, usually not exceeding six vaccinations, more usually not exceeding four vaccinations and preferably one or more, usually at least about three vaccinations. The vaccinations will normally be at from two to twelve week intervals, more usually from three to five week intervals. Periodic boosters at intervals of 1-5 years, usually three years, will be desirable to maintain the desired levels of protective immunity.

One reason for admixing the polypeptides of the invention with an adjuvant is to effectively activate a cellular immune response. However, this effect can also be achieved in other ways, for instance by expressing the effective antigen in a vaccine in a non-pathogenic microorganism. A well-known example of such a microorganism is *Mycobacterium bovis* BCG.

It is preferred that the non-pathogenic microorganism is a bacterium, e.g. selected from the group consisting of the genera *Mycobacterium, Salmonella*, *Pseudomonas* and *Eschericia*. It is especially preferred that the non-pathogenic microorganism is *Mycobacterium bovis.*

The incorporation of one or more copies of a nucleotide sequence encoding the molecule according to the invention in a mycobacterium from a *M. bovis* BCG strain will enhance the immunogenic effect of the BCG strain. The incorporation of more than one copy of a nucleotide sequence of the invention is contemplated to enhance the immune response even more, and consequently an aspect of the invention is a vaccine wherein at least 2 copies of a DNA sequence encoding a molecule is incorporated in the genome of the microorganism, such as at least 5 copies. The copies of DNA sequences may either be identical encoding identical molecule or be variants of the same DNA sequence encoding identical or homologues of a polypeptide, or in another embodiment be different DNA sequences encoding different polypeptides where at least one of the polypeptides is according to the present invention.

The living vaccine of the invention can be prepared by cultivating a transformed non-pathogenic cell according to the invention, and transferring these cells to a medium for a vaccine, and optionally adding a carrier, vehicle and/or adjuvant substance.

Apart from their use as starting points for the synthesis of molecule of the invention and for hybridization probes (useful for direct hybridization assays or as primers in e.g. PCR or other molecular amplification methods) the nucleic acid fragments of the invention may be used for effecting *in vivo* expression of antigens, *i.e*. the nucleic acid fragments may be used in so-called DNA vaccines. Recent research have revealed that a DNA fragment cloned in a vector which is non-replicative in eukaryotic cells may be introduced into an animal (including a human being) by e.g. intramuscular injection or percutaneous administration (the so-called "gene gun" approach). The DNA is taken up by e.g. muscle cells and the gene of interest is expressed by a promoter which is functioning in eukaryotes, e.g. a vital promoter, and the gene product thereafter stimulates the immune system. These newly discovered methods are reviewed in Ulmer et al., 1993, which hereby is included by reference.

The efficacy of such a "DNA vaccine" can possibly be enhanced by administering the gene encoding the expression product, together with a DNA fragment encoding a polypeptide which has the capability of modulating an immune response. For instance, a gene encoding lymphokine precursors or lymphokines (e.g. IFN-γ, IL-2, or IL-12) could be administered together with the gene encoding the immunogenic protein, either by administering two separate DNA fragments or by administering both DNA fragments included in the same vector.

The vaccines may be used for preventing/treating any of the diseases described above, the pathophysiology of which is characterised by TNFα release, in particular chronic inflammatory diseases. As examples can be mentioned rheumatoid arthritis and inflammatory bowel diseases (IBD). The latter includes ulcerative colitis and Crohn's disease, in particular Crohn's colitis. Other examples are cancer, cachexia, often related to cancer, disseminated sclerosis, diabetes, psoriasis, osteoporosis and asthma. Cancers, which can preferably be treated or prevented according to the invention can be histogenetically classified as malignant epithelial tumours, including carcinomas and adenocarcinomas, and as malignant non-epithelial tumours, including liposarcomas, fibrosarcomas, chondrosarcomas, osteosarcomas, leiomyosarcomas, rhabdomyosarcomas, gliomas, neuroblastomas, medulloblastomas, malignant melanoma, malignant meningioma, various leukemias, various myeloproliferative disorders, various lymphomas (Hodgkin's lymphoma and non-Hodgkin lymphoma), haemangiosarcoma, Kaposi's sarcoma, lymphangiosarcoma, malignant teratoma, dysgerminoma, seminoma, and choricarcinoma.

Carcinomas and adenocarcinomas are the most abundant (accounting for approximately 90% of deaths from cancer) and are therefore interesting target diseases to treat/prevent according to the invention. The most important carcinomas and adenocarcinomas are those of the airways (espially of bronchial origin), of the breast, of the colorectum and of the stomach. However, also carcinomas and adenocarcinomas of the prostate, the ovary, of the lymphoid tissue and bone marrow, of the uterus, of the pancreas, of the esophagus, the urinary bladder, and the kidney cause a significant number of deaths and are therefore of interest.

Preferably the vaccines will be given as a preventive treatment, but in view of the chronic nature of these diseases and their tendency to remission and recurvency, they may also be administered to patients, where one or more of the above-mentioned diseases has been diagnosed, and may serve to maintain the patient in a state of remission.

Based on earlier studies in mice, it is believed that the modified TNFα analogs according to the invention can also be administered as part of a curative treatment of the above-mentioned diseases in an acute state or at least with a view to bringing the patient in remission and maintain a steady state condition.

At present no specific effective dose range can be stated, since the vaccines have not yet been tested in human beings susceptible to any of the diseases.

At any rate the administered dose will be prescribed by the responsible doctor.

According to one embodiment of the invention the vaccine comprises a mixture of two differently modified TNFα molecules containing two different T cell epitopes e.g. P2 and P30 which are derived from tetanus toxoid. This mixture optionally contains appropriate amounts of a pharmaceutically acceptable adjuvant.

According to yet another aspect of the invention, the vaccines do not comprise the modified human TNFα molecules as such, but rather a construct comprising non-infectious non-integrating DNA sequence encoding said molecules operatively liked to a promoter sequence which can control the expression of said DNA sequence in humans, in an amount sufficient that uptake of said construct occurs, and sufficient expression occurs to induce a neutralizing antibody response against TNFα.

The utility of this type of vaccines, the so-called DNA vaccines, is illustrated e.g. in US patents nos. 5.589.466 and 5.580.859, in particular in relation to the methods of administration.

The DNA vaccines may comprise a viral expression vector, such as a retroviral expression vector.

Generally, the vaccines according to the invention may be adapted for oral or parenteral, in particular subcutaneous, intramuscular or intradermal administration.

The vaccines/recombinant proteins can be characterized using the following assays familiar to a person skilled in the art:
SDS-Page gels stained with coomassie or silver (information on size and purity),
IEF (Isoelectric focusing) (information on isoelectric point),
Endotoxin LAL assay (information on purity),
Host cell protein (information on purity),
Mass spectroscopy (information on molecular mass),
SE-HPLC with UV detection profile (information on molecule weight distribution),
N-terminal sequence (information on identity),
Circular Dichroism (information on tertiary structure),
SE-HPLC with malls detection (information on tertiary structure by light scattering),
Amino acid composition (information on identity),
Immunogenecity in heterologous species (mouse, rat or rabbit),
Reactivity to antibodies in Western Blots,
NMR spectroscopy,
Crystal structure,
Complete amino acid sequence determination.

### EXPERIMENTAL PART WITH DESCRIPTION OF PREFERRED EMBODIMENTS

### 1. Introduction

In the previous application WO 95/05849 it was shown that T cell epitope modified murine TNFα molecules could induce high titers of antibodies cross-reactive with native (wild-type) murine TNFα. These antibodies were able to interfere with TNFα and its receptor *in vitro* as well as *in vivo*. Beneficial effects of immunization against TNFα were demonstrated in several animal models of TNFα-induced disease such as experimental cachexia, collagen arthritis and experimental allergic encephalomyelitis (EAE). These animal experimental results were obtained despite the fact that the two modified murine TNFα molecules used (denominated MR 103 and MR 106) were not optimized to be immunogenic in the MHC class II haplotypes of DBA/1 and SJL mice. These mouse strains were used for the collagen arthritis and the EAE experiments, respectively. In another experiment a differently modified murine TNFα molecule (MR 105) was shown to provide a stronger immune response that the recombinant murine TNFα conjugated to E.coli proteins using formaldehyde.

MR 103, MR 105 and MR 106 were mouse molecules and based on the prior application WO 95/05849 no specific conclusions could be drawn with regard to the immunogenicity of appropriately T cell substituted human modified TNFα molecules nor about the potential ability of such molecules to induce TNFα neutralizing autoantibodies, since they were all active.

### 2. Development of human TNFα constructs

In general terms in a TNFα vaccine for human use the modified human TNFα molecules should fulfill the following requirements:
a. They should be immunogenic in a large proportion of the population
b. They should be optimally able to induce TNFα neutralizing antibodies
c. They should not possess any remaining biological TNFα activity

Furthermore, in a selection process other practical parameters such as levels of recombinant expression, ease of purification, solubility etc. could also be considered.

### 2.1. Immunological promiscuity of the modified TNFα molecules

During the development of the human TNFα vaccine the aim was to produce modified human TNFα molecules which eventually will be immunogenic in the largest possible part of the human population which of course represents a large number of different HLA class II types. Therefore, instead of the MHC specific epitopes used in the previous animal experiments, promiscuous T cell epitopes were used. It was not known from the previous application WO 95/05849 how such epitopes could influence the capability of such molecules to induce neutralizing antibodies.

The two tetanus toxoid (TT) derived T cell epitopes, P2 and P30, which have been well characterized in the scientific literature were chosen. These epitopes are known to be immunodominant in TT and to be able to bind to at least 80 % of the HLA class II molecules in the human population.

Furthermore, by using these TT epitopes it was expected to be possible to test the immunogenicity of the TNFα constructs *in vitro* on peripheral blood mononuclear cells (PBMC) and T cell lines generated from TT immune blood donors.

The amino acid sequence of the P2 epitope is QYIKANSKFIGITEL and corresponds to TT amino acids 830-844, and the sequence of the P30 epitope is FNNFTVSFWLRVPKVSASHLE and corresponds to TT amino acids 947-967. Substituting P2 and P30 into two different human TNFα molecules would exchange approximately 10 % and 15 %, respectively, of the native TNFα sequence. In case both epitopes were inserted into a single TNFα molecule, about 25 % of the molecule would be exchanged, and one could fear that this would interfere too much with the remaining native parts of the TNFα molecule. It was therefore decided to develop two TNFα molecules, each containing either P2 or P30. Together, such two molecules would be expected to be immunogenic in at least 80 % of the human population. In addition, it is very likely that truncated molecules composed partly of the P2 or P30 epitope and partly of TNFα flanking regions also will contribute to the immunogenicity resulting in the constructs being immunogenic in almost 100 % of the population.

Although it was possible to induce antibodies with all murine TNFα constructs in all mouse strains tested so far, cf. discussion of MR 103, 105 and 106 above, one would *a priori* expect, that insertion of the foreign T cell epitope at certain positions in TNFα would be more beneficial than other positions with regard to the presentation of the epitope to T cells by MHC class II molecules. It was therefore decided to produce an array of differently modified human TNFα molecules with the P2 and P30 epitope inserted at different positions in the molecule, see fig 4.. Subsequently, all molecules were tested *in vitro* in T cell assays based on peripheral blood mononuclear cells (PBMC) or P2/P30 specific T cell lines isolated from a number of healthy TT immune blood donors.

Contrary to what was expected, however, it was shown that although minor quantitative differences were seen, the intramolecular position of the P2 and P30 epitopes was not essential for the ability to be processed by antigen presenting cells and subsequently presented to TT specific T cells. Thus, P2 inserted in positions 132 to 146, 65 to 79 and 76 to 90 and P30 inserted in positions 40 to 60 and 132 to 152 (TNF2-5, 2-3, 2-7, 30-2, 30-5) were all processed and presented to T cells. So from the discussion above it is very likely, that these molecules eventually will be universally immunogenic in the human population.

### 2.2. The ability of the modified human TNFα molecules to induce neutralizing antibodies

As mentioned above, it was not possible from the previous mouse studies described in WO 95/05849 to predict which position would be most appropriate in order to be able to induce neutralizing TNFα antibodies since the three analogues MR 103, MR 105 and MR 106 were all able to induce antibodies despite their different areas of substitution. An array of different human TNFα molecules with P2 or P30 inserted at different positions was therefore produced. The substitutions were randomly distributed over the entire molecule. The antibodies induced in rabbits upon injection of these molecules were subsequently tested in biochemical as well as biological *in vitro* assays for their ability to interfere with TNFα biological activity.

It has been shown in non-published observations by the present inventors that depending on the intramolecular position of the inserted epitope a different overall specificity of the induced autoantibodies was observed. Quite contrary to what would be expected based on the structural data of the TNFα molecule it was observed, that substitutions made in the front β-sheet with either P2 or P30 totally deprived the molecules of biological TNFα activity, but at the same time preserved the ability of the modified molecules to induce TNFα neutralizing antibodies.

The molecules containing P2 or P30, in the positions mentioned above, were shown to be particularly effective at inducing neutralizing antibodies. Any of these molecules are therefore potential candidates for use in human TNFα vaccines.

### 2.3 The biological activity of the different TNFα constructs

It would obviously not be feasible to use a molecule which is as toxic as TNFα in a vaccine. The modified TNFα molecules would therefore have to be non-toxic i.e. devoid of any residual TNFα activity.

All mutant TNFα proteins were there fore tested *in vitro* in TNFα dependent bioassays as well as receptor binding assays in order to examine whether they are non-toxic. It was shown clearly that the modified human TNFα molecules (TNF2-5, 2-3, 2-7, 30-2, and 30-5,) all were deprived of TNFα biological activity. So all the necessary requirements of these molecules to be part of a universally, non-toxic vaccine capable of inducing anti-human TNFα neutralizing antibodies were fulfilled.

### EXAMPLE 1

### Genetic construction work

It was decided to produce 10 different modified human TNFα molecules - five containing the P2 and five containing the P30 epitope. The epitopes were distributed at different positions within the molecule. The genetic constructions were made by using various standard restriction enzyme and PCR based mutagenesis techniques. The genetic constructs are shown schematically in Fig. 4a and 4b. DNA sequences encoding the modified TNFα molecules and the corresponding amino acid sequences are incorporated as SEQ ID NO:1 - SEQ ID NO:20. The constructions of the mutant human TNF2-5 gene, the cloning and mutation strategy, and the subsequent expression, isolation and purification of the TNF2-5 analog is explained below by way of example.

### Construction and production of TNF2-5:

### Genetic construction of the mutant human TNF2-5 gene, cloning and mutation strategy.

The genetic construction of the gene encoding the mutant human TNF2-5 analog was based upon traditional PCR based mutagenesis techniques, as were all the other genetic constructions.

The native DNA sequence of human TNFα encoding the soluble part of this molecule was obtained by traditional PCP cloning using synthetically synthesized primers I and II (table 1 and SEQ ID NO:21 and 22) from a human commercially available cDNA library, CLONTECH Laboratories, Palo Alto, CA, USA (Fig. 16,1). The native gene was inserted into a commercial *E. coli* expression vector pET28c available from Novagen, Madison, WI 53711, USA, in such a way that the gene could be transcribed in frame from an IPTG inducible promoter.

The genetic construction of the TNFα mutant analog TNF2-5 was performed by a PCR mutagenesis technique applied to the native DNA sequence. The nucleic acid sequence encoding the T cell epitope was incorporated into a synthetically synthesized 75-mer oligonucleotide (Primer "mut2-5", table 1 and SEQ ID NO:27) between the two 3'- and 5'-annealing stretches of TNF homologous DNA, the "mut" primer is thus capable of annealing to the native human TNFα gene sequence at the defined site selected for TNF2-5, (see Fig 16, 2a). In the "mut" oligonucleotide the number of codons encoding the T cell epitope exactly matched the number of TNF-codons omitted between the two 3'- and 5'- annealing stretches of TNF homologous DNA. The mutagenesis primer was used to produce a PCR product containing the DNA encoding the T cell epitope and the TNFα sequence below (or 3') to the inserted epitope, (Fig. 16, 2a). The stretch of TNFα DNA above (or 5') to the point of insertion of the epitope was provided by a second PCR product using primers I and III (Table 1, SEQ ID NO:23) (Fig. 16, 2b). The two PCR products are eventually joined together in a final PCR reaction, (Fig. 16, 3) using the two most distal primers, (I, II) from the two reactions. The complete mutant TNF2-5 DNA sequence is then introduced into a commercial *E.coli* expression vector in analogy to the expression cloning of the native gene in such a way that the gene could be transcribed from an IPTC inducible promoter from transformed cells.

The "mut" primers used for construction of the other analogs (TNF2-1, 2-3, 2-4, 2-7, 30-1, 30-2, 30-3, 30-4 and 30-5) are identified as SEQ ID NO: 23-26 and 28-33, respectively.

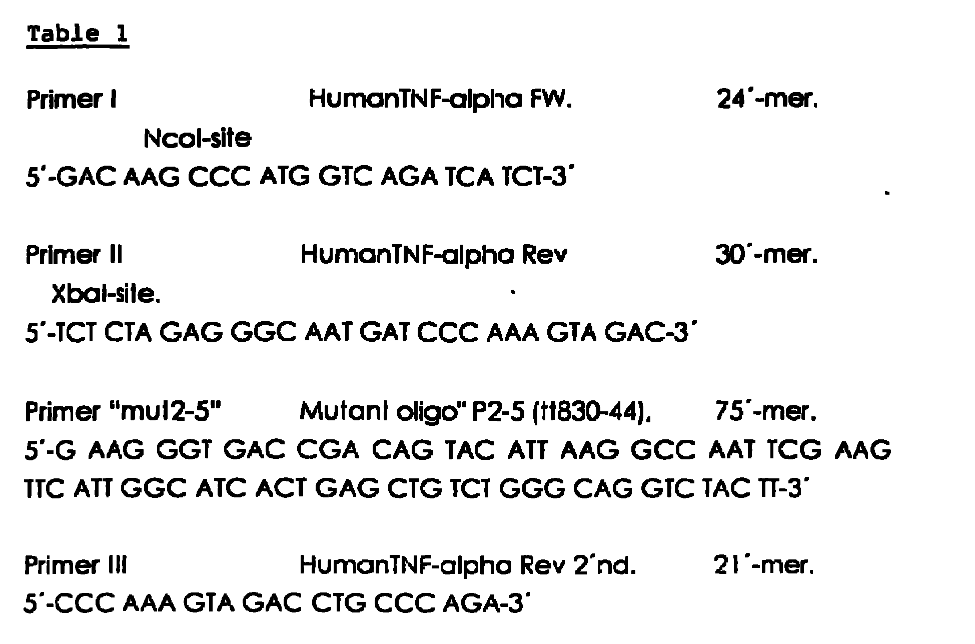

### Cultivation of Recombinant Bacteria, harvest and dissolve inclusion bodies.

### Protein purification of the TNF2-5 analog

The production of the TNF2-5 protein was analogous to the production of the other recombinant TNF molecules.
1. Inoculate 20 ml TB medium containing 50 µg/ml Carbenicillin with the transformed *E*. *coli* strain carrying the IPTG inducible plasmid vector harbouring the TNF gene encoding the recombinant protein, grow the *E.coli* over night at 37°C with shaking.
2. Dilute the over night culture 1:25 in 250 ml TB medium with 50 µg/ml Carbenicillin, and grow the culture until OD₄₅₀ is 1. Induce the expression of the recombinant protein by adding IPTG to a final concentration of 1 mM. Grow over night with vigorous shaking at 37 °C.
3. Harvest the recombinant cells from the medium by centrifugation at 3500 x g. Wash the pellet once in BSB buffer. Use 150 ml BSB per 50 g wet weight bacteria.
4. Sonicate 4 times 30 seconds at maximal amplitude until the bacterial suspension is completely homogeneous. The sonication is performed using a MSE Soniprep 150 sonicator mounted with a 9.5 mm *standard probe* (Soniprep 05 38 121-1154)
5. Add 8 µl PMSF (50 mM) and 80 µl lysosyme solution 110 mg/ml) per gram of cell pellet. Incubate 30 min at RT
6. Add 4 mg deoxycholic acid per gram pellet, mix and store at 37 °C
7. When the solution has become viscous add 20 µl DNAse (1 mg/ml) per gram pellet and MgCl to a final conc. of 5 mM, mix and store at room temperature for 30 min.
8. Sonicate in ice 5 times 30 seconds with 30 seconds intervals at maximum amplitude, until the solution has become fluid and non-viscous.
9. Centrifuge at 20.000 x g for one hour, preserve the supernatant for later checking of the washing procedure to check if all the inclusion bodies have been precipitated.
10. Resuspend the pellet in MiliQ water (1 ml H₂O per gram of E. coli), shake 1 hour.
11. Centrifuge at 20.000 x g for one hour, preserve the supernatant to check if all the inclusion bodies have been precipitated.
12. Resuspend the pellet in 1 M urea dissolve in 1 ml per gram of E. coli, shake 1 hour.
13. Centrifuge at 20.000 x g for one hour, preserve the supernatant to check if all the inclusion bodies have been precipitated.
14. Resuspend the pellet in 1 M guanidine dissolved in 1 ml per gram of E. coli, shake 1 hour.
15. Centrifuge at 20.000 x g for one hour, preserve the supernatant to check if all the inclusion bodies have been precipitated.
16. Resuspend the pellet in 25 ml 6 M guanidine + 20 mM Tris pH 8.0, agitate over night.
17. Centrifuge at 20.000 x g for one hour, preserve the supernatant containing the recombinant protein inclusion bodies, preserve the pellet to check if all the inclusion bodies have been dissolved
18. The protein solution is extensively dialysed against MilliQ water, and subsequently the solution is freeze dried.
19. The freeze dried material is solubilized in 20 mM Tris, 6 M guanidine, 30 % 2-propanol (pH 8.0) at a concentration of 20 mg/ml. It is allowed to solubilize overnight under gentle agitation. The presence of monomers is examined on a superdex 200 column (XK 16, Pharmacia, diameter: 1.6 cm, height: 750 cm.). Run in running buffer at 1 ml/min. Compared to standards in the same buffer.
20. Protein purification is performed by gel filtration on a superdex 200 column (XK26, Pharmacia; height: 100 cm, diameter: 2.6 cm) which is equilibrated with equilibration buffer. Run in the equilibration buffer. A sample volume of about 1% of total column volume is applied.
21. Refolding of the recombinant protein is performed by dialysis. The protein is diluted to 0.1 mg/ml in equilibration buffer, and this solution is placed in a boiled dialysis bag and dialyzed against: 20 mM Tris, 4 M urea (pH 8.5) with three changes, one night over at room temperature. The dialysis bag is transferred to a Tris buffer (20 mM Tris, 150 mM NaCl (pH 8.0)). Change three times of which the first one takes place at room temperature. Overnight in the cold room.

Refolding is evaluated on a Superose 12 column equilibrated in Tris buffer (20 mM Tris, 150 mM NaCl (pH 8.0)). Compare to standards.

Storage. The recombinant proteins are stored freeze dried.

Large scale production of the modified TNFα molecules may be carried out as described below:

### Starting Material:

500 Liter fermented *E*. *coli* culture, diafiltrated with water to approx. 50 Liter

### 1) Washing of the cells

A) Thaw the frozen cell slurry
B) Centrifuge the cells for 10 minutes at 4000 x g
C) Re-suspend the pellet in 50 mM Tris, 150 mM NaCl, pH 8.0
Repeat step B) and C) three times

### 2) Homogenizing the cells

A) Homogenize the cells by a Rannie homogenizer, 5 cyclic at 700 bar.
B) Centrifuge the inclusion bodies for 30 minutes at 16.500*g
C) Wash the inclusion bodies three times with 3M guanidine, 1M NaCl, 5 mM EDTA, 20% Sucrose, 50 mM Tris, pH 8. Centrifuge the inclusion bodies for 30 minutes at 16.500*g

### 3) Solubilizing of inclusion bodies

Re-suspend the pellet in 6 M guanidine, 10 mM DTT, 150 mM NaCl, 5x Ethanol, 50 mM Tris, pH 8. Use approx. 1 ml buffer/100 mg pellet

### 4) Ultrafiltration of the inclusion bodies

A) Remove rough impurities by a 0.45µ filter
B) Remove high molecular components by a 30 KD filter
C) Concentrate the inclusion bodies by a 5 KD filter.

### 5) Buffer change

Prepare the sample for ion-exchanger chromatography. Change the buffer to 6 14 Urea, 1 mM DTT in 50 mM Tris, pH 8 by diafiltration

### 6) SP-Sepharose purification

Load the protein on a SP-Sepharose column. Wash the column with four volumes of A-buffer and Elute the protein with 100% B-buffer and Pool all the fractions with TNFα.
A-buffer: 6 M Urea, 1 mM DTT, 50 mM Tris/Cl, pH 8.
B-buffer: 6 M Urea, 1 M NaCl, 1 mM DTT, 50 mM Tris/Cl, pH 8.

### 7) Refolding of human TNFα

Dilute the protein pool to approx. 0.1 mg TNFα/ml in 6 H Urea, 1 mM DTT, 50 mM NaCl, 5% EtOH in 20 mM Tris/Cl pH 8.8 and remove the Urea stepwise by going to following buffer composition - by diafiltration.
A) 2 M Urea, 1 mM DTT, 150 mM NaCl in 20 mM Tris/Cl, pH 8.8 - overnight at 5°C
B) 1M Urea, L mM DTT,150 mM NaCl in 20 mM Tris/Cl pH 8.8 - eight hours at 5°C
C) 1 mM DTT, 150 mM NaCl in 20 mM Tris/Ci pH 8.8 - overnight at 5°C
D) 150 mM NaCl in 20 mM Tris/CL pH 8.8 - overnight at 5°C

### 8) Storage of the human TNFα analogs

Concentrate the protein to 1.0 mg/ml and store the sample at -20°C.

### TB medium

Dissolve Terrific Broth (GIBCO BRL 22711-22) in MiliQ water according to the manufacturers instruction. Autoclave at 121 C for 20 min.

### Carbenicillin x 100 stock solution (50mg/ml)

Carbenicillin disodium salt (Sigma C1389) is dissolved in MiliQ water at a concentration of 50 mg/ml. The solution is filtersterilized through a 0.2 µm filter (Sterifix 0409 9206)

### IPTG x 100 stock solution 100mM

Isopropyl-beta-D-thiogalactopyranoside (IPTG, USB 17884) 1.19 g IPTG is dissolved in MiliQ water ad 50 ml. The solution is filtersterilized through a 0.2 µm filter (Sterifix 0409 9206)

### BSB buffer

Bacterial Suspension Buffer
50 mM TRIS (Trisma base, SigmaT1503)
0.5 M NaCl (Ridel-de.Haën 31434)
5 mM DTT (DL-dithiothretiol, Sigma D-0632)
pH 8.0

### PMSF

50 mM, phenylmethylsulfonylfluride, SIGMA # P-7626, dissolved in 2-propanol

### Lysosyme solution

10 mg/ml Grade III lysozyme from chicken egg white, (EC 3.2.1.17) SIGMA # L-7001

### Deoxycholic acid

(7-deoxycholic acid) Sigma # D-6750

### DNAse

1 mg/ml Dnase I, Deoxyribonuclease I, (EC.3.1.21.1) Boehringer Cat # 1284932

### UREA

Urea (GibcoBRL 15716-020)

### Guanidine

Guanidine hydrochloride (Sigma G4505)

### 2-Propanol

### Running buffer

20 mM TRIS, (Trisma base, SigmaT1503)
8 M urea, (GibcoBRL 15716-020)
0.1% β-mercaptoethanol
pH 8.0

### Equilibration buffer

20 mM TRIS, (Trisma base, SigmaT1503)
8m urea, (GibcoBRL 15716-020)
0-1% β-mercaptoethanol

### EXAMPLE 2

### Expression, purification and refolding of P2 and P30 modified TNFα molecules

It is well established that recombinant proteins behave differently during expression, purification and refolding. All proteins were expressed in *E*. *coli* and expression levels ranging from 2-20 % were obtained. All the proteins were recognized in Western blotting experiments using a commercially available polyclonal rabbit-anti human TNFα antibody.

The TNFα constructs were subsequently expressed one by one in 250 ml cultures in batch sizes of 3-4 l. All modified TNFα proteins were expressed as inclusion bodies and more than 85 % pure and refolded protein preparations were produced as described above.

The protein content was determined with standard BCA analysis. All protein purifications were performed in at least three separate runs for each molecule, and in all cases the separate protein batches were tested and found similar. The proteins were stored in concentrations from 0.2 - 1.0 mg/ml in PBS at -20 °C until use.

Standard quality control analyses included SDS gel electrophoresis with both Coomassie blue staining and silver staining of the individual protein preparations. In all cases the proteins were found to be of the expected size and to be more than 85 ° pure.

Molecules with epitopes inserted at position 4 (TNF2-4 and TNF30-4) were only expressed at relatively low levels (app. 2 %). These molecules were furthermore very difficult to purify and especially their refolding was troublesome. Both molecules, but in particular THF30-4, were not very soluble in PBS and tended to precipitate during storage.

### EXAMPLE 3.

### Biological activity of the different TNFα constructs

The direct biological activity of the purified TNFα molecules was tested in the L929 bioassay which is a standard *in vitro* assay for determination of biological TNFα activity. As seen in Fig. 6 none of the P2 or P30 modified TNFα molecules were able to kill L929 cells in the concentration range tested (up to 60 mg/ml). A commercially available recombinant TNFα molecule were fully active at about 0.6 ng/ml. The wild type TNFα preparation was also fully active in the entire concentration range tested.

### EXAMPLE 4.

### The ability of the modified TNFα molecules to induce neutralizing antibodies

Rabbits were immunized with each of the ten constructs to see whether these were able to induce antibodies capable of neutralizing biologically active human TNFα *in vitro.* Groups of three rabbits were used and each rabbit received 100 mg TNFα. s.c. in Freunds Complete Adjuvant and subsequently 100 mg approximately every third week in Freunds Incomplete Adjuvant.

During the entire immunisation period of 18 weeks blood samples were collected at regular intervals and the sera were tested for anti-TNFα activity in a conventional ELISA assay where commercially available, pure and non-modified human TNFα was used as the antigen.

All rabbits developed anti-TNFα antibodies during the immunization period and the maximum level or antibody titers varied within a decade within each group. The average antibody titers are shown in Fig. 7. TNF2-5, TNF2-7, TNF2-3, TNF2-1 and WT-TNFα induced a titer of one hundred within 2-4 weeks whereas TNF2-4 gave a remarkably slower response. Similar kinetics were observed for the TNF30 proteins where a slower response also was obtained with TNF30-4.

The ability of these sera to interfere with human TNFα and its receptor was tested in the L929 assay as well as in a solid phase receptor binding assay.

In the L929 assay dilutions of immune sera as well as a non-immune control serum were added to commercially available human TNFα prior to the addition of the mixture to L929 cells. Sera from all three rabbits in each group were tested in duplicates and the average values were calculated. Since normal serum tended to increase the background values of the color detecting system these were subtracted from all values and the relative inhibition in percent was calculated. The results for the inhibitory capacity at week 14 in the immunization schedule of all rabbits are shown in Fig. 8.

TNF2-5 which does not comprise substitutions in any segment of the back β-sheet strands is clearly superior compared to the other constructs and this molecule was comparable to the fully toxic WT-TNFα molecule with regard to the ability to induce neutralizing antibodies (data not shown). TNF2-3 which comprises a small substituted segment in the D β-strand and TNF2-7 which does not comprise any substituted segment of the back β-sheet strands, were also able to induce inhibitory antibodies, and the neutralizing antibody titer towards TNF2-7 increased significantly during the following three weeks (data not shown). TNF2-4 and THF2-1, which comprise the G and B β-strands, respectively, of the back β-sheet, were unable to induce neutralising antibodies in the L 929 assay.

The results with regard to the TNF30 proteins were more heterogeneous although TNF30-3 seemed to be best at week 14. THF30-1 and TNF30-4, which comprise substitutions in the G and B β-strands of the back β-sheet, respectively, did not induce neutralizing antibodies in the L929 assay.

In the solid phase receptor binding assay recombinant human 55 kD TNFα receptor 1 (TNF-R55) was immobilized on microtiter plates and commercially available biotinylated human TNFα was added in an appropriate dilution. Specific binding to the receptor was subsequently detected with horse radish peroxidase labeled streptavidin and a chromogenic substrate. When testing sera from immunized rabbits these were added to the biotinylated human TNFα solution prior to the addition of the mixture to the TNF-R55 coated microtiter plate. Sera from all three rabbits in each group were tested and the average values were calculated. Serum from a non-immunized rabbit was used as negative control. The background values were very low and the assay is highly sensitive. The results are shown in Fig. 9.

It can be seen that the results obtained from the L929 assay and the solid phase receptor binding assay, respectively, are almost identical with regard to the TNFα 2 constructs. In the solid phase assay the difference between TNF30-2, 30-3 and 30-5 was not as pronounced as observed in the L929 assay. The solid phase assay is, however, more reproducible due to its biochemical rather than cellular character, and normal serum values were not subtracted in this assay.

The relative amounts of serum (in percent) by which half maximum inhibition of TNFα binding was achieved (IC. values) were calculated for TNF2-3, TMF2-5, TNF2-7, TNF30-2, TNF30-3, TNF30-5 and WT-TNFα for each of the corresponding antisera. Assuming that a similar curve shape would appear for antisera raised against TNF2-1, TNF2-1, TNF30-1 and TNF30-4 extrapolations were performed, and IC50 values were also calculated for these sera. The results are shown in Table II.

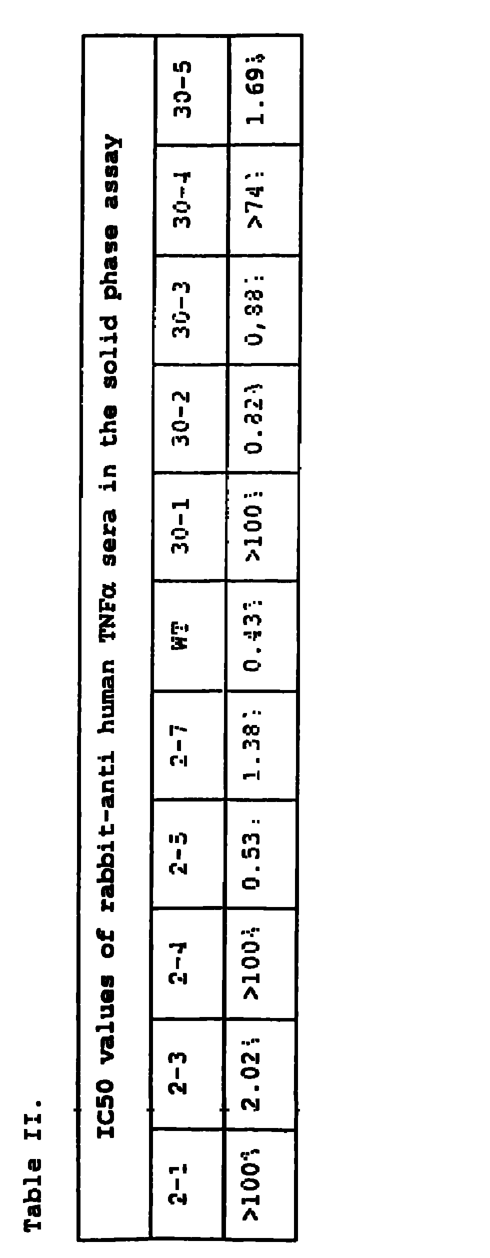

It can be seen that TNF2-5 is equivalent to wild type (WT) mouse TNFα with regard to the ability to induce neutralizing anti-mouse TNFα antibodies in rabbits. TNF2-1, TNF2-4, TNF30-1 and TNF30-4 which all comprise substitutions in the B or G strand of the back β-sheet are very poor or even totally unable to induce such antibodies. This indicates surprisingly that although the strands B and G of the back β-sheet are not involved in receptor binding, the mutations represented in this area anyhow lead to disturbance of the regions of human TNFα involved in receptor binding. TNF30-2 and TNF30-3 seem to induce neutralizing antibodies equally well.

### EXAMPLE 5.

### The ability of the modified TNFα molecules to stimulate T cells from P2 and P30 immune healthy blood donors

Since it was expected that the localisation of the P2 and P30 epitopes in the modified TNFα molecules also would affect the antigen processing and presentation of the inserted epitopes - and thus their immunogenicity - all 10 molecules were tested in different T cell assays. A polyclonal Peripheral Blood Molecular Cell (PBMC) assay was used, and a number of P2 and P30 specific T cell lines were established using conventional immunological methods to test TNFα peptides and recombinant proteins with inserted P2 and P30 epilopes.

Initially 28 healthy volunteers (donors) were tested in a conventional PBMC proliferation assay for their ability to respond to TT and the P2 and P30 peptides. On the basis of these results 19 individuals capable of responding significantly to TT, P2 as well as P30 peptides were selected for further experiments. Although the response levels varied much, this clearly confirmed that P2 and P30 are promiscuous as well as immunodominant T cell epitopes.

In addition to the 28 donors, seven further donors were also selected. For reasons explained below some of these were selected for their ability to respond either to P30 or to P2. Several of these were furthermore vaccinated with TT in order to he able to give a strong T cell response. Some of the second group of donors were also used to raise P2 or P30 specific T cell lines in order to study the antigen presentation or P2 and P30 modified synthetic TNFα peptides as well as the modified molecules. In Fig. 10 representative examples of the polyclonal PBMC proliferative response in three donors towards TT as well as the P2 and P30 peptides are shown.

All 10 recombinant proteins were tested in triplicate in at least 6 different concentrations starting from about 100 mg/ml, and all PBMC experiments were repeated 2-3 times for each individual. Intracellular incorporation of ³H-labelled thymidine was used to assess T cell proliferation, and the experiments were harvested and counted in a 96-well Format. The maximum proliferation indexes (P1) from each titration curve was calculated as the relation between the average number of CPMs in the experimental wells divided by the average number of CPMs obtained in the antigen free wells (PBS only). The T cell proliferation data were made in triplicate and Con A as well at the P2 and P30 were used as negative and positive controls, respectively. In Fig. 11 three examples are shown from experiments with two different blood donors using the different P2 and P30 modified TNFα molecules.

JH only responds to P2, whereas SR responds to both P2 and P30. This is also reflected in the proliferative responses to the P2 and P30 modified TNFα proteins which to a varying extent almost all are able to stimulate the PBMCs.

In Fig. 12 the Proliferation Indexes (PIs) calculated from 34 experiments are shown. Although it is very difficult to quantitatively compare PIs between different experiments due to varying PBS backgrounds it seems clear that all constructs more or less are capable of eliciting a proliferative response.

Among the second group of donors some individuals were vaccinated against TT 1-2 months before the experiments. The PIs obtained from these persons (HB, HB, KG, and MW) were all among the highest PIs observed for all the modified TNFα constructs and the data were easy to evaluate. Three other individuals (DL, ID, and LS) were vaccinated more than 5 years back, and they all showed PI values below average. This supports that the observed PIs were antigen specific. No data regarding the last TT vaccination dates were available for the first group of donors, but their immunization status was clearly very variable.

It is not possible to select a preferred TNFα 2 or TNFα 30 protein merely based on the average size of the PI values. This may be due to the heterogeneous vaccination status of the individual used and the variable nature of PBMC assays obtained from individuals with variable responder status. It was anyway surprising, that P2 and P30 at all the inserted positions in TNFα seemed to be able to be processed and presented to T cells. In order to exclude the possibility that despite repeated affinity chromatography, gelfiltration and dialysis, the antigen preparations could still contain significant concentrations of non-specific mitogens, some further experiments were performed.

Donors from the second group known to be non-responders to P2 and responders to P30 as well as donors with the opposite response pattern were tested in PBMC assays. In Fig. 13 the response to P2, P30 as well as the TNFα 2 and TNFα 30 proteins are shown for DL and ID.

It can be seen that specific responses are obtained to the respective T cell epitopes and the respectively modified TNFα Molecules. However, no significant proliferative responses of DL against TNFα 30 proteins were observed (upper panel) and no significant proliferative responses of ID to TNFα 2 proteins were observed (lower panel) supporting that non-specific mitogens were absent in the purified TNFα preparations.

This possibility was even further examined by the use of P2 and P30 specific T cell lines isolated from the second group of donors, which had been cultured for at least six weeks in at least three rounds of stimulation with the respective synthetic P2 and P30 peptides. In Fig. 14 the results of two such experiments are shown.

It can be seen that the P2 and P30 specific T cell lines were only stimulated by their corresponding P2 and P30 proteins. Furthermore, it can be seen again that all TNFα constructs are able to induce T cell proliferation emphasizing that although antigen processing may be quantitatively important for presentation of P2 and P30, it does not seem to be a significant qualitative limiting factor for antigen presentation.

It has been reported in the literature that flanking regions of T cell epitopes can influence the binding or antigenic peptides to MHC class II molecules. Since none of the positions in TNFα, which were chosen for insertion of P2 or P30, seemed to be prohibitive for antigen presentation, it was investigated whether the different flanking TNFα sequences of the inserted epitopes could influence the T cell response to P2 and P30 epitopes as a result of differential binding to the human HLA c.Lass II molecules. The peptides shown in Table III, which represent the inserted epitopes as well as the flanking human TNFα aminoacids were therefore synthesized. These are designated PP2-5, PP30-3, etc. The amino acid sequences are shown in the sequence listing as SEQ ID NO:34 to SEQ ID NO:42 designated Pep2-1 to Pep30-5.

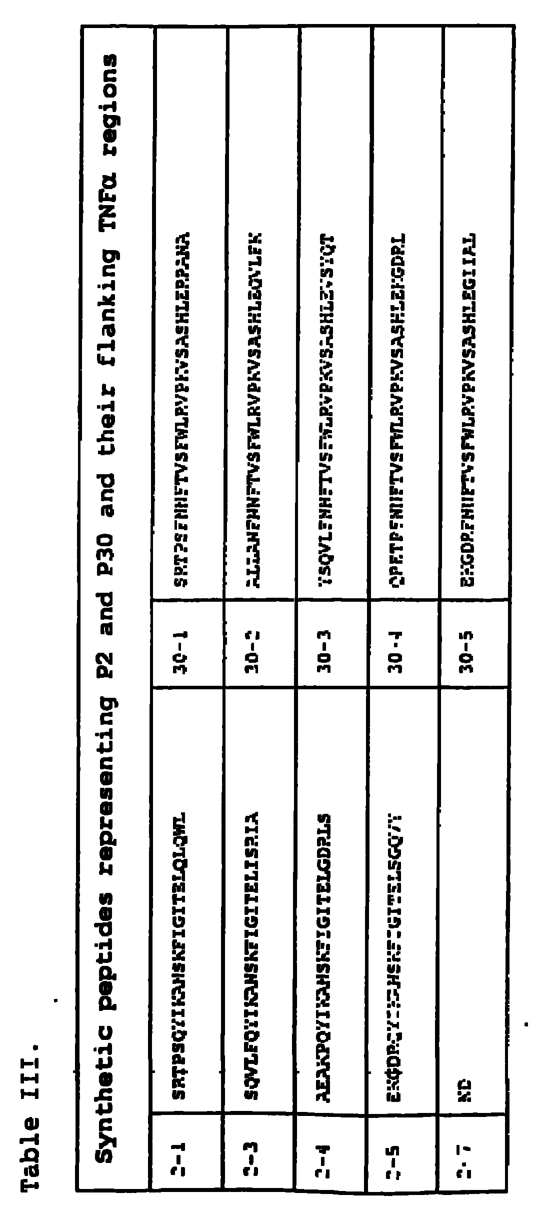

These peptides were used for stimulation of P2 and P30 specific T cell lines. The results from stimulation of the P30 lines are shown in Fig. 15. It can be seen that the P2 specific T cell lines from MR and KG shows parallel stimulation patterns when stimulated either with the peptide or with the corresponding TNFα 2 protein. It is clear that TNF2-5 is a good potential antigen and these data further supports that the observed T cell proliferations are antigen specific. There was generally no qualitative differences in the stimulation pattern when the P30 specific T cell lines from MR and KG were stimulated either with peptides or proteins (data not shown). The P30 specific T cell line from HC preferably recognized with TNF30-3 and reacted to a minor extent with TNF30-2.

### CONCLUSION

Ten differently modified human TNFα proteins have been produced and characterized. They were constructed to contain two well known promiscuous T cell epitopes P2 and P30 in order to be potentially immunogenic in at least 85% of the populations.

All proteins could be expressed and purified although TNF2-4 and TNF30-4 at low levels. Mutations at this position in TNFα also seems to interfere with refolding which results in proteins with poor solubility. No biological TNFα activity could be detected in any of the modified TNFα molecules.

Rabbits were immunized with all ten proteins as well as with native TNFα. After 2-3 months of immunization it was possible to detect high titers of strongly cross-reactive antibodies towards human, non-modified TNFα in all sera.

The ability of these antibodies to interfere with the biological activity of native TNFα was tested in two different *in vitro* assays - the L929 bioassay and a solid phase receptor binding assay. Both assays showed essentially the same TNF2-1, TNF2-4, TNF30-1 and TNF30-4 was not able to induce significant neutralising antibodies, whereas TNF2-5 was superior compared to the other constructs. TMF30-2 and TNF30-3 were equally efficient at inducing neutralising antibodies and twice as good as TNF30-5 which was reasonably good.

Somewhat surprisingly it was not possible to see significant differences between the abilities of the molecules to stimulate PBMC to proliferate. It could be demonstrated that this was not due to the presence of mitogens in the antigen preparations and based on these data, it was concluded that the locations chosen for P2 and P30 all allowed presentation of the respective epitopes. The specificity of the responses was further documented by using testing the modified TNFα proteins using epitope specific T cell lines as well as synthetic peptides representing the inserted epitopes as well as the flanking TNFα sequences. From these experiments it was clearly demonstrated that TNF2-5, TNF30-2 and TNF30-3 (among other constructs) were the most powerful potential immunogens.

### SEQUENCE LISTING

(1) GENERAL INFORMATION:
   (i) APPLICANT:
      (A) NAME: Farmaceutisk Laboratorium Ferring A/S
      (B) STREET: Indertoften 10
      (C) CITY: Vanloese
      (E) COUNTRY: Denmark
      (F) POSTAL CODE (ZIP): DK-2720
   (ii) TITLE OF INVENTION: Modified human THF-alpha molecules, DNA encoding them, and vaccines containing said modified THF-alpha of DNA
   (iii) NUMBER OF SEQUENCES: 42
   (iv) COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE; PatentIn Release #1.0, Version #1.30 (EPO)
(2) INFORMATION FOR SEQ ID NO: 1:
   (i) SEQUENCE CHARACTERISTICS:
      (A) LENGTH: 477 base pairs
      (B) TYPE: nucleic acid
      (C) STRANDEDNESS: double
      (D) TOPOLOGY: linear
   (ii) MOLECULE TYPE: cDNA
   (iii) HYPOTHETICAL: NO
   (iv) ANTI-SENSE: NO
   (vi) ORIGINAL SOURCE:
      (A) ORGANISM: Homo sapiens
   (ix) FEATURE;
      (A) NAME/KEY: CDS
      (B) LOCATION:1..477
      (C) IDENTIFICATION METHOD: experimental
      (D) OTHER INFORMATION:/codon_start= 1
         /function= "Antigen"
         /product= "TNF-alpha analog"
         /evidence= EXPERIMENTAL
         /gene= "tnfP2-1"
         /standard_name= "TNF2-1"
   (xi) SEQUENCE DESCRIPTION: SEQ ID NO: 1:
   ATG GTC AGA TCA TCT TCT CGA ACC CCG AGT CAG TAC ATT AAA GCC AAT      48
   Met Val Arg Ser Ser Ser Arg Thr Pro Ser Gln Tyr Ile Lys Ala Asn
     1               5                  10                  15
   TCT AAA TTC ATC GGT ATA ACT GAG CTG CAG CTC CAG TGG CTG AAC CGC      96
   Ser Lys Phe Ile Gly Ile Thr Glu Leu Gln Leu Gln Trp Leu Asn Arg
               20                  25                  30
   CGG GCC AAT GCC CTC CTG GCC AAT GGC GTG GAG CTG AGA GAT AAC CAG      144
   Arg Ala Asn Ala Leu Leu Ala Asn Gly Val Glu Leu Arg Asp Asn Gln
           35                  40                  45
   CTG GTG GTG CCA TCA GAG GGC CTG TAC CTC ATC TAC TCC CAG GTC CTC      192
   Leu Val Val Pro Ser Glu Gly Leu Tyr Leu Ile Tyr Ser Gln Val Leu
       50                  55                  60
   TTC AAG GGC CAA GGC TGC CCC TCC ACC CAT GTG CTC CTC ACC CAC ACC      240
   Phe Lys Gly Gln Gly Cys Pro Ser Thr His Val Leu Leu Thr His Thr
   65                  70                  75                  80
   ATC AGC CGC ATC GCC GTC TCC TAC CAG ACC AAG GTC AAC CTC CTC TCT      288
   Ile Ser Arg Ile Ala Val Ser Tyr Gln Thr Lys Val Asn Leu Leu Ser
                   85                  90                  95
   GCC ATC AAG AGC CCC TGC CAG AGG GAG ACC CCA GAG GGG GCT GAG GCC      336
   Ala Ile Lys Ser Pro Cys Gln Arg Glu Thr Pro Glu Gly Ala Glu Ala
               100                 105                 110
   AAG CCC TGG TAT GAG CCC ATC TAT CTG GGA GGG GTC TTC CAG CTG GAG      384
   Lys Pro Trp Tyr Glu Pro Ile Tyr Leu Gly Gly Val Phe Gln Leu Glu
           115                 120                 125
   AAG GGT GAC CGA CTC AGC GCT GAG ATC AAT CGG CCC GAC TAT CTC GAC      432
   Lys Gly Asp Arg Leu Ser Ala Glu Ile Asn Arg Pro Asp Tyr Leu Asp
       130                 135                 140
   TTT GCC GAG TCT GGG CAG GTC TAC TTT GGG ATC ATT GCC CTC TAG          477
   Phe Ala Glu Ser Gly Gln Val Tyr Phe Gly Ile Ile Ala Leu  *
   145                 150                 155
(2) INFORMATION FOR SEQ ID NO: 2:
   (1) SEQUENCE CHARACTERISTICS:
      (A) LENGTH: 159 amino acids
      (B) TYPE: amino acid
      (D) TOPOLOGY: linear
   (ii) MOLECULE TYPE: protein
   (xi) SEQUENCE DESCRIPTION: SEQ ID NO: 2:
   Met Val Arg Ser Ser Ser Arg Thr Pro Ser Gln Tyr Ile Lys Ala Asn
    1               5                  10                  15
   Ser Lys Phe Ile Gly Ile Thr Glu Leu Gln Leu Gln Trp Leu Asn Arg
               20                  25                  30
   Arg Ala Asn Ala Leu Leu Ala Asn Gly Val Glu Leu Arg Asp Asn Gln
           35                  40                  45
   Leu Val Val Pro Ser Glu Gly Leu Tyr Leu Ile Tyr Ser Gln Val Leu
       50                  55                  60
   Phe Lys Gly Gln Gly Cys Pro Ser Thr His Val Leu Leu Thr His Thr
   65                  70                  75                  80
   Ile Ser Arg Ile Ala Val Ser Tyr Gln Thr Lys Val Asn Leu Leu Ser
                   85                  90                  95
   Ala Ile Lys Ser Pro Cys Gln Arg Glu Thr Pro Glu Gly Ala Glu Ala
               100                 105                 110
   Lys Pro Trp Tyr Glu Pro Ile Tyr Leu Gly Gly Val Phe Gln Leu Glu
           115                 120                 125
   Lys Gly Asp Arg Leu Ser Ala Glu Ile Asn Arg Pro Asp Tyr Leu Asp
       130                 135                 140
   Phe Ala Glu Ser Gly Gln Val Tyr Phe Gly Ile Ile Ala Leu  *
   145                 150                 155
(2) INFORMATION FOR SEQ ID NO: 3:
   (i) SEQUENCE CHARACTERISTICS:
      (A) LENGTH: 477 base pairs
      (B) TYPE: nucleic acid
      (C) STRANDEDNESS: double
      (D) TOPOLOGY: Linear
   (ii) MOLECULE TYPE: DNA (genomic)
   (iii) HYPOTHETICAL: NO
   (iv) ANTI-SENSE: NO
   (vi) ORIGINAL SOURCE:
      (A) ORGANISM: Homo sapiens
   (ix) FEATURE:
      (A) NAME/KEY: CDS
      (B) LOCATION:1..477
      (D) OTHER INFORMATION:/codon_start" 1
         /function= "Antigen"
         /product= "TNF-alpha analog"
         /gene= "tnfP2-3"
         /standard_name= "TNF2-3"
   (xi) SEQUENCE DESCRIPTION: SEQ ID NO: 3:
   ATG GTC AGA TCA TCT TCT CGA ACC CCG AGT GAC AAG CCT GTA GCC CAT      48
   Met Val Arg Ser Ser Ser Arg Thr Pro Ser Asp Lys Pro Val Ala His
   160                 165                 170                 175
   GTT GTA GCA AAC CCT CAA GCT GAG GGG CAG CTC CAG TGG CTG AAC CGC      96
   Val Val Ala Asn Pro Gln Ala Glu Gly Gln Leu Gln Trp Leu Asn Arg
                   180                 185                 190
   CGG GCC AAT GCC CTC CTG GCC AAT GGC GTG GAG CTG AGA GAT AAC CAG     144
   Arg Ala Asn Ala Leu Leu Ala Asn Gly Val Glu Leu Arg Asp Asn Gln
               195                 200                 205
   CTG GTG GTG CCA TCA GAG GGC CTG TAC CTC ATC TAC TCC CAG GTC CTC     192
   Leu Val Val Pro Ser Glu Gly Leu Tyr Leu Ile Tyr Ser Gln Val Leu
           210                 215                 220
   TTC CAG TAC ATA AAG GCC AAC TCC AAG TTT ATC GGC ATC ACC GAG CTC     240
   Phe Gln Tyr Ile Lys Ala Asn Ser Lys Phe Ile Gly Ile Thr Glu Leu
       225                 230                 235
   ATC AGC CGC ATC GCC GTC TCC TAC CAG ACC AAG GTC AAC CTC CTC TCT     288
   Ile Ser Arg Ile Ala Val Ser Tyr Gln Thr Lys Val Asn Leu Leu Ser
   240                 245                 250                 255
   GCC ATC AAG AGC CCC TGC CAG AGG GAG ACC CCA GAG GGG GCT GAG GCC     336
   Ala Ile Lys Ser Pro Cys Gln Arg Glu Thr Pro Glu Gly Ala Glu Ala
                   260                 265                 270
   AAG CCC TGG TAT GAG CCC ATC TAT CTG GGA GGG GTC TTC CAG CTG GAG     384
   Lys Pro Trp Tyr Glu Pro Ile Tyr Leu Gly Gly Val Phe Gln Leu Glu
               275                 280                 285
   AAG GGT GAC CGA CTC AGC GCT GAG ATC AAT CGG CCC GAC TAT CTC GAC     432
   Lys Gly Asp Arg Leu Ser Ala Glu Ile Asn Arg Pro Asp Tyr Leu Asp
           290                 295                 300
   TTT GCC GAG TCT GGG CAG GTC TAC TTT GGG ATC ATT GCC CTC TAG         477
   Phe Ala Glu Ser Gly Gln Val Tyr Phe Gly Ile Ile Ala Leu  *
       305                 310                 315
(2) INFORMATION FOR SEQ ID NO: 4:
   (i) SEQUENCE CHARACTERISTICS:
      (A) LENGTH: 159 amino acids
      (B) TYPE: amino acid
      TOPOLOGY: linear
   (ii) MOLECULE TYPE: protein
   (xi) SEQUENCE DESCRIPTION: SEQ ID NO: 4:
   Met Val Arg Ser Ser Ser Arg Thr Pro Ser Asp Lys Pro Val Ala His
     1               5                  10                  15
   Val Val Ala Asn Pro Gln Ala Glu Gly Gln Leu Gln Trp Leu Asn Arg
                20                  25                  30
   Arg Ala Asn Ala Leu Leu Ala Asn Gly Val Glu Leu Arg Asp Asn Gln
            35                  40                  45
   Leu Val Val Pro Ser Glu Gly Leu Tyr Leu Ile Tyr Ser Gln Val Leu
        50                  55                  60
   Phe Gln Tyr Ile Lys Ala Asn Ser Lys Phe Ile Gly Ile Thr Glu Leu
    65                  70                  75                  80
   Ile Ser Arg Ile Ala Val Ser Tyr Gln Thr Lys Val Asn Leu Leu Ser
                    85                  90                  95
   Ala Ile Lys Ser Pro Cys Gln Arg Glu Thr Pro Glu Gly Ala Glu Ala
               100                 105                 110
   Lys Pro Trp Tyr Glu Pro Ile Tyr Leu Gly Gly Val Phe Gln Leu Glu
           115                 120                 125
   Lys Gly Asp Arg Leu Ser Ala Glu Ile Asn Arg Pro Asp Tyr Leu Asp
       130                 135                 140
   Phe Ala Glu Ser Gly Gln Val Tyr Phe Gly Ile Ile Ala Leu  *
   145                 150                 155
(2) INFORMATION FOR SEQ ID NO: 5:
   (i) SEQUENCE CHARACTERISTICS:
      (A) LENGTH: 477 base pairs
      (B) TYPE: nucleic acid
      (C) STRANDEDNESS: double
      (D) TOPOLOGY: linear
   (ii) MOLECULE TYPE: DNA (genomic)
   (iii) HYPOTHETICAL: NO
   (iv) ANTI-SENSE: NO
   (vi) ORIGINAL SOURCE:
      (A) ORGANISM: Homo sapiens
   (ix) FEATURE:
      (A) NAME/KEY: CDS
      (B) LOCATION:1..477
      (D) OTHER INFORMATION:/codon_start= 1
         /function= "Antigen"
         /product= "TNF-alpha analog"
         /gene= "tnfP2-4"
         /standard_name= "TNF2-4"
   (xi) SEQUENCE DESCRIPTION: SEQ ID NO: 5:
   ATG GTC AGA TCA TCT TCT CGA ACC CCG AGT GAC AAG CCT GTA GCC CAT      48
   Met Val Arg Ser Ser Ser Arg Thr Pro Ser Asp Lys Pro Val Ala His
   160                 165                 170                 175
   GTT GTA GCA AAC CCT CAA GCT GAG GGG CAG CTC CAG TGG CTG AAC CGC      96
   Val Val Ala Asn Pro Gln Ala Glu Gly Gln Leu Gln Trp Leu Asn Arg
                   180                 185                 190
   CGG GCC AAT GCC CTC CTG GCC AAT GGC GTG GAG CTG AGA GAT AAC CAG     144
   Arg Ala Asn Ala Leu Leu Ala Asn Gly Val Glu Leu Arg Asp Asn Gln
               195                 200                 205
   CTG GTG GTG CCA TCA GAG GGC CTG TAC CTC ATC TAC TCC CAG GTC CTC     192
   Leu Val Val Pro Ser Glu Gly Leu Tyr Leu Ile Tyr Ser Gln Val Leu
           210                 215                 220
   TTC AAG GGC CAA GGC TGC CCC TCC ACC CAT GTG CTC CTC ACC CAC ACC     240
   Phe Lys Gly Gln Gly Cys Pro Ser Thr His Val Leu Leu Thr His Thr
       225                 230                 235
   ATC AGC CGC ATC GCC GTC TCC TAC CAG ACC AAG GTC AAC CTC CTC TCT     288
   Ile Ser Arg Ile Ala Val Ser Tyr Gln Thr Lys Val Asn Leu Leu Ser
   240                 245                 250                 255
   GCC ATC AAG AGC CCC TGC CAG AGG GAG ACC CCA GAG GGG GCT GAG GCC     336
   Ala Ile Lys Ser Pro Cys Gln Arg Glu Thr Pro Glu Gly Ala Glu Ala
                   260                 265                 270
   AAG CCC CAG TAT ATC AAG GCC AAT TCG AAA TTC ATC GGC ATC ACG GAG     384
   Lys Pro Gln Tyr Ile Lys Ala Asn Ser Lys Phe Ile Gly Ile Thr Glu
               275                 280                 285
   CTC GGT GAC CGA CTC AGC GCT GAG ATC AAT CGG CCC GAC TAT CTC GAC     432
   Leu Gly Asp Arg Leu Ser Ala Glu Ile Asn Arg Pro Asp Tyr Leu Asp
           290                 295                 300
   TTT GCC GAG TCT GGG CAG GTC TAC TTT GGG ATC ATT GCC CTG TAG         477
   Phe Ala Glu Ser Gly Gln Val Tyr Phe Gly Ile Ile Ala Leu  *
       305                 310                 315
(2) INFORMATION FOR SEQ ID NO: 6:
   (i) SEQUENCE CHARACTERISTICS:
      (A) LENGTH: 159 amino acids
      (B) TYPE: amino acid
      (D) TOPOLOGY: linear
   (ii) MOLECULE TYPE: protein
   (xi) SEQUENCE DESCRIPTION: SEQ ID NO: 6:
   Met Val Arg Ser Ser Ser Arg Thr Pro Ser Asp Lys Pro Val Ala His
    1               5                  10                  15
   Val Val Ala Asn Pro Gln Ala Glu Gly Gln Leu Gln Trp Leu Asn Arg
               20                  25                  30
   Arg Ala Asn Ala Leu Leu Ala Asn Gly Val Glu Leu Arg Asp Asn Gln
           35                  40                  45
   Leu Val Val Pro Ser Glu Gly Leu Tyr Leu Ile Tyr Ser Gln Val Leu
       50                  55                  60
   Phe Lys Gly Gln Gly Cys Pro Ser Thr His Val Leu Leu Thr His Thr
   65                  70                  75                  80
   Ile Ser Arg Ile Ala Val Ser Tyr Gln Thr Lys Val Asn Leu Leu Ser
                   85                  90                  95
   Ala Ile Lys Ser Pro Cys Gln Arg Glu Thr Pro Glu Gly Ala Glu Ala
               100                 105                 110
   Lys Pro Gln Tyr Ile Lys Ala Asn Ser Lys Phe Ile Gly Ile Thr Glu
           115                 120                 125
   Leu Gly Asp Arg Leu Ser Ala Glu Ile Asn Arg Pro Asp Tyr Leu Asp
       130                 135                 140
   Phe Ala Glu Ser Gly Gln Val Tyr Phe Gly Ile Ile Ala Leu   *
   145                 150                 155
(2) INFORMATION FOR SEQ ID NO: 7:
   (i) SEQUENCE CHARACTERISTICS:
      (A) LENGTH: 477 base pairs
      (B) TYPE: nucleic acid
      (C) STRANDEDNESS: double
      (D) TOPOLOGY: linear
   (ii) MOLECULE TYPE: DNA (genomic)
   (iii) HYPOTHETICAL: NO
   (iv) ANTI-SENSE: NO
   (vi) ORIGINAL SOURCE:
      (A) ORCANISM: Homo sapiens
   (ix) FEATURE:
      (A) NAME/KEY: CDS
      (B) LOCATION:1..477
      (D) OTHER INFORMATION:/function= "Antigen"
         /product= "TNF-alpha analog"
         /gene= "tnfP2-5"
         /standard_name= "TNF2-5"
   (xi) SEQUENCE DESCRIPTION: SEQ ID NO: 7:
   ATG GTC AGA TCA TCT TCT CGA ACC CCG AGT GAC AAG CCT GTA GCC CAT        48
   Met Val Arg Ser Ser Ser Arg Thr Pro Ser Asp Lys Pro Val Ala His
   160                 165                 170                 175
   GTT GTA GCA AAC CCT CAA GCT GAG GGG CAG CTC CAG TGG CTG AAC CGC        96
   Val Val Ala Asn Pro Gln Ala Glu Gly Gln Leu Gln Trp Leu Asn Arg
                   180                 185                 190
   CGG GCC AAT GCC CTC CTG GCC AAT GGC GTG GAG CTG AGA GAT AAC CAG       144
   Arg Ala Asn Ala Leu Leu Ala Asn Gly Val Glu Leu Arg Asp Asn Gln
               195                 200                 205
   CTG GTG GTG CCA TCA GAG GGC CTG TAC CTC ATC TAC TCC CAG GTC CTC       192
   Leu Val Val Pro Ser Glu Gly Leu Tyr Leu Ile Tyr Ser Gln Val Leu
           210                 215                 220
   TTC AAG GGC CAA GGC TGC CCC TCC ACC CAT GTG CTC CTC ACC CAC ACC       240
   Phe Lys Gly Gln Gly Cys Pro Ser Thr His Val Leu Leu Thr His Thr
       225                 230                 235
   ATC AGC CGC ATC GCC GTC TCC TAC CAG ACC AAG GTC AAC CTC CTC TCT       288
   Ile Ser Arg Ile Ala Val Ser Tyr Gln Thr Lys Val Asn Leu Leu Ser
   240                 245                 250                 255
   GCC ATC AAG AGC CCC TGC CAG AGG GAG ACC CCA GAG GGG GCT GAG GCC       336
   Ala Ile Lys Ser Pro Cys Gln Arg Glu Thr Pro Glu Gly Ala Glu Ala
                   260                 265                 270
   AAG CCC TGG TAT GAG CCC ATC TAT CTG GGA GGG GTC TTC CAG CTG GAG       384
   Lys Pro Trp Tyr Glu Pro Ile Tyr Leu Gly Gly Val Phe Gln Leu Glu
               275                 280                 285
   AAG GGT GAC CGA CAG TAC ATT AAG GCC AAT TCG AAG TTC ATT GGC ATC       432
   Lys Gly Asp Arg Gln Tyr Ile Lys Ala Asn Ser Lys Phe Ile Gly Ile
           290                 295                 300
   ACT GAG CTG TCT GGG CAG GTC TAC TTT GGG ATC ATT GCC CTC TAG           477
   Thr Glu Leu Ser Gly Gln Val Tyr Phe Gly Ile Ile Ala Leu  *
       305                 310                 315
(2) INFORMATION FOR SEQ ID NO: 8:
   (i) SEQUENCE CHARACTERISTICS:
      (A) LENGTH: 159 amino acids
      (B) TYPE: amino acid
      (D) TOPOLOGY: linear
   (ii) MOLECULE TYPE: protein
   (xi) SEQUENCE DESCRIPTION SEQ ID NO: 8:
   Met Val Arg Ser Ser Ser Arg Thr Pro Ser Asp Lys Pro Val Ala His
    1               5                  10                  15
   Val Val Ala Asn Pro Gln Ala Glu Gly Gln Leu Gln Trp Leu Asn Arg
               20                  25                  30
   Arg Ala Asn Ala Leu Leu Ala Asn Gly Val Glu Leu Arg Asp Asn Gln
           35                  40                  45
   Leu Val Val Pro Ser Glu Gly Leu Tyr Leu Ile Tyr Ser Gln Val Leu
       50                  55                  60
   Phe Lys Gly Gln Gly Cys Pro Ser Thr His Val Leu Leu Thr His Thr
   65                  70                  75                  80
   Ile Ser Arg Ile Ala Val Ser Tyr Gln Thr Lys Val Asn Leu Leu Ser
                   85                  90                  95
   Ala Ile Lys Ser Pro Cys Gln Arg Glu Thr Pro Glu Gly Ala Glu Ala
               100                 105                 110
   Lys Pro Trp Tyr Glu Pro Ile Tyr Leu Gly Gly Val Phe Gln Leu Glu
           115                 120                 125
   Lys Gly Asp Arg Gln Tyr Ile Lys Ala Asn Ser Lys Phe Ile Gly Ile
       130                 135                 140
   Thr Glu Leu Ser Gly Gln Val Tyr Phe Gly Ile Ile Ala Leu   *
   145                 150                 155
(2) INFORMATION FOR SEQ ID NO: 9:
   (i) SEQUENCE CHARACTERISTICS:
      (A) LENGTH: 477 base pairs
      (B) TYPE: nucleic acid
      (C) STRANDEDNESS: double
      (D) TOPOLOGY: linear
   (ii) MOLECULE TYPE: DNA (genomic)
   (iii) HYPOTHETICAL: NO
   (iv) ANTI-SENSE: NO
   (vi) ORIGINAL SOURCE:
      (A) ORGANISM: Homo sapiens
   (ix) FEATURE:
      (A) NAME/KEY: CDS
      (B) LOCATION:1..477
      (D) OTHER INFORMATION:/codon_start = 1
         /function= "Antigen"
         /product= "TNF-alpha analog"
         /gene= "tnfP2-7"
         /standard_name= "TNF2-7"
   (xi) SEQUENCE DESCRIPTION: SEQ ID NO: 9:
   ATG GTC AGA TCA TCT TCT CGA ACC CCG AGT GAC AAG CCT GTA GCC CAT    48
   Met Val Arg Ser Ser Ser Arg Thr Pro Ser Asp Lys Pro Val Ala His
   160                 165                 170                 175
   GTT GTA GCA AAC CCT CAA GCT GAG GGG CAG CTC CAG TGG CTG AAC CGC    96
   Val Val Ala Asn Pro Gln Ala Glu Gly Gln Leu Gln Trp Leu Asn Arg
                   180                 185                 190
   CGG GCC AAT GCC CTC CTG GCC AAT GGC GTG GAG CTG AGA GAT AAC CAG    144
   Arg Ala Asn Ala Leu Leu Ala Asn Gly Val Glu Leu Arg Asp Asn Gln
               195                 200                 205
   CTG GTG GTG CCA TCA GAG GGC CTG TAC CTC ATC TAC TCC CAG GTC CTC    192
   Leu Val Val Pro Ser Glu Gly Leu Tyr Leu Ile Tyr Ser Gln Val Leu
           210                 215                 220
   TTC AAG GGC CAA GGC TGC CCC TCC ACC CAT GTG CTC CAG TAC ATC AAA    240
   Phe Lys Gly Gln Gly Cys Pro Ser Thr His Val Leu Gln Tyr Ile Lys
       225                 230                 235
   GCT AAC TCC AAA TTC ATC GGC ATC ACC GAA CTG GTT AAC CTC CTC TCT    288
   Ala Asn Ser Lys Phe Ile Gly Ile Thr Glu Leu Val Asn Leu Leu Ser
   240                 245                 250                 255
   GCC ATC AAG AGC CCC TGC CAG AGG GAG ACC CCA GAG GGG GCT GAG GCC    336
   Ala Ile Lys Ser Pro Cys Gln Arg Glu Thr Pro Glu Gly Ala Glu Ala
                   260                 265                 270
   AAG CCC TGG TAT GAG CCC ATC TAT CTG GGA GGG GTC TTC CAG CTG GAG    384
   Lys Pro Trp Tyr Glu Pro Ile Tyr Leu Gly Gly Val Phe Gln Leu Glu
               275                 280                 285
   AAG GGT GAC CGA CTC AGC GCT GAG ATC AAT CGG CCC GAC TAT CTC GAC    432
   Lys Gly Asp Arg Leu Ser Ala Glu Ile Asn Arg Pro Asp Tyr Leu Asp
           290                 295                 300
   TTT GCC GAG TCT GGG CAG GTC TAC TTT GGG ATC ATT GCC CTG TAG        477
   Phe Ala Glu Ser Gly Gln Val Tyr Phe Gly Ile Ile Ala Leu  *
       305                 310                 315
(2) INFORMATION FOR SEQ ID NO: 10:
   (i) SEQUENCE CHARACTERISTICS:
      (A) LENGTH: 159 amino acids
      (B) TYPE: amino acid
      (D) TOPOLOGY: linear
   (ii) MOLECULE TYPE: protein
   (xi) SEQUENCE DESCRIPTION: SEQ ID NO: 10:
   Met Val Arg Ser Ser Ser Arg Thr Pro Ser Asp Lys Pro Val Ala His
   1               5                   10                  15
   Val Val Ala Asn Pro Gln Ala Glu Gly Gln Leu Gln Trp Leu Asn Arg
               20                  25                  30
   Arg Ala Asn Ala Leu Leu Ala Asn Gly Val Glu Leu Arg Asp Asn Gln
           35                  40                  45
   Leu Val Val Pro Ser Glu Gly Leu Tyr Leu Ile Tyr Ser Gln Val Leu
       50                  55                  60
   Phe Lys Gly Gln Gly Cys Pro Ser Thr His Val Leu Gln Tyr Ile Lys
   65                  70                  75                  80
   Ala Asn Ser Lys Phe Ile Gly Ile Thr Glu Leu Val Asn Leu Leu Ser
                   85                  90                  95
   Ala Ile Lys Ser Pro Cys Gln Arg Glu Thr Pro Glu Gly Ala Glu Ala
               100                 105                 110
   Lys Pro Trp Tyr Glu Pro Ile Tyr Leu Gly Gly Val Phe Gln Leu Glu
           115                 120                 125
   Lys Gly Asp Arg Leu Ser Ala Glu Ile Asn Arg Pro Asp Tyr Leu Asp
       130                 135                 140
   Phe Ala Glu Ser Gly Gln Val Tyr Phe Gly Ile Ile Ala Leu  *
   145                 150                 155
(2) INFORMATION FOR SEQ ID NO: 11:
   (i) SEQUENCE CHARACTERISTICS:
      (A) LENGTH: 477 base pairs
      (B) TYPE: nucleic acid
      (C) STRANDEDNESS: double
      (D) TOPOLOGY: linear
   (ii) MOLECULE TYPE: DNA (genomic)
   (iii) HYPOTHETICAL: NO
   (iv) ANTI-SENSE: 110
   (vi) ORIGINAL SOURCE:
      (A) ORGANISM: Homo sapiens
   (ix) FEATURE:
      (A) NAME/KEY: CDS
      (B) LOCATION:1..477
      (D) OTHER INFORMATION:/codon_start= 1
         /function= "Antigen"
         /product= "TNF-alpha analog"
         /gene= "tnfP30-1"
         /standard_name= "TNF30-1"
   (xi) SEQUENCE DESCRIPTION: SEQ ID NO: 11:
   ATG GTC AGA TCA TCT TCT CGA ACC CCG AGT TTC AAC AAT TTT ACC GTA 48
   Met Val Arg Ser Ser Ser Arg Thr Pro Ser Phe Asn Asn Phe Thr Val
   160 165 170 175
   AGC TTT TGG CTC CGT GTA CCT AAG GTG TCG GCC TCG CAC CTG GAG CGC 96
   Ser Phe Trp Leu Arg Val Pro Lys Val Ser Ala Ser His Leu Glu Arg
   180 185 190
   CGG GCC AAT GCC CTC CTG GCC AAT GGC GTG GAG CTG AGA GAT AAC CAG 144
   Arg Ala Asn Ala Leu Leu Ala Asn Gly Val Glu Leu Arg Asp Asn Gln
   195 200 205
   CTG GTG GTG CCA TCA GAG GGC CTG TAC CTC ATC TAC TCC CAG GTC CTC 192
   Leu Val Val Pro Ser Glu Gly Leu Tyr Leu Ile Tyr Ser Gln Val Leu
   210 215 220
   TTC AAG GGC CAA GGC TGC CCC TCC ACC CAT GTG CTC CTC ACC CAC ACC 240
   Phe Lys Gly Gln Gly Cys Pro Ser Thr His Val Leu Leu Thr His Thr
   225 230 235
   ATC AGC CGC ATC GCC GTC TCC TAC CAG ACC AAG GTC AAC CTC CTC TCT 288
   Ile Ser Arg Ile Ala Val Ser Tyr Gln Thr Lys Val Asn Leu Leu Ser
   240 245 250 255
   GCC ATC AAG AGC CCC TGC CAG AGG GAG ACC CCA GAG GGG GCT GAG GCC 336
   Ala Ile Lys Ser Pro Cys Gln Arg Glu Thr Pro Glu Gly Ala Glu Ala
   260 265 270
   AAG CCC TGG TAT GAG CCC ATC TAT CTG GGA GGG GTC TTC CAG CTG GAG 384
   Lys Pro Trp Tyr Glu Pro Ile Tyr Leu Gly Gly Val Phe Gln Leu Glu
   275 280 285
   AAG GGT GAC CGA CTC AGC GCT GAG ATC AAT CGG CCC GAC TAT CTC GAC 432
   Lys Gly Asp Arg Leu Ser Ala Glu Ile Asn Arg Pro Asp Tyr Leu Asp
   290 295 300
   TTT GCC GAG TCT GGG CAG GTC TAC TTT GGG ATC ATT GCC CTG TAG 477
   Phe Ala Glu Ser Gly Gln Val Tyr Phe Gly Ile Ile Ala Leu *
   305 310 315
(2) INFORMATION FOR SEQ ID NO: 12:
   (i) SEQUENCE CHARACTERISTICS:
      (A) LENGTH: 159 amino acids
      (B) TYPE: amino acid
      (D) TOPOLOGY: linear
   (ii) MOLECULE TYPE: protein
   (xi) SEQUENCE DESCRIPTION: SEQ ID NO: 12:
   Met Val Arg Ser Ser Ser Arg Thr Pro Ser Phe Asn Asn Phe Thr Val
   1 5 10 15
   Ser Phe Trp Leu Arg Val Pro Lys Val Ser Ala Ser His Leu Glu Arg
   20 25 30
   Arg Ala Asn Ala Leu Leu Ala Asn Gly Val Glu Leu Arg Asp Asn Gln
   35 40 45
   Leu Val Val Pro Ser Glu Gly Leu Tyr Leu Ile Tyr Ser Gln Val Leu
   50 55 60
   Phe Lys Gly Gln Gly Cys Pro Ser Thr His Val Leu Leu Thr His Thr
   65 70 75 80
   Ile Ser Arg Ile Ala Val Ser Tyr Gln Thr Lys Val Asn Leu Leu Ser
   85 90 95
   Ala Ile Lys Ser Pro Cys Gln Arg Glu Thr Pro Glu Gly Ala Glu Ala
   100 105 110
   Lys Pro Trp Tyr Glu Pro Ile Tyr Leu Gly Gly Val Phe Gln Leu Glu
   115 120 125
   Lys Gly Asp Arg Leu Ser Ala Glu Ile Asn Arg Pro Asp Tyr Leu Asp
   130 135 140
   Phe Ala Glu Ser Gly Gln Val Tyr Phe Gly Ile Ile Ala Leu *
   145 150 155
(2) INFORMATION FOR SEQ ID NO: 13:
   (i) SEQUENCE CHARACTERISTICS:
      (A) LENGTH: 477 base pairs
      (B) TYPE: nucleic acid
      (C) STRANDEDNESS: double
      (D) TOPOLOGY: linear
   (ii) MOLECULE TYPE: DNA (genomic)
   (iii) HYPOTHETICAL: NO
   (iv) ANTI-SENSE: NO
   (vi) ORIGINAL SOURCE:
      (A) ORGANISM: Homo sapiens
   (ix) FEATURE:
      (A) NAME/KEY: CDS
      (B) LOCATION:1..477
      (D) OTHER INFORMATION: /codon**_**start= 1
         /function= "Antigen"
         /product= "TNF-alpha analog"
         /gene= "tnfP30-2"
         /standard_name= "TNF30-2"
   (xi) SEQUENCE DESCRIPTION: SEQ ID NO: 13:
   ATG GTC AGA TCA TCT TCT CGA ACC CCG AGT GAC AAG CCT GTA GCC CAT     48
   Met Val Arg Ser Ser Ser Arg Thr Pro Ser Asp Lys Pro Val Ala His
   160                 165                 170                 175
   GTT GTA GCA AAC CCT CAA GCT GAG GGG CAG CTC CAG TGG CTG AAC CGC     96
   Val Val Ala Asn Pro Gln Ala Glu Gly Gln Leu Gln Trp Leu Asn Arg
                   180                 185                 190
   CGG GCC AAT GCC CTC CTG GCC AAT TTC AAC AAC TTC ACA GTT AGC TTC    144
   Arg Ala Asn Ala Leu Leu Ala Asn Phe Asn Asn Phe Thr Val Ser Phe
               195                 200                 205
   TGG TTG AGG GTA CCA AAG GTC TCG GCC AGC CAC CTC GAG CAG GTC CTC    192
   Trp Leu Arg Val Pro Lys Val Ser Ala Ser His Leu Glu Gln Val Leu
           210                 215                 220
   TTC AAG GGC CAA GGC TGC CCC TCC ACC CAT GTG CTC CTC ACC CAC ACC    240
   Phe Lys Gly Gln Gly Cys Pro Ser Thr His Val Leu Leu Thr His Thr
       225                 230                 235
   ATC AGC CGC ATC GCC GTC TCC TAC CAG ACC AAG GTC AAC CTC CTC TCT    288
   Ile Ser Arg Ile Ala Val Ser Tyr Gln Thr Lys Val Asn Leu Leu Ser
   240                 245                 250                 255
   GCC ATC AAG AGC CCC TGC CAG AGG GAG ACC CCA GAG GGG GCT GAG GCC    336
   Ala Ile Lys Ser Pro Cys Gln Arg Glu Thr Pro Glu Gly Ala Glu Ala
                   260                 265                 270
   AAG CCC TGG TAT GAG CCC ATC TAT CTG GGA GGG GTC TTC CAG CTG GAG    384
   Lys Pro Trp Tyr Glu Pro Ile Tyr Leu Gly Gly Val Phe Gln Leu Glu
               275                 280                 285
   AAG GGT GAC CGA CTC AGC GCT GAG ATC AAT CGG CCC GAC TAT CTC GAC    432
   Lys Gly Asp Arg Leu Ser Ala Glu Ile Asn Arg Pro Asp Tyr Leu Asp
           290                 295                 300
   TTT GCC GAG TCT GGG CAG GTC TAC TTT GGG ATC ATT GCC CTC TAG        477
   Phe Ala Glu Ser Gly Gln Val Tyr Phe Gly Ile Ile Ala Leu  *
       305                 310                 315
(2) INFORMATION FOR SEQ ID NO: 14:
   (i) SEQUENCE CHARACTERISTICS:
      (A) LENGTH: 159 amino acids
      (B) TYPE: amino acid
      (D) TOPOLOGY: linear
   (ii) MOLECULE TYPE: protein
   (xi) SEQUENCE DESCRIPTION: SEQ ID NO: 14:
   Met Val Arg Ser Ser Ser Arg Thr Pro Ser Asp Lys Pro Val Ala His
     1               5                  10                  15
   Val Val Ala Asn Pro Gln Ala Glu Gly Gln Leu Gln Trp Leu Asn Arg
                    20                  25                  30
   Arg Ala Asn Ala Leu Leu Ala Asn Phe Asn Asn Phe Thr Val Ser Phe
                35                  40                  45
   Trp Leu Arg Val Pro Lys Val Ser Ala Ser His Leu Glu Gln Val Leu
            50                  55                  60
   Phe Lys Gly Gln Gly Cys Pro Ser Thr His Val Leu Leu Thr His Thr
    65                  70                  75                  80
   Ile Ser Arg Ile Ala Val Ser Tyr Gln Thr Lys Val Asn Leu Leu Ser
                    85                  90                  95
   Ala Ile Lys Ser Pro Cys Gln Arg Glu Thr Pro Glu Gly Ala Glu Ala
               100                 105                 110
   Lys Pro Trp Tyr Glu Pro Ile Tyr Leu Gly Gly Val Phe Gln Leu Glu
           115                 120                 125
   Lys Gly Asp Arg Leu Ser Ala Glu Ile Asn Arg Pro Asp Tyr Leu Asp
       130                 135                 140
   Phe Ala Glu Ser Gly Gln Val Tyr Phe Gly Ile Ile Ala Leu   *
   145                 150                 155
(2) INFORMATION FOR SEQ ID NO: 15:
   (i) SEQUENCE CHARACTERISTICS:
      (A) LENGTH: 477 base pairs
      (B) TYPE: nucleic acid
      (C) STRANDEDNESS: double
      (D) TOPOLOGY: linear
   (ii) MOLECULE TYPE: DNA (genomic)
   (iii) HYPOTHETICAL: NO
   (iv) ANTI-SENSE: NO
   (vi) ORIGINAL SOURCE:
      (A) ORGANISM: Homo sapiens
   (ix) FEATURE:
      (A) NAME/KEY: CDS
      (B) LOCATION:1..477
      (D) OTHER INFORMATION: /codon_start= 1
         /function= "Antigen"
         /product= "TNF-alpha analog"
         /gene= "tnfP30-3"
         /standard_name= "TNF30-3"
   (xi) SEQUENCE DESCRIPTION: SEQ ID NO: 15:
   ATG GTC AGA TCA TCT TCT CGA ACC CCG AGT GAC AAG CCT GTA GCC CAT    48
   Met Val Arg Ser Ser Ser Arg Thr Pro Ser Asp Lys Pro Val Ala His
   160                 165                 170                 175
   GTT GTA GCA AAC CCT CAA GCT GAG GGG CAG CTC CAG TGG CTG AAC CGC    96
   Val Val Ala Asn Pro Gln Ala Glu Gly Gln Leu Gln Trp Leu Asn Arg
                   180                 185                 190
   CGG GCC AAT GCC CTC CTG GCC AAT GGC GTG GAG CTG AGA GAT AAC CAG   144
   Arg Ala Asn Ala Leu Leu Ala Asn Gly Val Glu Leu Arg Asp Asn Gln
               195                 200                 205
   CTG GTG GTG CCA TCA GAG GGC CTG TAC CTC ATC TAC TCC CAG GTC CTC   192
   Leu Val Val Pro Ser Glu Gly Leu Tyr Leu Ile Tyr Ser Gln Val Leu
           210                 215                 220
   TTC AAC AAC TTT ACC GTC TCC TTC TGG CTT CGG GTA CCC AAG GTC AGC   240
   Phe Asn Asn Phe Thr Val Ser Phe Trp Leu Arg Val Pro Lys Val Ser
       225                 230                 235
   GCT AGC CAC CTC GAG GTC TCC TAC CAG ACC AAG GTC AAC CTC CTC TCT   288
   Ala Ser His Leu Glu Val Ser Tyr Gln Thr Lys Val Asn Leu Leu Ser
   240                 245                 250                 255
   GCC ATC AAG AGC CCC TGC CAG AGG GAG ACC CCA GAG GGG GCT GAG GCC   336
   Ala Ile Lys Ser Pro Cys Gln Arg Glu Thr Pro Glu Gly Ala Glu Ala
                   260                 265                 270
   AAG CCC TGG TAT GAG CCC ATC TAT CTG GGA GGG GTC TTC CAG CTG GAG   384
   Lys Pro Trp Tyr Glu Pro Ile Tyr Leu Gly Gly Val Phe Gln Leu Glu
               275                 280                 285
   AAG GGT GAC CGA CTC AGC GCT GAG ATC AAT CGG CCC GAC TAT CTC GAC   432
   Lys Gly Asp Arg Leu Ser Ala Glu Ile Asn Arg Pro Asp Tyr Leu Asp
           290                 295                 300
   TTT GCC GAG TCT GGG CAG GTC TAC TTT GGG ATC ATT GCC CTC TAG       477
   Phe Ala Glu Ser Gly Gln Val Tyr Phe Gly Ile Ile Ala Leu  *
       305                 310                 315
(2) INFORMATION FOR SEQ ID NO: 16:
   (i) SEQUENCE CHARACTERISTICS:
      (A) LENGTH: 159 amino acids
      (B) TYPE: amino acid
      (D) TOPOLOGY: linear
   (ii) MOLECULE TYPE: protein
   (xi) SEQUENCE DESCRIPTION: SEQ ID NO: 16:
(2) INFORMATION FOR SEQ ID NO: 17:
   (i) SEQUENCE CHARACTERISTICS:
      (A) LENGTH: 477 base pairs
      (B) TYPE: nucleic acid
      (C) STRANDEDNESS: double
      (D) TOPOLOGY: linear
   (ii) MOLECULE TYPE: DNA (genomic)
   (iii) HYPOTHETICAL: NO
   (iv) ANTI-SENSE: NO
   (vi) ORIGINAL SOURCE:
      (A) ORGANISM: Homo sapiens
   (ix) FEATURE:
      (A) NAME/KEY: CDS
      (B) LOCATION:1..477
      (D) OTHER INFORMATION/function= "Antigen"
         /product= "TNF-alpha analog"
         /gene= tnfP30-4"
         /standard_name= "TNF30-4"
   (xi) SEQUENCE DESCRIPTION: SEQ ID NO: 17:
   ATG GTC AGA TCA TCT TCT CGA ACC CCG AGT GAC AAG CCT GTA GCC CAT      48
   Met Val Arg Ser Ser Ser Arg Thr Pro Ser Asp Lys Pro Val Ala His
   160                 165                 170                 175
   GTT GTA GCA AAC CCT CAA GCT GAG GGG CAG CTC CAG TGG CTG AAC CGC      96
   Val Val Ala Asn Pro Gln Ala Glu Gly Gln Leu Gln Trp Leu Asn Arg
                   180                 185                 190
   CGG GCC AAT GCC CTC CTG GCC AAT GGC GTG GAG CTG AGA GAT AAC CAG     144
   Arg Ala Asn Ala Leu Leu Ala Asn Gly Val Glu Leu Arg Asp Asn Gln
               195                 200                 205
   CTG GTG GTG CCA TCA GAG GGC CTG TAC CTC ATC TAC TCC CAG GTC CTC     192
   Leu Val Val Pro Ser Glu Gly Leu Tyr Leu Ile Tyr Ser Gln Val Leu
           210                 215                 220
   TTC AAG GGC CAA GGC TGC CCC TCC ACC CAT GTG CTC CTC ACC CAC ACC     240
   Phe Lys Gly Gln Gly Cys Pro Ser Thr His Val Leu Leu Thr His Thr
       225                 230                 235
   ATC AGC CGC ATC GCC GTC TCC TAC CAG ACC AAG GTC AAC CTC CTC TCT     288
   Ile Ser Arg Ile Ala Val Ser Tyr Gln Thr Lys Val Asn Leu Leu Ser
   240                 245                 250                 255
   GCC ATC AAG AGC CCC TGC CAG AGG GAG ACC CCA TTT AAT AAT TTC ACC     336
   Ala Ile Lys Ser Pro Cys Gln Arg Glu Thr Pro Phe Asn Asn Phe Thr
                   260                 265                 270
   GTG TCC TTC TGG TTG CGC GTC CCT AAG GTA AGC GCT TCC CAC CTG GAG     384
   Val Ser Phe Trp Leu Arg Val Pro Lys Val Ser Ala Ser His Leu Glu
               275                 280                 285
   AAG GGT GAC CGA CTC AGC GCT GAG ATC AAT CGG CCC GAC TAT CTC GAC     432
   Lys Gly Asp Arg Leu Ser Ala Glu Ile Asn Arg Pro Asp Tyr Leu Asp
           290                 295                 300
   TTT GCC GAG TCT GGG CAG GTC TAC TTT GGG ATC ATT GCC CTC TAG         477
   Phe Ala Glu Ser Gly Gln Val Tyr Phe Gly Ile Ile Ala Leu  *
       305                 310                 315
(2) INFORMATION FOR SEQ ID NO: 18:
   (i) SEQUENCE CHARACTERISTICS:
      (A) LENGTH: 159 amino acids
      (B) TYPE: amino acid
      (D) TOPOLOGY: linear
   (ii) MOLECULE TYPE: protein
   (xi) SEQUENCE DESCRIPTION: SEQ ID NO: 18:
   Met Val Arg Ser Ser Ser Arg Thr Pro Ser Asp Lys Pro Val Ala His
    1               5                  10                  15
   Val Val Ala Asn Pro Gln Ala Glu Gly Gln Leu Gln Trp Leu Asn Arg
               20                  25                  30
   Arg Ala Asn Ala Leu Leu Ala Asn Gly Val Glu Leu Arg Asp Asn Gln
           35                  40                  45
   Leu Val Val Pro Ser Glu Gly Leu Tyr Leu Ile Tyr Ser Gln Val Leu
       50                  55                  60
   Phe Lys Gly Gln Gly Cys Pro Ser Thr His Val Leu Leu Thr His Thr
   65                  70                  75                  80
   Ile Ser Arg Ile Ala Val Ser Tyr Gln Thr Lys Val Asn Leu Leu Ser
                   85                  90                  95
   Ala Ile Lys Ser Pro Cys Gln Arg Glu Thr Pro Phe Asn Asn Phe Thr
               100                 105                 110
   Val Ser Phe Trp Leu Arg Val Pro Lys Val Ser Ala Ser His Leu Glu
           115                 120                 125
   Lys Gly Asp Arg Leu Ser Ala Glu Ile Asn Arg Pro Asp Tyr Leu Asp
       130                 135                 140
   Phe Ala Glu Ser Gly Gln Val Tyr Phe Gly Ile Ile Ala Leu  *
   145                 150                 155
(2) INFORMATION FOR SEQ ID NO: 19:
   (i) SEQUENCE CHARACTERISTICS:
      (A) LENGTH: 477 base pairs
      (B) TYPE: nucleic acid
      (C) STRANDEDNESS: double
      (D) TOPOLOGY: linear
   (ii) MOLECULE TYPE: DNA (genomic)
   (iii) HYPOTHETICAL: NO
   (iv) ANTI-SENSE: NO
   (vi) ORIGINAL SOURCE:
      (A) ORGANISM: Homo sapiens
   (ix) FEATURE:
      (A) NAME/KEY: CDS
      (B) LOCATION:1..477
      (D) OTHER INFORMATION:/codon_start= 1
         /function= "Antigen"
         /product= "TNF-alpha analog"
         /gene= "tnfP30-5"
         /standard_name= "TNF30-5"
   (xi) SEQUENCE DESCRIPTION: SEQ ID NO: 19:
   ATG GTC AGA TCA TCT TCT CGA ACC CCG AGT GAC AAG CCT GTA GCC CAT 48
   Met Val Arg Ser Ser Ser Arg Thr Pro Ser Asp Lys Pro Val Ala His
   160 165 170 175
   GTT GTA GCA AAC CCT CAA GCT GAG GGG CAG CTC CAG TGG CTG AAC CGC 96
   Val Val Ala Asn Pro Gln Ala Glu Gly Gln Leu Gln Trp Leu Asn Arg
   180 185 190
   CGG GCC AAT GCC CTC CTG GCC AAT GGC GTG GAG CTG AGA GAT AAC CAG 144
   Arg Ala Asn Ala Leu Leu Ala Asn Gly Val Glu Leu Arg Asp Asn Gln
   195 200 205
   CTG GTG GTG CCA TCA GAG GGC CTG TAC CTC ATC TAC TCC CAG GTC CTC 192
   Leu Val Val Pro Ser Glu Gly Leu Tyr Leu Ile Tyr Ser Gln Val Leu
   210 215 220
   TTC AAG GGC CAA GGC TGC CCC TCC ACC CAT GTG CTC CTC ACC CAC ACC 240
   Phe Lys Gly Gln Gly Cys Pro Ser Thr His Val Leu Leu Thr His Thr
   225 230 235
   ATC AGC CGC ATC GCC GTC TCC TAC CAG ACC AAG GTC AAC CTC CTC TCT 288
   Ile Ser Arg Ile Ala Val Ser Tyr Gln Thr Lys Val Asn Leu Leu Ser
   240 245 250 255
   GCC ATC AAG AGC CCC TGC CAG AGG GAG ACC CCA GAG GGG GCT GAG GCC 336
   Ala Ile Lys Ser Pro Cys Gln Arg Glu Thr Pro Glu Gly Ala Glu Ala
   260 265 270
   AAG CCC TGG TAT GAG CCC ATC TAT CTG GGA GGG GTC TTC CAG CTG GAG 384
   Lys Pro Trp Tyr Glu Pro Ile Tyr Leu Gly Gly Val Phe Gln Leu Glu
   275 280 285
   AAG GGT GAC CGA TTC AAC AAT TTC ACC GTA AGC TTC TGG CTT CGC GTC 432
   Lys Gly Asp Arg Phe Asn Asn Phe Thr Val Ser Phe Trp Leu Arg Val
   290 295 300
   CCT AAG GTG TCT GCG TCG CAC CTC GAA GGG ATC ATT GCC CTC TAG 477
   Pro Lys Val Ser Ala Ser His Leu Glu Gly Ile Ile Ala Leu *
   305 310 315
(2) INFORMATION FOR SEQ ID NO: 20:
   (i) SEQUENCE CHARACTERISTICS:
      (A) LENGTH: 159 amino acids
      (B) TYPE: amino acid
      (D) TOPOLOGY: linear
   (ii) MOLECULE TYPE: protein
   (xi) SEQUENCE DESCRIPTION: SEQ ID NO: 20:
   Met Val Arg Ser Ser Ser Arg Thr Pro Ser Asp Lys Pro Val Ala His
   1 5 10 15
   Val Val Ala Asn Pro Gln Ala Glu Gly Gln Leu Gln Trp Leu Asn Arg
   20 25 30
   Arg Ala Asn Ala Leu Leu Ala Asn Gly Val Glu Leu Arg Asp Asn Gln
   35 40 45
   Leu Val Val Pro Ser Glu Gly Leu Tyr Leu Ile Tyr Ser Gln Val Leu
   50 55 60
   Phe Lys Gly Gln Gly Cys Pro Ser Thr His Val Leu Leu Thr His Thr
   65 70 75 80
   Ile Ser Arg Ile Ala Val Ser Tyr Gln Thr Lys Val Asn Leu Leu Ser
   85 90 95
   Ala Ile Lys Ser Pro Cys Gln Arg Glu Thr Pro Glu Gly Ala Glu Ala
   100 105 110
   Lys Pro Trp Tyr Glu Pro Ile Tyr Leu Gly Gly Val Phe Gln Leu Glu
   115 120 125
   Lys Gly Asp Arg Phe Asn Asn Phe Thr Val Ser Phe Trp Leu Arg Val
   130 135 140
   Pro Lys Val Ser Ala Ser His Leu Glu Gly Ile Ile Ala Leu *
   145 150 155
(2) INFORMATION FOR SEQ ID NO: 21:
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (ix) FEATURE:
      (A) NAME/KEY: misc_feature
      (B) LOCATION:1..24
      (C) IDENTIFICATION METHOD: experimental
      (D) OTHER INFORMATION:/function= "Primer for PCR cloning of DNA encoding TNF-alpha"
         /product= "Primer binding to TNF-alpha gene"
         /evidence= EXPERIMENTAL
         /standard_name= "TNF-alpha Primer I"
         /label= Primer1
   (xi) SEQUENCE DESCRIPTION: SEQ ID NO: 21:
   GACAAGCCCA TGGTCAGATC ATCT 24
(2) INFORMATION FOR SEQ ID NO: 22:
   (i) SEQUENCE CHARACTERISTICS:
      (A) LENGTH: 30 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (ix) FEATURE:
      (A) NAME/KEY: misc_feature
      (B) LOCATION: 1..30
      (C) IDENTIFICATION METHOD: experimental
      (D) OTHER INFORMATION:/function "Primer for PCR cloning of DNA encoding TNF-alpha"
         /product= "Primer binding to TNF-alpha gene"
         /evidence= EXPERIMENTAL
         /standard_name- "TNF-alpha Primer It"
         /label= Primer2
   (xi) SEQUENCE DESCRIPTION: SEQ ID NO: 22:
   TCTCTAGAGG GCAATGATCC CAAAGTAGAC 30
(2) INFORMATION FOR SEQ ID NO: 23:
   (i) SEQUENCE CHARACTERISTICS:
      (A) LENGTH: 21 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: HO
   (ix) FEATURE:
      (A) NAME/KEY: mise feature
      (B) LOCATION:1..21
      (C) IDENTIFICATION METHOD: experimental
      (D) OTHER INFORMATION:/function= "Primer for PCR cloning of DNA encoding TNF-alpha"
         /product= "Primer binding to TNF-alpha gene"
         /evidence= EXPERIMENTAL
         /standard_name= "TNF-alpha Primer III"
         /label= Primer3
   (xi) SEQUENCE DESCRIPTION: SEQ ID NO: 23:
   CCCAAAGTAG ACCTGCCCAG A 21
(2) INFORMATION FOR SEQ ID NO: 24:
   (i) SEQUENCE CHARACTERISTICS:
      (A) LENGTH: 69 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (vi) ORIGINAL SOURCE:
      (A) ORGANISM: Homo sapiens
   (ix) FEATURE:
      (A) NAME/KEY: insertion_seq
      (B) LOCATION:7..51
      (C) IDENTIFICATION METHOD: experimental
      (D) OTHER INFORMATION:/function= "Primer for PCR cloning of DHA encoding THE-alpha analog"
         /evidence= EXPERIMENTAL
         /organism= "Homo sapiens"
         /standard_name= "Primer "mut2-1""
         /label= mut2-1
         /note= "Primer "mut2-1" is a synthetically synthesised 69-mer oligonucleotide comprising DNA encoding the human T cell epitope P2 between stretches of DHA homologous to stretches of the human TNF-alpha gene"
   (xi) SEQUENCE DESCRIPTION: SEQ ID NO: 24:
   ACCCCGAGTC AGTACATTAA AGCCAATTCT AAATTCATCG GTATAACTGA GCTGCAGCTC 60
   CAGTGGCTG 69
(2) INFORMATION FOR SEQ ID NO: 25:
   (i) SEQUENCE CHARACTERISTICS:
      (A) LENGTH: 73 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (vi) ORIGINAL SOURCE:
      (A) ORGANISM: Homo sapiens
   (ix) FEATURE:
      (A) HAME/KEY: insertion seq
      (B) LOCATION: 15..59
      (C) IDENTIFICATION METHOD: experimental
      (D) OTHER INFORMATION:/function= "Primer for PCR cloning of DNA encoding TNF-alpha analog"
         /evidence= EXPERIMENTAL
         /organism = "Homo sapiens"
         /standard_name= "Primer "mut2-3""
         /label = mut2-3
         /note = "Primer "mut2-3" is a synthetically synthesised 73-mer oligonucleotide comprising DNA encoding the human T cell epitope P2 between stretches of DNA homologous to stretches of the human TNF-alpha gene"
   (xi) SEQUENCE DESCRIPTION: SEQ ID NO: 25:
   CCCAGGTCCT CTTCCAGTAC ATAAAGGCCA ACTCCAAGTT TATCGCCATC ACCGAGCTCA 60
   TCAGCCGCAT CGC 73
(2) INFORMATION FOR SEQ ID NO: 26:
   (i) SEQUENCE CHARACTERISTICS:
      (A) LENGTH: 75 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (vi) ORIGINAL SOURCE:
      (A) ORGANISM: Homo sapiens
   (ix) FEATURE:
      (A) NAME/KEY: insertion_seq
      (B) LOCATION:12..56
      (C) IDENTIFICATION METHOD: experimental
      (D) OTHER INFORMATION:/function= "Primer for PCR cloning of DNA encoding TNF-alpha analog"
         /evidence= EXPERIMENTAL
         /organism= "Homo sapiens"
         /standard_name= "Primer "mut2-4""
         /label= mut2-4
         /note= "Primer "mut2-4" is a synthetically synthesised 75-mer oligonucleotide comprising DNA encoding the human T cell epitope P2 between stretches of DNA homologous to stretches of the human TNF-alpha gene"
   (xi) SEQUENCE DESCRIPTION: SEQ ID NO: 26:
   AGTCGGTCAC CGAGCTCCGT GATGCCGATG AATTTCGAAT TGGCCTTGAT ATACTGGGGC 60
   TTGGCCTCAG CCCCC 75
(2) INFORMATION FOR SEQ ID NO: 27:
   (i) SEQUENCE CHARACTERISTICS:
      (A) LENGTH: 75 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (vi) ORIGINAL SOURCE:
      (A) ORGANISM: Homo sapiens
   (ix) FEATURE:
      (A) NAME/KEY: insertion_seq
      (B) LOCATION:8..52
      (C) IDENTIFICATION METHOD: experimental
      (D) OTHER INFORMATION:/function= "Primer for PCR cloning of DNA encoding TNF-alpha analog"
         /evidence= EXPERIMENTAL
         /organism= "Homo sapiens"
         /standard_name= "Primer "mut2-5""
         /label= mut2-5
         /note= "Primer "mut2-5" is a synthetically synthesised 75-mer oligonucleotide comprising DNA encoding the human T cell epitope P2 between stretches of DNA homologous to stretches of the human TNF-alpha gene"
   (xi) SEQUENCE DESCRIPTION: SEQ ID NO: 27:
   GAAGGGTGAC CGACAGTACA TTAAGGCCAA TTCGAAGTTC ATTGGCATCA CTGAGCTGTC 60
   TGGGCAGGTC TACTT 75
(2) INFORMATION FOR SEQ ID NO: 28:
   (i) SEQUENCE CHARACTERISTICS:
      (A) LENGTH: 80 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (vi) ORIGINAL SOURCE:
      (A) ORGANISM: Homo sapiens
   (ix) FEATURE:
      (A) NAME/KEY: insertion_seq
      (B) LOCATION:14..58
      (C) IDENTIFICATION METHOD: experimental
      (D) OTHER INFORMATION :/function= "Primer for PCR cloning of DNA encoding TNF-alpha analog"
         /evidence= EXPERIMENTAL
         /organism= "Homo sapiens"
         /standard_name= "Primer "mut2-7""
         /label= mut2-7
         /note= "Primer "mut2-7" is a synthetically synthesised 80-mer oligonucleotide comprising DNA encoding the human T cell epitope P2 between stretches of DNA homologous to stretches of the human TNF-alpha gene"
   (xi) SEQUENCE DESCRIPTION: SEQ ID NO: 28:
   CACCCATGTG CTCCAGTACA TCAAAGCTAA CTCCAAATTC ATCGGCATCA CCGAACTGGT 60
   TAACCTCCTC TCTGCCATCA 80
(2) INFORMATION FOR SEQ ID NO: 29:
   (i) SEQUENCE CHARACTERISTICS:
      (A) LENGTH: 96 base pairs
      (B) TYPE: nucleic acid
      (C) STRANDEDNESS: single
      (D) TOPOLOGY: Linear
   (ii) MOLECULE TYPE: DNA (genomic)
   (iii) HYPOTHETICAL: NO
   (iv) ANTI-SENSE: NO
   (vi) ORIGINAL SOURCE:
      (A) ORGANISM: Homo sapiens
   (ix) FEATURE:
      (A) NAME/KEY: insertion_seq
      (B) LOCATION:10..72
      (C) IDENTIFICATION METHOD: experimental
      (D) OTHER INFORMATION/function= "Primer tor PCR cloning of DNA encoding TNF-alpha analog"
         /evidence= EXPERIMENTAL
         /organism= "Homo sapiens"
         /standard_name= "Primer "mut30-1""
         /label= mut30-1
         /note= "Primer "mut30-1" is a synthetically synthesised 96-mer oligonucleotide comprising DNA encoding the human T cell epitope P30 between stretches of DNA homologous to stretches of the human TNF-alpha gene"
   (xi) SEQUENCE DESCRIPTION: SEQ ID NO: 29:
   ACCCCGAGTT TCAACAATTT TACCGTAAGC TTTTGGCTCC GTGTACCTAA GGTGTCGGCC 60
   TCGCACCTGG AGCGCCGGGC CAATGCCCTC CTGGCC 96
(2) INFORMATION FOR SEQ ID NO: 30:
   (i) SEQUENCE CHARACTERISTICS:
      (A) LENGTH: 100 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (vi) ORIGINAL SOURCE:
      (A) ORGANISM: Homo sapiens
   (ix) FEATURE:
      (A) NAME/KEY: insertion seq
      (B) LOCATION:12..74
      (C) IDENTIFICATION METHOD: experimental
      (D) OTHER INFORMATION:/function= "Primer for PCR cloning of DNA encoding TNF-alpha analog"
         /evidence= EXPERIMENTAL
         /organism= "Homo sapiens"
         /standard name= "Primer "mut 30-2" "
         /label= mut30-2
         /note= "Primer "mut30-2" is a synthetically synthesised 100-mer oligonucleotide comprising DNA encoding human T cell epitope P30 between stretches of DNA homologous to stretches of the human TNF-alpha gene"
   (xi) SEQUENCE DESCRIPTION : SEQ ID NO: 30:
   TCCTGGCCAA TTTCAACAAC TTCACAGTTA GCTTCTGGTT GAGGGTACCA AAGGTCTCGG 60
   CCAGCCACCT CGAGCAGGTC CTCTTCAAGG GCCAAGGCTG 100
(2) INFORMATION FOR SEQ ID NO: 31:
   (i) SEQUENCE CHARACTERISTICS:
      (A) LENGTH: 100 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (vi) ORIGINAL SOURCE:
      (A) ORGANISM: Homo sapiens
   (ix) FEATURE:
      (A) NAME/KEY: insertion seq
      (B) LOCATION:12..74
      (C) IDENTIFICATION METHOD: experimental
      (D) OTHER INFORMATION:/function= "Primer for PCR cloning of DNA encoding TNF-alpha analog"
         /evidence= EXPERIMENTAL
         /organism= "Homo sapiens"
         /standard_name= "Primer "mut30-3""
         /label= mut30-3
         /note= "Primer "mut30-3" is a synthetically synthesised 100-mer oligonucleotide comprising DNA encoding human T cell epitope P30 between stretches of DNA homologous to stretches of the human TNF-alpha gene"
   (xi) SEQUENCE DESCRIPTION: SEQ ID NO: 31:
   CCCAGGTCCT CTTCAACAAC TTTACCGTCT CCTTCTGGCT TCGGGTACCC AAGGTCAGCG 60
   CTAGCCACCT CGAGGTCTCC TACCAGACCA AGGTCAACCT 100
(2) INFORMATION FOR SEQ ID NO: 32:
   (i) SEQUENCE CHARACTERISTICS:
      (A) LENGTH: 100 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (vi) ORIGINAL SOURCE:
      (A) ORGANISM: Homo sapiens
   (ix) FEATURE:
      (A) NAME/KEY: insertion seq
      (B) LOCATION:15..77
      (C) IDENTIFICATION METHOD: experimental
      (D) OTHER INFORMATION:/function= "Primer for PCR cloning of DNA encoding TNF-alpha analog"
         /evidence= EXPERIMENTAL
         /organism= "Homo sapiens''
         /standard_name= "Primer "mut30-4""
         /label= mut30-4
         /note= "Primer "mut30-4" is a synthetically synthesised 100-mer oligonucleotide comprising DNA encoding human T cell epitope P30 between stretches of DNA homologous to stretches of the human TNF-alpha gene"
   (xi) SEQUENCE DESCRIPTION: SEQ ID NO: 32:
   AGTCGGTCAC CCTTCTCCAG GTGGGAAGCG CTTACCTTAG GGACGCGCAA CCAGAAGGAC 60
   ACGGTGAAAT TATTAAATGG GGTCTCCCTC TGGCAGGGGC 100
(2) INFORMATION FOR SEQ ID NO: 33:
   (i) SEQUENCE CHARACTERISTICS:
      (A) LENGTH: 100 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (vi) ORIGINAL SOURCE:
      (A) ORGANISM: Homo sapiens
   (ix) FEATURE:
      (A) NAME/KEY: insertion_seq
      (B) LOCATION: 14..76
      (C) IDENTIFICATION METHOD: experimental
      (D) OTHER INFORMATION: /function= "Primer for PCR cloning of DNA encoding TNF-alpha analog"
         /evidence= EXPERIMENTAL
         /organism= "Homo sapiens"
         /standard_name= "Primer "mut30-5""
         /label= mut30-5
         /note= "Primer "mut30-5" is a synthetically synthesised 100-mer oligonucleotide comprising DNA encoding human T cell epitope P30 between stretches of DNA homologous to stretches of the human TNF-alpha gene"
   (xi) SEQUENCE DESCRIPTION: SEQ ID NO: 33:
   GAAGGGTGAC CGATTCAACA ATTTCACCGT AAGCTTCTGG CTTCGCGTCC CTAAGGTGTC    60
   TGCGTCGCAC CTCGAAGGGA TCATTGCCCT CTAGAGTCGA                         100
(2) INFORMATION FOR SEQ ID NO: 34:
   (i) SEQUENCE CHARACTERISTICS:
      (A) LENGTH: 25 amino acids
      (B) TYPE: amino acid
      (C) STRANDEDNESS:
      (D) TOPOLOGY: linear
   (ii) MOLECULE TYPE: peptide
   (iii) HYPOTHETICAL: HO
   (v) FRAGMENT TYPE: internal
   (vi) ORIGINAL SOURCE:
      (A) ORGANISM: Homo sapiens
   (ix) FEATURE:
      (A) NAME/KEY: Peptide
      (B) LOCATION:1..25
      (D) OTHER INFORMATION:/label= Pep2-1
         /note= "Pep2-1 is a synthetically prepared truncated form of a TNF-alpha analog comprising the human T cell epitope P2 and flanking portions of human TNF-alpha"
   (xi) SEQUENCE DESCRIPTION: SEQ ID NO: 34:
   Ser Arg Thr Pro Ser Gln Tyr Ile Lys Ala Asn Ser Lys Phe Ile Gly
   1               5                   10                  15
   Ile Thr Glu Leu Gln Leu Gln Trp Leu
               20                  25
(2) INFORMATION FOR SEQ ID NO: 35:
   (i) SEQUENCE CHARACTERISTICS:
      (A) LENGTH: 25 amino acids
      (B) TYPE: amino acid
      (C) STRANDEDNESS:
      (D) TOPOLOGY: linear
   (ii) MOLECULE TYPE: peptide
   (iii) HYPOTHETICAL: NO
   (v) FRAGMENT TYPE: internal
   (vi) ORIGINAL SOURCE:
      (A) ORGANISM: Homo sapiens
   (ix) FEATURE:
      (A) NAME/KEY: Peptide
      (B) LOCATION:1..25
      (D) OTHER INFORMATION:/label= Pep2-3
         /note= "Pep2-3 is a synthetically prepared truncated form of a TNF-alpha analog comprising the human T cell epitope P2 and flanking portions of human TNF-alpha"
   (xi) SEQUENCE DESCRIPTION: SEQ ID NO: 35:
   Ser Gln Val Leu Phe Gln Tyr Ile Lys Ala Asn Ser Lys Phe Ile Gly
   1               5                   10                  15
   Ile Thr Glu Leu Ile Ser Arg Ile Ala
               20                  25
(2) INFORMATION FOR SEQ ID NO: 36:
   (i) SEQUENCE CHARACTERISTICS:
      (A) LENGTH: 25 amino acids
      (B) TYPE: amino acid
      (C) STRANDEDNESS:
      (D) TOPOLOGY: linear
   (ii) MOLECULE TYPE: peptide
   (iii) HYPOTHETICAL: NO
   (v) FRAGMENT TYPE: internal
   (vi) ORIGINAL SOURCE:
      (A) ORGANISM: Homo sapiens
   (ix) FEATURE:
      (A) NAME/KEY: Peptide
      (B) LOCATION: 1..25
      (D) OTHER INFORMATION:/label= Pep2-4
         /note= "Pep2-4 is a synthetically prepared truncated form of a TNF-alpha analog comprising the human T cell epitope P2 and flanking portions of human TNF-alpha"
   (xi) SEQUENCE DESCRIPTION: SEQ ID NO: 36:
   Ala Glu Ala Lys Pro Gln Tyr Ile Lys Ala Asn Ser Lys Phe Ile Gly
   1               5                   10                  15
   Ile Thr Glu Leu Gly Asp Arg Leu Ser
               20                  25
(2) INFORMATION FOR SEQ ID NO: 37:
   (i) SEQUENCE CHARACTERISTICS:
      (A) LENGTH: 25 amino acids
      (B) TYPE: amino acid
      (C) STRANDEDNESS:
      (D) TOPOLOGY: linear
   (ii) MOLECULE TYPE: peptide
   (iii) HYPOTHETICAL: NO
   (v) FRAGMENT TYPE: internal
   (vi) ORIGINAL SOURCE:
      (A) ORGANISM: Homo sapiens
   (ix) FEATURE:
      (A) HAME/KEY: Peptide
      (B) LOCATION: 1..25
      (D) OTHER INFORMATION:/label= Pep2-5
         /note= "Pep2-5 is a synthetically prepared truncated form of a TNF-alpha analog comprising the human T cell epitope P2 and flanking portions of human TNF-alpha"
   (xi) SEQUENCE DESCRIPTION: SEQ ID NO: 37:
   Glu Lys Gly Asp Arg Gln Tyr Ile Lys Ala Asn Ser Lys Phe Ile Gly
   1               5                   10                  15
   Ile Thr Glu Leu Ser Gly Gln Val Tyr
               20                  25
(2) INFORMATION FOR SEQ ID NO: 38:
   (i) SEQUENCE CHARACTERISTICS:
      (A) LENGTH: 31 amino acids
      (B) TYPE: amino acid
      (C) STRANDEDNESS:
      (D) TOPOLOGY: linear
   (ii) MOLECULE TYPE: peptide
   (iii) HYPOTHETICAL: NO
   (v) FRAGMENT TYPE: internal
   (vi) ORIGINAL SOURCE:
      (A) ORGANISM: Homo sapiens
   (ix) FEATURE:
      (A) NAME/KEY: Peptide
      (B) LOCATION:1..31
      (D) OTHER INFORMATION:/label= Pep30-1
         /note= "Pep30-1 is a synthetically prepared truncated form of a TNF-alpha analog comprising human T cell epitope P30 and flanking portions of human TNF-alpha"
   (xi) SEQUENCE DESCRIPTION: SEQ ID NO: 38:
   Ser Arg Thr Pro Ser Phe Asn Asn Phe Thr Val Ser Phe Trp Leu Arg
   1               5                   10                  15
   Val Pro Lys Val Ser Ala Ser His Leu Glu Arg Arg Ala Asn Ala
               20                  25                  30
(2) INFORMATION FOR SEQ ID NO: 39:
   (i) SEQUENCE CHARACTERISTICS:
      (A) LENGTH: 31 amino acids
      (B) TYPE: amino acid
      (C) STRANDEDNESS:
      (D) TOPOLOGY: linear
   (ii) MOLECULE TYPE: peptide
   (iii) HYPOTHETICAL : NO
   (v) FRAGMENT TYPE: internal
   (vi) ORIGINAL SOURCE:
      (A) ORGANISM: Homo sapiens
   (ix) FEATURE:
      (A) NAME/KEY: Peptide
      (B) LOCATION:1..31
      (D) OTHER INFORMATION:label= Pep 30-2
         /note= "Pep30-2 in a synthetically prepared truncated form of a TNF-alpha analog comprising the human T cell epitope P30 and flanking portions of human TNF-alpha"
   (xi) SEQUENCE DESCRIPTION: SEQ ID NO: 39:
   Ala Leu Leu Ala Asn Phe Asn Asn Phe Thr Val Ser Phe Trp Leu Arg
   1 5 10 15
   Val Pro Lys Val Ser Ala Ser His Leu Glu Gln Val Leu Phe Lys
   20 25 30
(2) INFORMATION FOR SEQ ID NO: 40:
   (i) SEQUENCE CHARACTERISTICS :
      (A) LENGTH: 31 amino acids
      (B) TYPE: amino acid
      (C) STRANDEDNESS:
      (D) TOPOLOGY: linear
   (ii) MOLECULE TYPE: peptide
   (iii) HYPOTHETICAL: NO
   (v) FRAGMENT TYPE: internal
   (vi) ORIGINAL SOURCE:
      (A) ORGANISM: Homo sapiens
   (ix) FEATURE:
      (A) NAME/KEY: Peptide
      (B) LOCATION: 1..31
      (D) OTHER INFORMATION:/label= Pep30-3
         /note= "Pep30-3 is a synthetically prepared truncated form of a TNF-alpha analog comprising the human T cell epitope P30 and flanking portions of human TNF-alpha"
   (xi) SEQUENCE DESCRIPTION: SEQ ID NO: 40:
   Tyr Ser Gln Val Leu Phe Asn Asn Phe Thr Val Ser Phe Trp Leu Arg
   1 5 10 15
   Val Pro Lys Val Ser Ala Ser His Leu Glu Val Ser Tyr Gln Thr
   20 25 30
(2) INFORMATION FOR SEQ ID NO: 41:
   (i) SEQUENCE CHARACTERISTICS:
      (A) LENGTH: 31 amino acids
      (B) TYPE: amino acid
      (C) STRANDEDNESS:
      (D) TOPOLOGY: linear
   (ii) MOLECULE TYPE: peptide
   (iii) HYPOTHETICAL: NO
   (v) FRAGMENT TYPE: internal
   (vi) ORIGINAL SOURCE:
      (A) ORGANISM: Homo sapiens
   (ix) FEATURE:
      (A) NAME/KEY: Peptide
      (B) LOCATION:1..31
      (D) OTHER INFORMATION:/label= Pep30-4
         /note= "Pep30-4 is a synthetically prepared truncated form of a TNF-alpha analog comprising the human T cell epitope P30 and flanking portions of human TNF-alpha"
   (xi) SEQUENCE DESCRIPTION: SEQ ID NO: 41:
   Gln Arg Glu Thr Pro Phe Asn Asn Phe Thr Val Ser Phe Trp Leu Arg
   1               5                   10                  15
   Val Pro Lys Val Ser Ala Ser His Leu Glu Lys Gly Asp Arg Leu
               20                  25                  30
(2) INFORMATION FOR SEQ ID NO: 42:
   (i) SEQUENCE CHARACTERISTICS:
      (A) LENGTH: 31 amino acids
      (B) TYPE: amino acid
      (C) STRANDEDNESS:
      (D) TOPOLOGY: linear
   (ii) MOLECULE TYPE: peptide
   (iii) HYPOTHETICAL: 110
   (v) FRAGMENT TYPE: internal
   (vi) ORIGINAL SOURCE:
      (A) ORGANISM: Homo sapiens
   (ix) FEATURE:
      (A) NAME/KEY: Peptide
      (B) LOCATION:1..31
      (D) OTHER INFORMATION:/label= Pep30-5
         /note= "Pep30-5 is a synthetically prepared truncated from of a TNF-alpha analog comprising the human T cell epitope P30 and flanking portions of human TNF-alpha"
   (xi) SEQUENCE DESCRIPTION: SEQ ID NO: 42:
   Glu Lys Gly Asp Arg Phe Asn Asn Phe Thr Val Ser Phe Trp Leu Arg
   1               5                   10                  15
   Val Pro Lys Val Ser Ala Ser His Leu Glu Gly Ile Ile Ala Leu
               20                  25                  30

## Claims

1. A modified human TNFα molecule capable of raising neutralizing antibodies towards wild-type human TNFα following administration of said modified TNFα molecule to a human host, wherein at least one segment of the human TNFα molecule has been substituted by at least one peptide known to contain an immunodominant T cell epitope, or a truncated form of said molecule containing an immunodominant T-cell epitope and one or both flanking regions of the human TNFα molecule comprising at least one TNFα B cell epitope, wherein the substitution is introduced in anyone of the strands of the front β sheet, in anyone of the connecting loops and/or in any one of the B', I or D strands of the back β-sheet, wherein the substitution has been made in regions of the TNFα molecule so as to preserve the β-sheet structure of the B and G strands, and wherein the substitution leads to inactivation of the biological activity of human TNFα when tested in the L929 bioassay.

2. Modified human TNFα molecule according to claim 1, wherein the substitution does not comprise any complete strand of the back β-sheet.

3. Modified human TNFα molecule according to claim 1 or 2, wherein the substitution comprises at least a segment of the H strand of the front β-sheet and of the connecting loop to the I strand of the back β sheet, a segment of the H and I strands and the entire connecting loop, a segment of the D strand of the back β sheet and at least a segment of the E strand of the front β-sheet and the entire connecting loop, the entire C' and C strands of the front β-sheet and a segment of the D strand of the back β-sheet, or at least a segment of the E strand of the front β-sheet and of one or both the connecting loops.

4. Modified human TNFα molecule according to any one of claims 1-3, wherein the substitution has been made in regions of the TNFα molecule which comprise the strands of the front β sheets and/or the connecting loops so as to essentially preserve the β-sheet structure of any of the strands of the back β-sheet.

5. Modified human TNFα molecule according to claim 3, wherein the substitution has been made in regions of the TNFα molecule, which comprise a segment of the D strand of the back β-sheet.

6. Modified human TNFα molecule according to claim 3, wherein the substitution comprises at least a segment of the H strand of the front β-sheet and of the connecting loop to the I strand, preferably amino acids 132 to 146.

7. Modified human TNFα molecule according to claim 3, wherein the substitution comprises segments of the H and I strands and the entire connecting loop, preferably amino acids 132 to 152

8. Modified human TNFα molecule according to claim 3, wherein the substitution comprises a segment of the D strand, at least a segment of the E strand and the entire connecting loop, preferably amino acids 65 to 79 or 64 to 84.

9. Modified human TNFα molecule according to claim 3, wherein the substitution comprises the entire C' and C strands and a segment of the D strand, preferably amino acids 40 to 60.

10. Modified human TNFα molecule according to claim 3, wherein the substitution comprises at least a segment of the E strand of the front β-sheet and of one or both of the connecting loops, preferably amino acids 76 to 90.

11. Modified TNFα molecule according to any of the previous claims, wherein neutralizing antibodies raised against said modified TNFα molecule in a suitable host is able to significantly inhibit the activity of native TNFα in the L929 bioassay, and/or wherein said antibodies significantly inhibit the binding of wild-type human TNFα to the 55 kD TNFα receptor 1 (TNFα-R55) or to the 75 kD TNFα receptor (TNFα-R75).

12. Modified human TNFα molecule according to any of claims 1-11, wherein the inserted T cell epitope is promiscuous and known to be immunogenic in a majority of human HLA class II types.

13. Modified human TNFα molecule according to claim 12, wherein the epitope is derived from Tetanus toxoid, preferably epitope P2 and/or P30.

14. Modified TNFα according to claim 13, having the amino acid sequence shown in SEQ ID NO:8.

15. Modified TNFα according to claim 13, having the amino acid sequence shown in SEQ ID NO:10.

16. Modified TNFα molecule according to claim 13, having the amino acid sequence shown in SEQ ID NO:4 or SEQ ID NO:16.

17. Modified TNFα according to claim 13, having the amino acid sequence shown in SEQ ID NO:20.

18. Modified TNFα according to claim 13, having the amino acid sequence shown in SEQ ID NO:14.

19. Dimers, oligomers or multimers of the modified human TNFα molecule according to anyone of claims 1-18.

20. An isolated DNA molecule that codes for a modified TNFα molecule according to any one of claims 1-18.

21. A vector which comprises the isolated DNA molecule according to claim 20.

22. An expression vector, which comprises the isolated DNA molecule according to claim 21 operatively linked to an expression control sequence.

23. A host cell, which is transformed with the expression vector of claim 22.

24. A host cell according to claim 23, which is selected from strains of bacteria, yeast, or other fungi and insect, mammalian or avian cell lines.

25. A method of producing a modified human TNFα molecule according to anyone of claims 1-18, which comprises growing the host cells of claim 23 or 24 under suitable conditions permitting production of the modified TNFα and recovering the modified TNFα so produced.

26. A modified human TNFα molecule according to any of claims 1-18 in the form of a fusion protein with an adjuvant molecule.

27. Modified human TNFα molecule according to claim 26, wherein the adjuvant molecule is an immunologically active adjuvant.

28. Modified human TNFα molecule according to claim 26, wherein the adjuvant molecule is GM-SCF, HSP70 or interleukin.

29. A vaccine against TNFα, comprising one or more modified human TNFα molecules according to any of claims 1-20 and optionally a pharmaceutically acceptable adjuvant.

30. A vaccine according to claim 29, wherein the pharmaceutically acceptable adjuvant is aluminium phosphate, aluminium hydroxide, calcium phosphate, muramyl dipeptide or iscom.

31. A vaccine according to claim 29 or 30 for the prevention or treatment of diseases promoted by TNFα release or activity such as chronic inflammatory diseases.

32. A vaccine according to claim 31, wherein the chronic inflammatory diseases are rheumatoid arthritis and inflammatory bowel diseases.

33. A vaccine according to claim 32, wherein the inflammatory bowel diseases are Crohn's disease and Colitis Ulcerosa.

34. A vaccine against TNFα comprising isolated DNA, which codes for the modified human TNFα molecule according to anyone of claims 1-18 inserted in a suitable expression vector.

35. A vaccine according to claim 34 containing a construct comprising a non-infectious non-integrating DNA sequence encoding a modified TNFα molecule according to any of claims 1-18 operatively linked to a promoter sequence which can control the expression of said DNA sequence in humans, in an amount sufficient that uptake of said construct occurs, and sufficient expression occurs to induce a neutralizing antibody response against TNFα.

36. A vaccine according to claim 33 or 34, comprising a viral expression vector.

37. A vaccine according to claim 36, wherein the viral expression vector is a retroviral expression vector.

38. A vaccine according to anyone of claims 29-37 for oral or parenteral administration.

39. A vaccine according to claim 38, wherein the vaccine is for subcutaneous, intramuscular or intradermal administration.

40. Use of at least one modified TNFα molecule according to claim 1-18, optionally in combination with a suitable adjuvant or carrier molecule, in the manufacture of a medicament for the treatment or prevention of chronic inflammation, cancer, cachexia, disseminated sclerosis, diabetes, psoriasis, osteoporosis or asthma.

## Patentansprüche

1. Modifiziertes humanes TNFα-Molekül, das fähig ist, neutralisierende Antikörper gegen humanen Wildtyp-TNFα nach Verabreichung des modifizierten TNFα-Moleküls an einen humanen Wirt zu erzeugen, wobei wenigstens ein Segment des humanen TNFα-Moleküls durch wenigstens ein Peptid, von dem bekannt ist, dass es ein immundominantes T-Zellepitop enthält, oder eine verkürzte Form des Moleküls, die ein immundominantes T-Zellepitop und eine oder beide flankierenden Regionen des humanen TNFα-Moleküls, die wenigstens ein TNFα-B-Zellepitop umfasst (umfassen), enthält, ersetzt worden ist, wobei die Substitution in einen der Stränge des vorderen β-Faltblatts, in eine der Verknüpfungsschleifen und/oder in einen der B'-, I- oder D-Stränge des hinteren β-Faltblatts eingeführt wurde, wobei die Substitution in Regionen des TNFα-Moleküls so durchgeführt wurde, dass die β-Faltblattstruktur der B- und G-Stränge konserviert wurden, und wobei die Substitution zu einer Inaktivierung der biologischen Aktivität von humanem TNFα führt, wenn in einem L929-Bioassay getestet wird.

2. Modifiziertes humanes TNFα-Molekül nach Anspruch 1, wobei die Substitution keinen vollständigen Strang des hinteren β-Faltblatts umfasst.

3. Modifiziertes humanes TNFα-Molekül nach Anspruch 1 oder 2, wobei die Substitution wenigstens ein Segment des H-Strangs des vorderen β-Faltblatts und der Verknüpfungsschleife zu dem I-Strang des hinteren β-Faltblatts, ein Segment der H- und I-Stränge und die gesamte Verknüpfungsschleife, ein Segment des D-Strangs des hinteren β-Faltblatts und wenigstens ein Segment des I-Strangs des vorderen β-Faltblatts und die ganze Verknüpfungeschleife, die ganzen C'- und C-Stränge des vorderen β-Faltblatts und ein Segment des D-Strangs des hinteren β-Faltblatts oder wenigstens ein Segment des E-Strangs des vorderen β-Faltblatts und einer oder beider Verknüpfungaschleifen umfasst.

4. Modifiziertes humanes TNFα-Molekül nach einem der Ansprüche 1 bis 3, wobei die Substitution in Regionen des TNFα-Moleküls durchgeführt wurde, die die Stränge der vorderen β-Faltblätter und/oder die Verknüpfungsschleifen umfassen, so dass die β-Faltblattstruktur eines der Stränge des hinteren β-Faltblatts im Wesentlichen konserviert wird.

5. Modifiziertes humanes TNFα-Molekül nach Anspruch 3, wobei die Substitution in Regionen des TNFα-Moleküls durchgeführt wurde, die ein Segment des D-Strangs des hinteren β-Faltblatts umfassen.

6. Modifiziertes humanes TNFα-Molekül nach Anspruch 3, wobei die Substitution wenigstens ein Segment des H-Strangs des vorderen β-Faltblatts und der Verknüpfungsschleife zu dem I-Strang, vorzugsweise Aminosäuren 132 bis 146, umfasst.

7. Modifiziertes humanes TNFα-Molekül nach Anspruch 3, wobei die Substitution Segmente der H- und I-Stränge und die gesamte Verknüpfungsschleife, vorzugsweise Aminosäuren 132 bis 152, umfasst.

8. Modifiziertes humanes TNFα-Molekül nach Anspruch 3, wobei die Substitution ein Segment des D-Strangs, wenigstens ein Segment des E-Strangs und die gesamte Verknüpfungeschleife, vorzugsweise Aminosäuren 65 bis 79 oder 64 bis 84, umfasst.

9. Modifiziertes humanes TNFα-Molekül nach Anspruch 3, wobei die Substitution die ganzen C'- und C-Stränge und ein Segment des D-Strangs, vorzugsweise Aminosäuren 40 bis 60, umfasst.

10. Modifiziertes humanes TNFα-Molekül nach Anspruch 3, wobei die Substitution wenigstens ein Segment des E-Strangs des vorderen β-Faltblatts und einer oder beider der Verknüpfungsschleifen, vorzugsweise Aminosäuren 76 bis 90, umfasst.

11. Modifiziertes TNFα-Molekül nach einem der vorangehenden Ansprüche, wobei neutralisierende Antikörper, die gegen modifiziertes TNFα-Molekül in einem geeigneten Wirt gebildet werden, fähig sind, die Aktivität von nativem TNFα in L929-Bioassay deutlich zu inhibieren, und/oder wobei die Antikörper die Bindung von humanem Wildtyp-TNFα an den 55-kD-TNFα-Rezeptor-1 (TNFα-R55) oder an den 75-kD-TNFα-Rezeptor (TNFα-R75) signifikant inhibieren.

12. Modifiziertes humanes TNFα-Molekül nach einem der Ansprüche 1 bis 11, wobei das innertierte T-Zellepitop promiskuitiv ist und bekanntermaßen in einer Mehrheit von humanen HLA-Klasse-II-Typen immunogen ist.

13. Modifiziertes humanes TNFα-Molekül nach Anspruch 12, wobei das Epitop von Tetanus-Toxoid, vorzugsweise Epitop P2 und/oder P30, stammt.

14. Modifizierter TNFα nach Anspruch 13, der die in SEQ ID NO:8 gezeigte Aminosäuresequenz hat.

15. Modifizierter TNFα nach Anspruch 13, der die in SEQ ID NO:10 gezeigte Aminosäuresequenz hat.

16. Modifiziertes TNFα-Molekül nach Anspruch 13, das die in SEQ ID NO:4 oder SEQ ID NO:16 gezeigte Aminosäuresequenz hat.

17. Modifizierter TNFα nach Anspruch 13, der die in SEQ ID NO:20 gezeigte Aminosäuresequenz hat.

18. Modifizierter TNFα nach Anspruch 13, der die in SEQ ID NO:14 gezeigte Aminosäuresequenz hat.

19. Dimere, Oligomere oder Multimere des modifizierten humanen TNFα-Moleküls nach einem der Ansprüche 1-18.

20. Isoliertes DNA-Molekül, das für ein modifiziertes TNFα-Molekül nach einem der Ansprüche 1-18 codiert.

21. Vektor, der das isolierte DNA-Molekül nach Anspruch 20 umfasst.

22. Expressionsvektor, der das isolierte DNA-Molekül nach Anspruch 21 funktionell an eine Expressionskontrollsequenz gebunden umfasst.

23. Wirtszelle, die mit dem Expressionsvektor von Anspruch 22 transformiert ist.

24. Wirtszelle nach Anspruch 23, die aus Bakterienstämmen, Hefe oder anderen Pilzen und Insekten-, Säuger- oder Vogelzelllinien ausgewählt ist.

25. Verfahren zur Herstellung eines modifizierten humanen TNFα-Moleküls nach einem der Ansprüche 1-18, das Wachsenlassen der Wirtszellen nach Anspruch 23 oder 24 unter geeigneten Bedingungen, die die Produktion des modifizierten TNFα zulassen, und Gewinnung des so produzierten modifizierten TNFα umfasst.

26. Modifiziertes humanes TNFα-Molekül nach einem der Ansprüche 1-18 in Form eines Fusionsproteins mit einem Adjuvansmolekül.

27. Modifiziertes humanes TNFα-Molekül nach Anspruch 26, wobei das Adjuvansmolekül ein immunologisch aktives Adjuvans ist.

28. Modifiziertes humanes TNFα-Molekül nach Anspruch 26, wobei das Adjuvansmolekül GM-SCF, HSP70 oder Interleukin ist.

29. Vakzin gegen TNFα, umfassend ein modifiziertes humanes TNFα-Molekül oder mehrere modifizierte humane TNFα-Moleküle nach einem der Ansprüche 1-20 und gegebenenfalls ein pharmazeutisch annehmbares Adjuvans.

30. Vakzin nach Anspruch 29, wobei das pharmazeutisch akzeptable Adjuvans Aluminiumphosphat, Aluminiumhydroxid, Calciumphosphat, Muramyldipeptid oder Iscom ist.

31. Vakzin nach Anspruch 29 oder 30 zur Prävention oder Behandlung von Krankheiten, die durch TNFα-Freisetzung oder -Aktivität begünstigt werden, zum Beispiel chronische inflammatorische Krankheiten.

32. Vakzin nach Anspruch 31, wobei die chronischen inflammatorischen Krankheiten rheumatoide Arthritis und inflammatorische Darmkrankheiten sind.

33. Vakzin nach Anspruch 32, wobei die entzündlichen Darmkrankheiten Crohn'sche Krankheit und Colitis ulcerosa sind.

34. Vakzin gegen TNFα, umfassend isolierte DNA, die für das modifizierte humane TNFα-Molekül nach einem der Ansprüche 1-18 codiert, insertiert in einen geeigneten Expressionsvektor.

35. Vakzin nach Anspruch 34, enthaltend ein Konstrukt, umfassend eine nicht-infektiöse nicht-integrierende DNA-Sequenz, die für ein modifiziertes TNFα-Molekül nach einem der Ansprüche 1-18 codiert, funktionell an eine Promotorsequenz gebunden, welche die Kontrolle der Expression der DNA-Sequenz in Menschen kontrolliert, in einer Menge, die ausreicht, damit eine Aufnahme des Konstrukts erfolgt, und ausreichend Expression erfolgt, um eine neutralisierende Antikörperreaktion gegen TNFα zu induzieren.

36. Vakzin nach Anspruch 33 oder 34, das einen viralen Expressionsvektor umfasst.

37. Vakzin nach Anspruch 36, wobei der virale Expressionsvektor ein retroviraler Expressionsvektor ist.

38. Vakzin nach einem der Ansprüche 29-37, zur oralen oder parenteralen Verabreichung.

39. Vakzin nach Anspruch 38, wobei das Vakzin zur subkutanen, intramuskulären oder intradermalen Verabreichung ist.

40. Verwendung wenigstens eines modifizierten TNFα-Moleküls nach Anspruch 1-18, gegebenenfalls in Kombination mit einem geeigneten Adjuvans- oder Trägermolekül, bei der Herstellung eines Medikaments für die Behandlung oder Prävention von chronischer Entzündung, Krebs, Kachexie, disseminierter Sklerose, Diabetes, Psoriasis, Osteoporose oder Asthma.

## Revendications

1. Molécule de TNFα humain modifiée capable d'induire la production d'anticorps neutralisants dirigés contre le TNFα humain de type sauvage à la suite de l'administration de ladite molécule de TNFα modifiée à un hôte humain, dans laquelle au moins un segment de la molécule de TNFα humain a été substitué par au moins un peptide connu pour contenir un épitope de cellules T immunodominant, ou une forme tronquée de ladite molécule contenant un épitope de cellules T immunodominant et l'une ou chacune des deux régions flanquantes de la molécule de TNFα humain comprenant au moins un épitope de cellules B du TNFα, dans laquelle la substitution est introduite dans l'un quelconque des brins du feuillet β avant, dans l'une quelconque des boucles de jonction et/ou dans l'un quelconque des brins B', I, ou D du feuillet β arrière, dans laquelle la substitution a été réalisée dans des régions de la molécule de TNFα de façon à conserver la structure en feuillet β des brins B et G, et où la substitution aboutit à une inactivation de l'activité biologique du TNFα humain lorsque celle-ci est testée selon le bioessai L929.

2. Molécule de TNFα humain modifiée selon la revendication 1, dans laquelle la substitution ne comprend pas un quelconque brin entier du feuillet β arrière.

3. Molécule de TNFα humain modifiée selon la revendication 1 ou 2, dans laquelle la substitution comprend au moins un segment du brin H du feuillet β avant et de la boucle de jonction au brin I du feuillet β arrière, un segment des brins H et I et la totalité de la boucle de jonction, un segment du brin D du feuillet β arrière, et au moins un segment du brin E du feuillet β avant et la totalité de la boucle de jonction, la totalité des brins C' et C du feuillet β avant et un segment du brin D du feuillet β arrière, et au moins un segment du brin E du feuillet β avant et l'une de ou chacune des deux boucles de jonction.

4. Molécule de TNFα humain modifiée selon l'une quelconque des revendications 1 à 3, dans laquelle la substitution a été réalisée dans des régions de la molécule de TNFα qui comprennent les brins des feuillet β avant et/ou les boucles de jonction de façon à conserver essentiellement la structure du feuillet β de l'un quelconque des brins du feuillet β arrière.

5. Molécule de TNFα humain modifiée selon la revendication 3, dans laquelle la substitution a été effectuée dans des régions de la molécule de TNFα, qui comprennent un segment du brin D du feuillet β arrière.

6. Molécule de TNFα humain modifiée selon la revendication 3, dans laquelle la substitution comprend au moins un segment du brin H du feuillet β avant et de la boucle de jonction au brin I, de préférence les acides aminés 132 à 146.

7. Molécule de TNFα humain modifiée selon la revendication 3, dans laquelle la substitution comprend des segments des brins H et I et la totalité de la boucle de jonction, de préférence les acides aminés 132 à 152.

8. Molécule de TNFα humain modifiée selon la revendication 3, dans laquelle la substitution comprend un segment du brin D, au moins un segment du brin E et la totalité de la boucle de jonction, de préférence les acides aminés 85 à 79 ou 64 à 84.

9. Molécule de TNFα humain modifiée selon la revendication 3, dans laquelle la substitution comprend la totalité des brins C' et C et un segment du brin D, de préférence les acides aminés 40 à 60.

10. Molécule de TNFα humain modifiée selon la revendication 3, dans laquelle la substitution comprend au moins un segment du brin E du feuillet β avant et l'une de ou chacune des deux boucles de jonction, de préférence les acides aminés 76 à 90.

11. Molécule de TNFα modifiée selon l'une quelconque des revendications précédentes, dans laquelle des anticorps neutralisants produits contre ladite molécule de TNFα modifiée dans un hôte convenable, sont capables d'inhiber de façon significative l'activité de TNFα natif dans le bioessai L929, et/ou dans laquelle lesdits anticorps inhibent de façon significative la liaison du TNFα humain de type sauvage au récepteur 1 du TNFα de 55 kDa (TNFα-R55) ou au récepteur du TNFα de 75 kDa (TNFα-R75).

12. Molécule de TNFα humain modifiée selon l'une quelconque des revendications 1 à 11, dans laquelle l'épitope de cellules T inséré est universel et connu pour être immunogène pour une majorité de types de HLA de classe II humains.

13. Molécule de TNFα humain modifiée selon la revendication 12, dans laquelle l'épitope dérive de l'anatoxine tétanique, de préférence l'épitope P2 et/ou P30.

14. TNFα modifié selon la revendication 13, ayant la séquence d'acides aminés indiquée dans SEQ ID NO : 8.

15. TNFα modifié selon la revendication 13, ayant la séquence d'acides aminés indiquée dans SEQ IDNO : 10.

16. Molécule de TNFα humain modifiée selon la revendication 13, ayant la séquence d'acides aminés indiquée dans SEQ ID NO : 4 ou SEQ ID NO : 16.

17. TNFα modifié selon la revendication 13, ayant la séquence d'acides aminés indiquée dans SEQ ID NO : 20.

18. TNFα modifié selon la revendication 13, ayant la séquence d'acides aminés indiquée dans SEQ ID NO : 14.

19. Dimères, oligomères ou multimères de la molécule de TNFα humain modifiée selon l'une quelconque des revendications 1 à 18.

20. Molécule d'ADN isolée qui code pour une molécule de TNFα modifiée selon l'une quelconque des revendications 1 à 18.

21. Vecteur qui comprend la molécule d'ADN isolée selon la revendication 20.

22. Vecteur d'expression, qui comprend la molécule d'ADN isolée selon la revendication 21 liée de manière opérationnelle avec une séquence de controle de l'expression.

23. Cellule hôte, qui est transformée par le vecteur d'expression selon la revendication 22.

24. Cellule hôte selon la revendication 23, qui est sélectionnée parmi des souches de bactéries, de levures ou d'autres champignons et des lignées cellulaires d'insectes, de mammifères ou aviaires.

25. Procédé de production d'une molécule de TNFα humain modifiée selon l'une quelconque des revendications 1 à 18, qui comprend la mise en croissance de cellules hôtes selon la revendication 23 ou 24 dans des conditions appropriées permettant la production de TNFα modifié et la récupération du TNFα modifié ainsi produit.

26. Molécule de TNFα humain modifiée selon l'une quelconque des revendications 1 à 18 sous forme d'une protéine de fusion à une molécule adjuvante.

27. Molécule de TNFα humain modifiée selon la revendication 26, dans laquelle la molécule adjuvante est un adjuvant immunologiquement actif.

28. Molécule de TNFα humain modifiée selon la revendication 26, dans laquelle la molécule adjuvante est GM-CSF, HSP70 ou l'interleukine.

29. Vaccin contre le TNFα, comprenant une ou plusieurs molécules de TNF α humain modifiées selon l'une quelconque des revendications 1 à 20 et éventuellement un adjuvant pharmaceutiquement acceptable.

30. Vaccin selon la revendication 29, dans lequel l'adjuvant pharmaceutiquement acceptable est le phosphate d'aluminium, l'hydroxyde d'aluminium, le phosphate de calcium, le muramyldipeptide ou l'iscom.

31. Vaccin selon la revendication 29 ou 30 pour la prévention ou le traitement de maladies favorisées par la libération ou l'activité de TNFα telles que les maladies inflammatoires chroniques.

32. Vaccin selon la revendication 31, dans lequel les maladies inflammatoires chroniques sont l'arthrite rhumatoïde et les maladies inflammatoires de l'intestin.

33. Vaccin selon la revendication 32, dans lequel les maladies inflammatoires de l'intestin sont la maladie de Crohn et la colite ulcéreuse.

34. Vaccin contre le TNFα comprenant un ADN isolé, qui code pour la molécule de TNFα humain modifiée selon l'une quelconque des revendications 1 à 18, inséré dans un vecteur d'expression convenable.

35. Vaccin selon la revendication 34 contenant une construction comprenant une séquence d'ADN non infectieuse ne s'intégrant pas qui code pour une molécule de TNFα modifiée selon l'une quelconque des revendications 1 à 18 liée de manière opérationnelle avec une séquence promotrice qui est capable de réguler l'expression de ladite séquence d'ADN chez l'homme, en quantité suffisante pour qu'il y ait incorporation de ladite construction, et pour qu'il y ait expression suffisante pour induire une réponse médiée par des anticorps neutralisants dirigée contre le TNFα.

36. Vaccin selon la revendication 33 ou 34, comprenant un vecteur d'expression virale.

37. Vaccin selon la revendication 38, dans lequel le vecteur d'expression virale est un vecteur d'expression rétroviral.

38. Vaccin selon l'une quelconque des revendications 29 à 37 pour l'administration par voie orale ou parentérale.

39. Vaccin selon la revendication 38, dans lequel le vaccin est conçu pour l'administration par voie sous-cutanée, intramusculaine ou intradermique.

40. Utilisation d'au moins une molécule de TNFα modifiée selon les revendications 1 à 18, en combinaison le cas échéant avec une molécule porteuse ou adjuvante convenable, pour la fabrication d'un médicament pour le traitement ou la prévention d'une inflammation chronique, du cancer, de la cachexie, de la sclérose en plaques, du diabète, du psoriasis, de l'ostéoporose ou de l'asthme.
